(19) Europäisches Patentamt
European Patent Office
Office européen des brevets

(11) EP 4 512 845 B1

(12) # EUROPEAN PATENT SPECIFICATION

(45) Date of publication and mention of the grant of the patent:
**15.07.2026 Bulletin 2026/29**

(21) Application number: **23791904.8**

(22) Date of filing: **19.04.2023**

(51) International Patent Classification (IPC):
***C08G 65/331*** *(2006.01)* ***A61K 47/60*** *(2017.01)*
***C07D 319/08*** *(2006.01)* ***C08G 65/332*** *(2006.01)*
***C08G 65/333*** *(2006.01)* ***C08G 65/337*** *(2006.01)*

(52) Cooperative Patent Classification (CPC):
**C07D 319/08; A61K 47/60; C08G 65/3315; C08G 65/3322; C08G 65/333; C08G 65/33337; C08G 65/33396; C08G 65/337;** C08L 2203/02; Y02P 20/55

(86) International application number:
**PCT/JP2023/015663**

(87) International publication number:
**WO 2023/204256 (26.10.2023 Gazette 2023/43)**

(54) **ACETAL-TYPE RELEASABLE POLYOXYETHYLENE DERIVATIVE, PRODUCTION METHOD THEREOF AND ACETAL-TYPE RELEASABLE POLYOXYETHYLENE CONJUGATE**

ACETALARTIGES FREISETZBARES POLYOXYETHYLENDERIVAT, HERSTELLUNGSVERFAHREN DAFÜR UND ACETALARTIGES FREISETZBARES POLYOXYETHYLENKONJUGAT

DÉRIVÉ DE POLYOXYÉTHYLÈNE LIBÉRABLE DE TYPE ACÉTAL, SON PROCÉDÉ DE PRODUCTION ET CONJUGUÉ DE POLYOXYÉTHYLÈNE LIBÉRABLE DE TYPE ACÉTAL

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **22.04.2022 JP 2022070669**

(43) Date of publication of application:
**26.02.2025 Bulletin 2025/09**

(73) Proprietor: **NOF Corporation**
**Shibuya-ku**
**Tokyo 150-6019 (JP)**

(72) Inventors:
- **HAMURA, Ken**
  **Kawasaki-shi, Kanagawa 210-0865 (JP)**
- **OSAKAMA, Kazuki**
  **Kawasaki-shi, Kanagawa 210-0865 (JP)**
- **DOHI, Reina**
  **Kawasaki-shi, Kanagawa 210-0865 (JP)**
- **SUZUKI, Akira**
  **Kawasaki-shi, Kanagawa 210-0865 (JP)**

(74) Representative: **Hoffmann Eitle**
**Patent- und Rechtsanwälte PartmbB**
**Arabellastraße 30**
**81925 München (DE)**

(56) References cited:
**WO-A1-2018/180914**
**US-A1- 2020 010 620**
**US-A1- 2020 289 656**

EP 4 512 845 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relates to an acetal-type releasable polyoxyethylene derivative, a production method thereof, and an acetal-type releasable polyoxyethylene conjugate which is a conjugate with a biofunctional molecule.

### BACKGROUND ART

When a pharmaceutical that uses biofunctional molecules such as hormones, cytokines, and enzymes and the like is administered into a general living body, it is promptly excreted from the living body through glomerular filtration in the kidneys and uptake by macrophages in the liver or spleen. Therefore, a half-life in blood of the pharmaceutical is short, and it is often difficult to obtain a sufficient pharmacological action. To solve this problem, attempts have been made to chemically modify the biofunctional molecules with water-soluble polymers such as polyoxyethylene (PEG). As a result, it is possible to extend the half-life in blood of the biofunctional molecules by increasing a molecular weight and forming a hydration layer. It is also well known that these modifications can produce effects such as reduction in toxicity and antigenicity of the biofunctional molecules, improvement in aggregation properties, and the like.

However, it is known that biofunctional molecules chemically modified with water-soluble polymers such as PEG may have undesirable effects such as reduced interactions with target endogenous molecules or receptors due to the formation of the hydration layer by the water-soluble polymers or a steric shielding effect of an active site, resulting in a reduction in the inherent pharmacological action of the biofunctional molecule or changes in dynamics in the living body or cells.

As an approach to the above-mentioned problems, a method is used in which a biofunctional molecule is chemically modified with a water-soluble polymer via a temporary bond, and the temporary bond is cleaved in vivo to release the biofunctional molecule that is not chemically modified, that is, a prodrug conversion method.

In recent years, for biofunctional molecules modified with water-soluble polymers, techniques such as those described in Patent Literatures 1 and 2 have been known that can prevent a decrease in the inherent pharmacological action of the biofunctional molecules and changes in dynamics in the living body or cells by using the prodrug conversion method. Patent Literatures 1 and 2 report that PEGylated hGH and PEGylated IL-2, which are obtained by modifying a human growth hormone (hGH) and interleukin 2 (IL-2) as biofunctional molecules with PEG via a linker that is enzyme-independent and decomposes at a moderate rate under a physiological condition, that is, a neutral condition, can extend the half-life in blood thereof, and can improve the reduced pharmacological action caused by PEGylation by releasing the biofunctional molecules as the linker decomposes. For this reason, the prodrug conversion technique that can extend the half-life in blood of biofunctional molecules and release the biofunctional molecules at an appropriate rate under a physiological condition in an enzyme-independent manner to exert the pharmacological actions is important.

Patent Literature 3 reports a compound that enzyme-independently cleaves a temporary bond in a biofunctional molecule that has been chemically modified with PEG, thereby releasing the biofunctional molecule that is not chemically modified.

Further biodegradable polyethylene glycol derivatives can be found in Patent Literature 4.

Specifically, it has been reported that hydrolysis of a linker having an acetal structure that is hydrolyzed under an acidic condition triggers cleavage of a carbamate bond, which is a temporary bond, through 1,4- or 1,6-benzyl elimination, thereby releasing a biofunctional molecule that is not chemically modified.

### PRIOR ART DOCUMENTS

### PATENT LITERATURE

Patent Literature 1: JP2018-150311A
Patent Literature 2: JP2022-000043A
Patent Literature 3: JP2018-172648A
Patent Literature 4: US 2020/289656 A1

### SUMMARY OF INVENTION

OBJECT TO BE ACHIEVED BY THE INVENTION

In Examples of Patent Literature 3, specific structures capable of releasing biofunctional molecules in an enzyme-independent manner and under an acidic condition are exemplified. However, there has been no description of a prodrug conversion technique that can release a biofunctional molecule at an appropriate rate and exert a pharmacological action under a physiological condition, that is, a neutral condition of approximately pH 7.4, in an enzyme-independent manner.

An object of the present invention is to provide a polyoxyethylene derivative having an acetal structure, which is characterized by converting a biofunctional molecule into a prodrug and gradually releasing the biofunctional molecule under a physiological condition, a stable production method thereof, and an acetal-type releasable polyoxyethylene conjugate.

MEANS FOR ACHIEVING THE OBJECT

As a result of diligent studies, the present inventors have discovered a polyoxyethylene derivative having an acetal structure, which is characterized by converting a biofunctional molecule into a prodrug and gradually hydrolyzing the acetal under a physiological condition to induce benzyl elimination, thereby releasing the biofunctional molecule, a stable production method thereof, and an acetal-type releasable polyoxyethylene conjugate.

That is, the present invention relates to the following [1] to [6].

[1] An acetal-type releasable polyoxyethylene derivative, expressed by the following Formula (1), (2), (3) or (4), and cleaved under a physiological condition,

[Chem. 1]

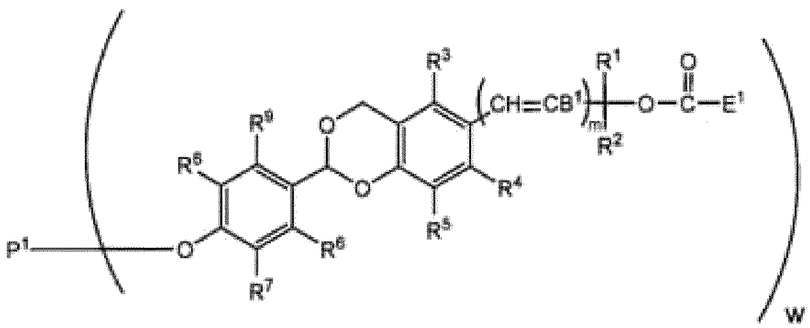

(1)

[Chem. 2]

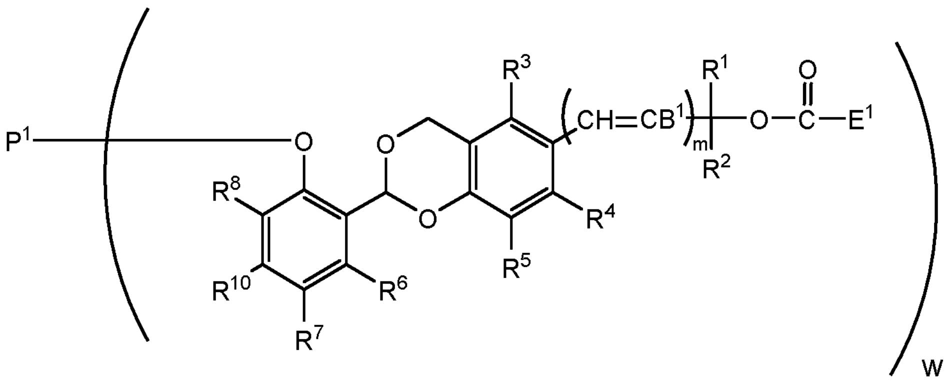

(2)

[Chem. 3]

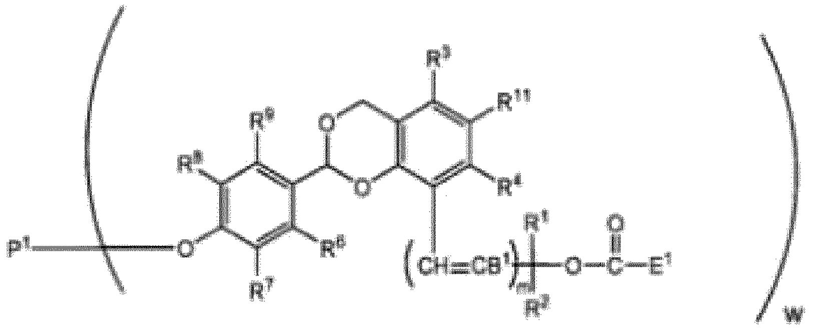

(3)

[Chem. 4]

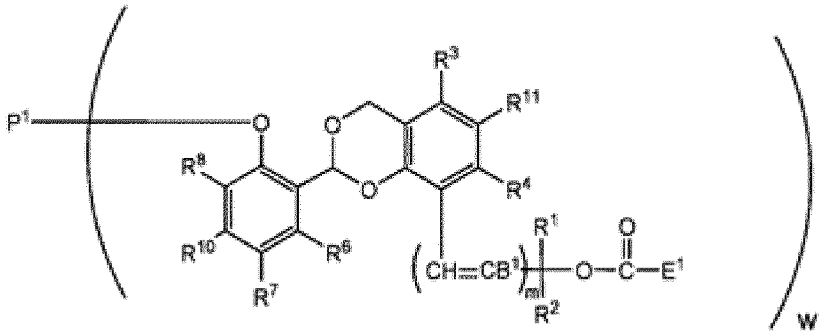

(4)

in which,

- $B^1$ represents a hydrogen atom or $-C(R^1)(R^2)OC(O)E^1$,
- $E^1$ represents a leaving group,
- $P^1$ represents a polyoxyethylene derivative having a dehydroxylated group,
- w represents an integer of 1 to 8,
- $R^1$, $R^2$, $R^3$, $R^4$, $R^5$ and $R^{11}$ each independently represent a hydrocarbon group having 1 to 10 carbon atoms or a hydrogen atom,
- $R^6$, $R^7$, $R^8$, $R^9$ and $R^{10}$ each independently represent an electron-withdrawing substituent, an electron-donating substituent, or a hydrogen atom, and
- m represents 0 or 1.

[2] The acetal-type releasable polyoxyethylene derivative according to [1], in which m represents 0, $R^1$ and $R^2$ represent a hydrogen atom, and $R^3$, $R^4$, $R^5$ and $R^{11}$ each independently represent a hydrogen atom or a methyl group.
[3] A method for producing the acetal-type releasable polyoxyethylene derivative according to [1] or [2], the method including:

- a coupling step of coupling a polyoxyethylene derivative with a hydroxybenzaldehyde derivative to obtain a coupling product expressed by the following Formula (5) or (6);
- an acetalization step of, after the coupling step, reacting the coupling product expressed by Formula (5) or (6) with a phenol having a hydroxymethyl group at 2-position and having a substituent $(-CH=CB^1)_mC(R^1)(R^2)-OH$ ($B^1$, m, $R^1$, and $R^2$ are as described above) at 4-position or 6-position under an acidic condition to obtain an acetal structure; and
- a leaving group structure introduction step of introducing a leaving group structure $(-OC(O)E^1)$ to a terminal of the substituent at the 4-position or the 6-position after the acetalization step,

[Chem. 5]

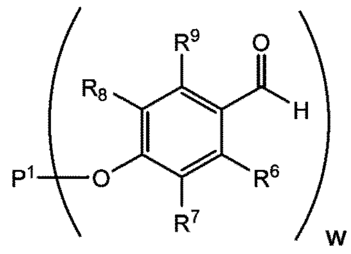

(5)

[Chem. 6]

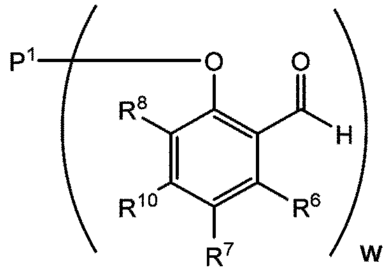

(6)

in which $P^1$, w, $R^6$, $R^7$, $R^8$, $R^9$ and $R^{10}$ are as described above.

[4] The method for producing the acetal-type releasable polyoxyethylene derivative according to [3], the method further including, between the acetalization step and the leaving group structure introduction step: a deprotection step of deprotecting an amino group protected by a protecting group in the coupling product expressed by Formulas (5) and (6); and a step of introducing a group capable of reacting with a biofunctional molecule into the amino group deprotected after the deprotection step.

[5] An acetal-type releasable polyoxyethylene conjugate, expressed by the following Formula (7), (8), (9) or (10), and cleaved under a physiological condition,

[Chem. 7]

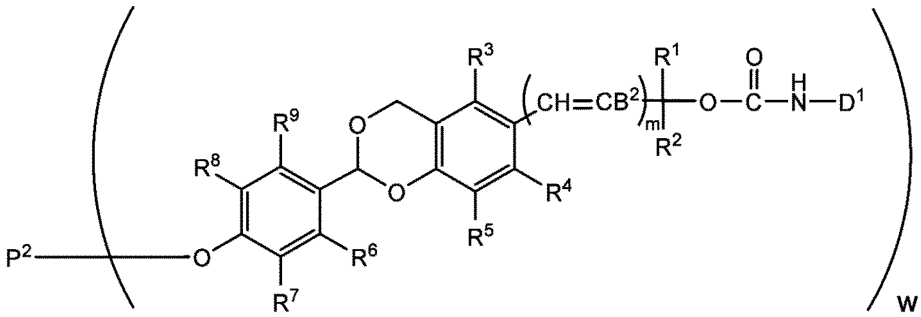

(7)

[Chem. 8]

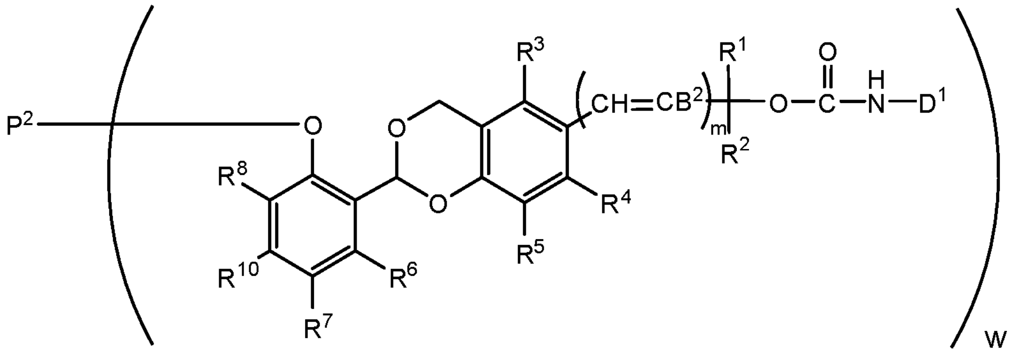

(8)

[Chem. 9]

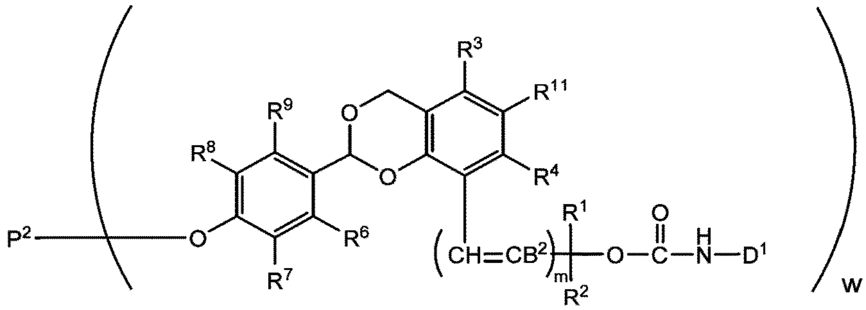

(9)

[Chem. 10]

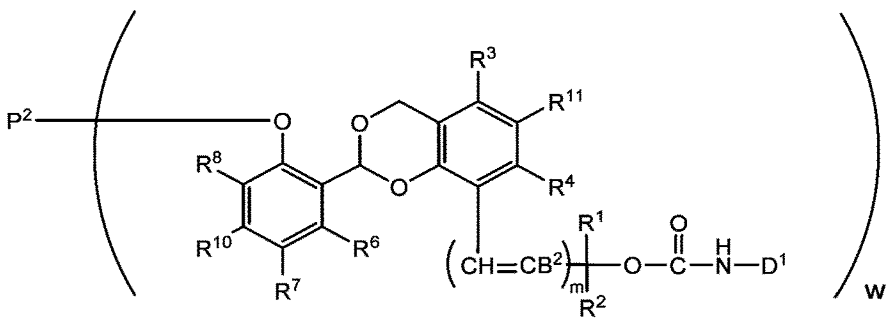

(10)

in which

$B^2$ represents a hydrogen atom or $-C(R^1)(R^2)OC(O)NHD^1$,
$D^1$ represents a residue obtained by removing an amino group that constitutes a carbamate bond from an amino group contained in a biofunctional molecule,
$P^2$ represents a polyoxyethylene derivative having a dehydroxylated group, or a conjugate of a biofunctional molecule and a polyoxyethylene derivative having a dehydroxylated group,
w represents an integer of 1 to 8,
$R^1$, $R^2$, $R^3$, $R^4$, $R^5$ and $R^{11}$ each independently represent a hydrocarbon group having 1 to 10 carbon atoms or a hydrogen atom,
$R^6$, $R^7$, $R^8$, $R^9$ and $R^{10}$ each independently represent an electron-withdrawing substituent, an electron-donating substituent, or a hydrogen atom, and
m represents 0 or 1.

[6] A method for producing the acetal-type releasable polyoxyethylene conjugate according to [5], the method including:

a coupling step of reacting the acetal-type releasable polyoxyethylene derivative according to [1] or [2] with a biofunctional molecule in a neutral or basic buffer solution which may contain a water-soluble organic solvent; and
a purification step under a neutral or basic condition after the coupling step.

## EFFECTS OF INVENTION

The acetal-type releasable polyoxyethylene derivative of the present invention can improve a pharmacological action of a physiologically functional molecule modified with a polyoxyethylene derivative by converting the biofunctional molecule into a prodrug, gradually hydrolyzes acetal under a physiological condition to induce benzyl elimination, and gradually releasing the biofunctional molecule.

Note that although in Examples of Patent Literature 3, specific structures capable of releasing a biofunctional molecule in an enzyme-independent manner and under an acidic condition are exemplified, there is no description regarding a specific structure capable of releasing a biofunctional molecule by benzyl elimination triggered by acetal hydrolysis under a physiological condition, that is, a neutral condition at approximately pH 7.4.

Here, an acetal structure in a compound of Patent Literature 3 is generally used as a protecting group for a diol or carbonyl group, and it is known that the deprotection thereof is performed under an acidic condition and to be stable under a neutral or basic condition. Therefore, in the compound in Patent Literature 3, there is no suggestion of creating a compound that is hydrolyzed at an appropriate rate under a physiological condition, that is, under a neutral condition, to release a physiologically functional molecule.

A production method in Patent Literature 3 includes a step of coupling a phenolic hydroxyl group of a low molecular weight compound having an acetal structure with a hydroxy group of PEG under a basic condition, but it has been found that when the production of the acetal-type releasable polyoxyethylene derivative of the present invention is performed through a coupling step similar to that of Patent Literature 3, the acetal structure in the low molecular weight compound becomes unstable, making it difficult to obtain the acetal-type releasable polyoxyethylene derivative of the present invention.

## BRIEF DESCRIPTION OF DRAWINGS

Fig. 1 shows results of a decomposition test at 40°C in a deuterium oxide buffer solution having a pD of 7.4 using compounds expressed by Formulas (50), (51), (52) and (53).
Fig. 2 shows results of HPLC analysis of purified PEGylated RNase described in Example 13.
Fig. 3 shows results of a decomposition test at 40°C in a deuterium oxide buffer solution having a pD of 7.4 using compounds expressed by Formulas (75), (76), (77) and (78).
Fig. 4 shows a nucleotide sequence of an aptamer having TNF$\alpha$ inhibitory activity.
Fig. 5 is a drawing in which A shows an HPLC analysis result of purified PEG(20k)-OCO-NH-C6-(Apt-TNF$\alpha$) described in Comparative Example 3, and B shows an HPLC analysis result of purified 2MPEG(20k)-OCO-NH-C6-(Apt-TNF$\alpha$) described in Example 25.
Fig. 6 shows evaluation results of TNF$\alpha$ inhibitory activity using PEG(20k)-OCO-NH-C6-(Apt-TNF$\alpha$), 2MPEG(20k)-OCO-NH-C6-(Apt-TNF$\alpha$), and $NH_2$-C6-(Apt-TNF$\alpha$) modified with a C6 amino linker at 5' terminal of an aptamer of SEQ ID NO: 1.
Fig. 7 is a drawing in which A shows an HPLC analysis result of purified PEG(20k)-OCO-NH-insulin described in

Comparative Example 4, and B shows an HPLC analysis result of purified 2MPEG(20k)-OCO-NH-insulin described in Example 26.

Fig. 8 is a drawing in which A shows stability evaluation results of PEG(20k)-OCO-NH-insulin in buffer solutions with various pH values, and B shows stability evaluation results of 2MPEG(20k)-OCO-NH-insulin in buffer solutions with various pH values.

EMBODIMENTS FOR CARRYING OUT THE INVENTION

The present invention will be described in detail below.

The present invention is characterized in that, under a physiological condition, acetal undergoes hydrolysis, followed by benzyl elimination, and a biofunctional molecule is gradually released. In the present description, the "physiological condition" refers to a pH of 6.0 to 8.0.

A release rate of the biofunctional molecule under the physiological condition can be evaluated as a half-life of the acetal in a buffer solution of pH 7.4. The half-life of the acetal in a buffer solution of pH 7.4 is preferably 0.5 days to 35 days. Here, the "half-life" refers to a time required for the acetal to be hydrolyzed to convert 1/2 equivalent thereof to aldehyde.

A compound exemplified in Examples of Patent Literature 3, in which a polyoxyethylene derivative is introduced via an ether bond to 3-position of a phenyl group bonded to acetal, could not achieve the above-mentioned acetal half-life under a physiological condition. In contrast, the present inventors have found that a compound in which a polyoxyethylene derivative is introduced via an ether bond to 2-position or 4-position of a phenyl group bonded to acetal can achieve the above-mentioned acetal half-life when applied under a physiological condition.

That is, the present invention is an acetal-type releasable polyoxyethylene derivative expressed by the following Formula (1), (2), (3) or (4), and cleaved under a physiological condition.

[Chem. 11]

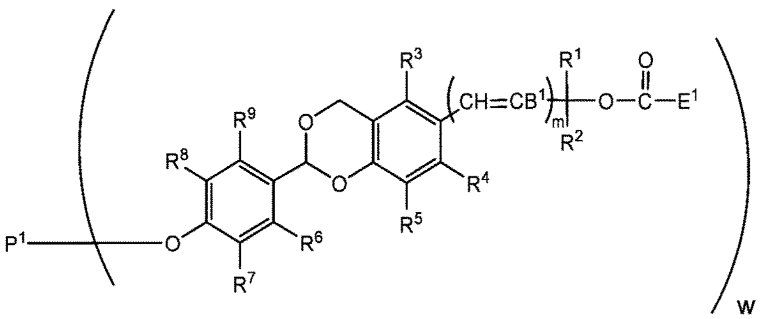

(1)

[Chem. 12]

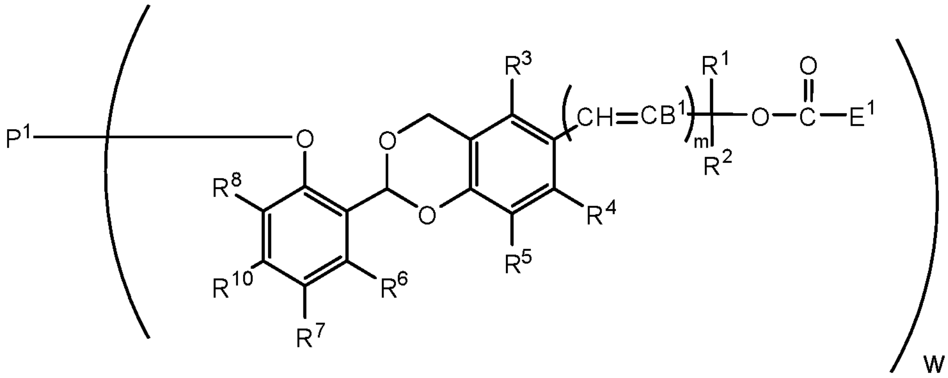

(2)

[Chem. 13]

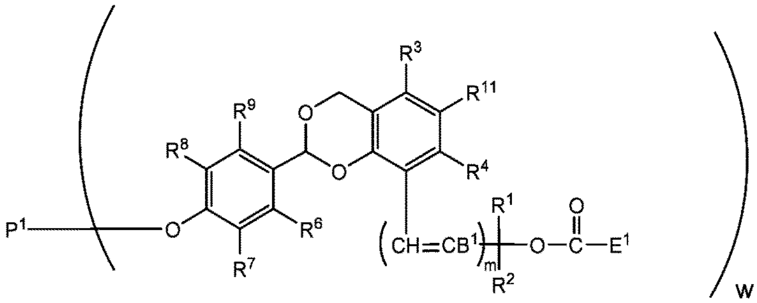

( 3 )

[Chem. 14]

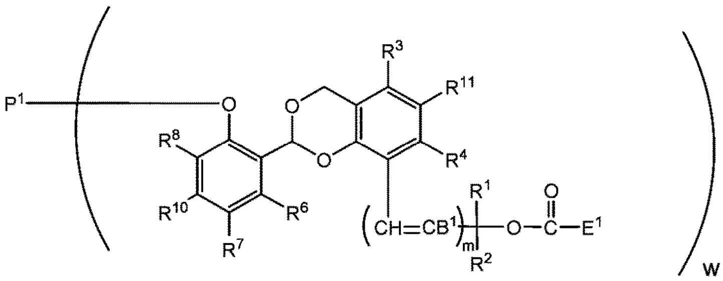

( 4 )

In Formula (1), Formula (2), Formula (3) and Formula (4),

$B^1$ represents a hydrogen atom or -C($R^1$)($R^2$)OC(O)$E^1$,
$E^1$ represents a leaving group,
$P^1$ represents a polyoxyethylene derivative having a dehydroxylated group,
w represents an integer of 1 to 8,
$R^1$, $R^2$, $R^3$, $R^4$, $R^5$ and $R^{11}$ each independently represent a hydrocarbon group having 1 to 10 carbon atoms or a hydrogen atom,
$R^6$, $R^7$, $R^8$, $R^9$ and $R^{10}$ each independently represent an electron-withdrawing substituent, an electron-donating substituent, or a hydrogen atom, and
m represents 0 or 1.

In Formulas (1) to (4), $R^1$, $R^2$ and $E^1$ will be described in detail later, and $B^1$ represents a hydrogen atom or -C($R^1$)($R^2$)OC(O)$E^1$, and preferably represents a hydrogen atom.

In Formulas (1) to (4), $E^1$ represents a leaving group, and preferred examples of the leaving group include a succinimidyloxy group, a phthalimidyloxy group, a 4-nitrophenoxy group, a 1-imidazolyl group, a pentafluorophenoxy group, a benzotriazol-1-yloxy group, and a 7-azabenzotriazol-1-yloxy group, with the succinimidyloxy group, 4-nitrophenoxy group, 1-imidazolyl group, pentafluorophenoxy group being more preferred, and the succinimidyloxy group and 4-nitrophenoxy group being still more preferred.

In Formulas (1) to (4), $P^1$ represents a polyoxyethylene derivative having a dehydroxylated group, specifically a residue obtained by removing a hydroxy group (OH) constituting an ether bond (-O-) from a polyoxyethylene derivative having a hydroxy group ($P^1$-OH). Polyoxyethylene includes both polyoxyethylene having a molecular weight distribution obtained by polymerization of ethylene oxides, and monodisperse polyoxyethylene obtained by bonded oligooxyethylenes having a single molecular weight by a coupling reaction.

Preferred examples of $P^1$ include the following residue depending on the number of W.

When w = 1, the residue is expressed by the following Formula (p1) or (p2).

[Chem. 15]

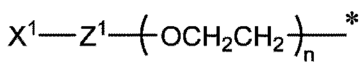

(p1)

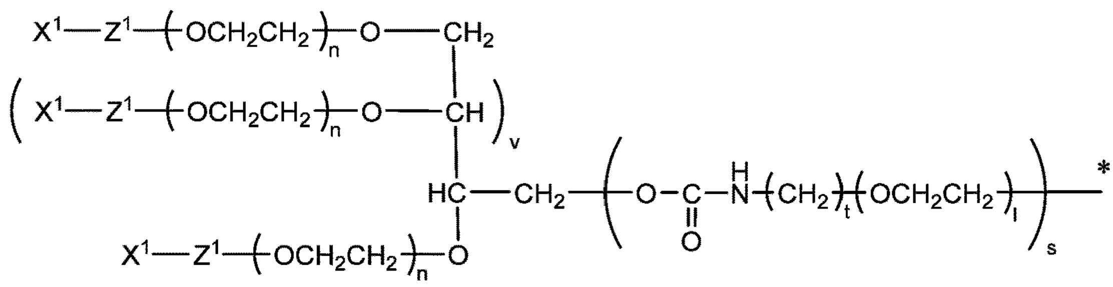

(p2)

($X^1$ represents a hydrocarbon group having 1 to 24 carbon atoms, an amino group protected by a protecting group, or a group capable of reacting with a biofunctional molecule,
$Z^1$ represents a divalent spacer or a single bond,
n and l each independently represent 3 to 2,000, and
s represents 0 or 1, t represents 2 or 3, and v represents 0 or 2.)

When w = 2, the residue is expressed by the following Formula (p3) or (p4).

[Chem. 16]

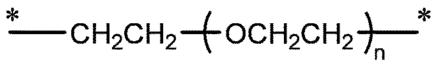

(p3)

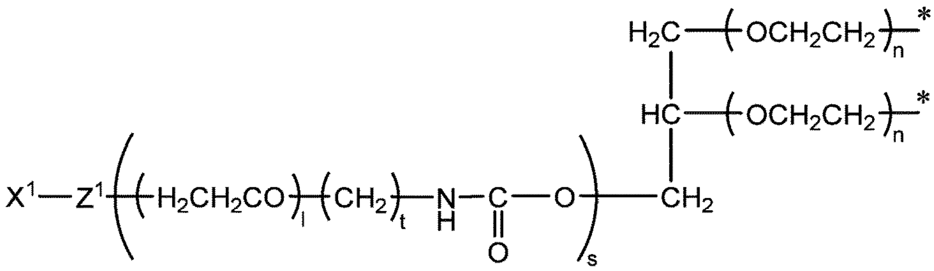

(p4)

($X^1$, $Z^1$, n, l, s and t are as defined above.)

When w = 3, the residue is expressed by the following Formula (p5).

[Chem. 17]

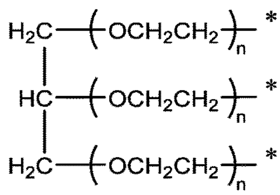

(p5)

(n is as defined above.)

When w = 4, the residue is expressed by the following Formulas (p6), (p7), or (p8).

[Chem. 18]

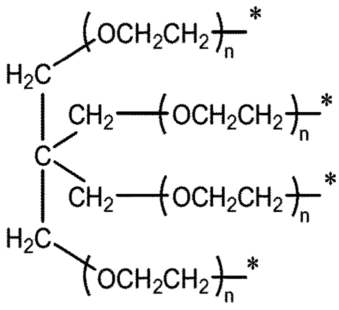

(p6)

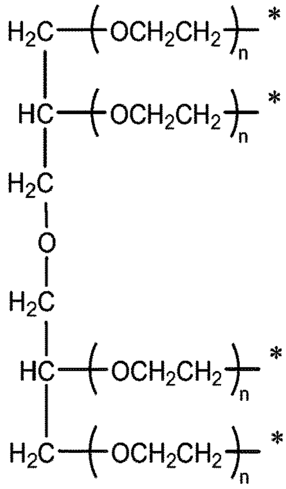

(p7)

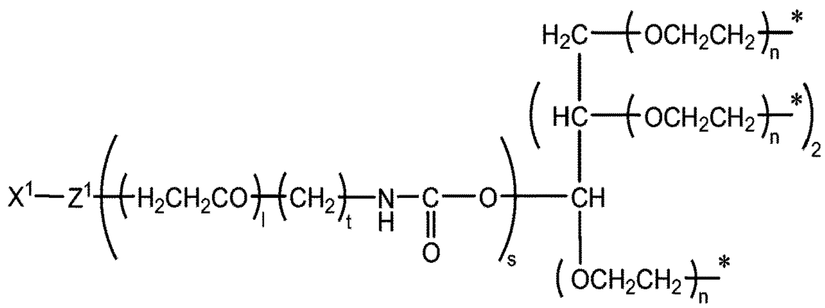

(p8)

($X^1$, $Z^1$, n, l, s and t are as defined above.)

When w = 5, the residue is expressed by the following Formula (p9).

[Chem. 19]

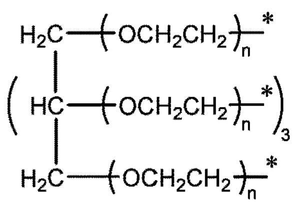

(p9)

(n is as defined above.)

When w = 6, the residue is expressed by the following Formula (p10).

[Chem. 20]

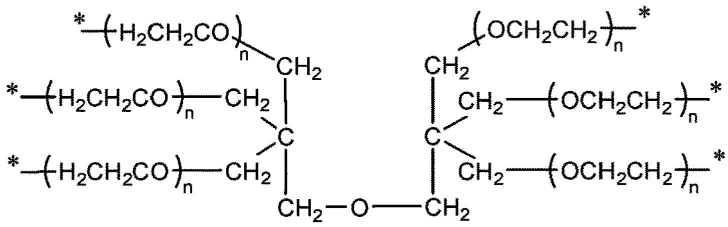

(p10)

(n is as defined above.)

When w = 8, the residue is expressed by the following Formulas (p11), (p12), or (p13).

[Chem. 21]

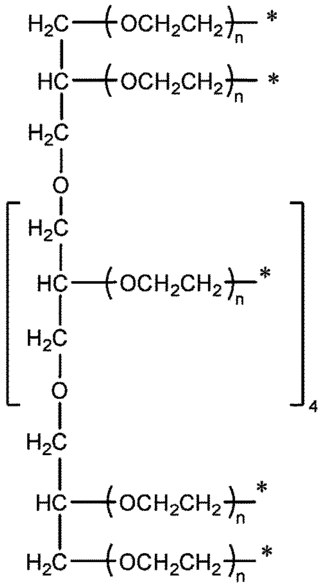

(p11)

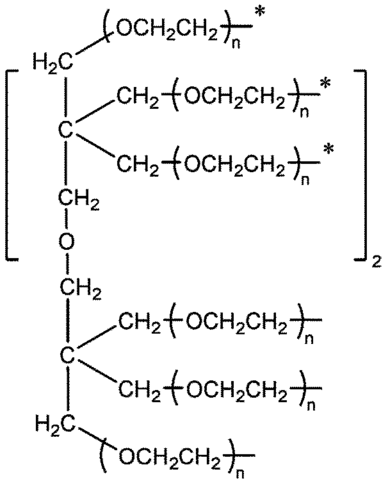

(p12)

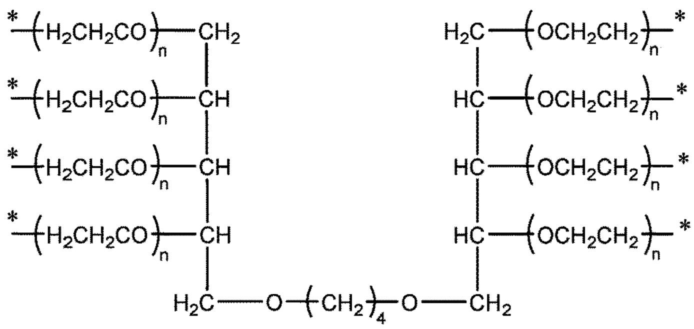

(p13)

The symbol "*" indicates a bonding point with an oxygen atom.

In Formulas (p1) to (p13), n and l both represent an addition mole number of an oxyethylene group expressed by $-(OCH_2CH_2)-$, and each independently represent 3 to 2,000, preferably 20 to 1,500, more preferably 40 to 1,000, and still more preferably 60 to 500, and can be calculated by subtracting a molecular weight derived from molecules other than a polyoxyethylene chain expressed by $-(OCH_2CH_2)_n-$ from a number average molecular weight of the polyoxyethylene derivative determined by size exclusion chromatography, mass spectrometry, or the like, and then dividing the result by a molecular weight of 44 derived from the oxyethylene group.

In Formulas (p1), (p2), (p4) and Formula (p8), $Z^1$ represents a divalent spacer or a single bond connecting the polyoxyethylene group and $X^1$, and the divalent spacer is not particularly limited as long as it is more stable than the acetal structure, and is preferably an ether bond, an ester bond, a carbonate bond, a urethane bond, an amide bond, a secondary amino group, or an alkylene group containing the above. The alkylene group has 1 to 24 carbon atoms, and examples thereof include spacers (z1) to (z8) described in Group (I).

Group (I)

[Chem. 22]

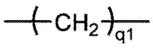

(z1)

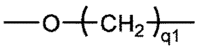

(z2)

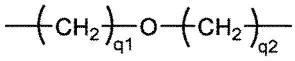

(z3)

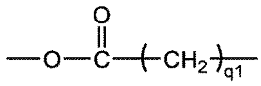

(z4)

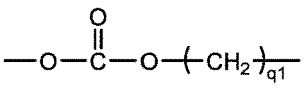

(z5)

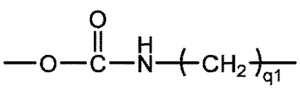

(z6)

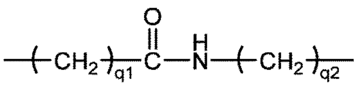

(z7)

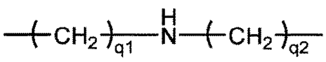

(z8)

**In** the formulas, q1 and q2 each independently represent an integer of 1 to 12. For example, when it is desired to bond a terminal active carbonate group in a hydrophobic environment such as an inside of a protein, it is preferable that q1 and q2 are large, whereas when it is desired to bond in a hydrophilic environment, it is preferable that q1 and q2 are small. However, $Z^1$ represents an ether bond, an ester bond, a carbonate bond, a urethane bond, an amide bond, a secondary amino group, or an alkylene group containing the above, and when a plurality of identical structural units are bonded, the number of the structural units is 2 or less.

**In** Formulas (p1), (p2), (p4) and Formula (p8), $X^1$ represents a hydrocarbon group having 1 to 24 carbon atoms, an amino group protected by a protecting group, or a group capable of reacting with a biofunctional molecule.

Specific examples of the hydrocarbon group include a methyl group, an ethyl group, a propyl group, an isopropyl group, a butyl group, a t-butyl group, a pentyl group, an isopentyl group, a hexyl group, a heptyl group, a 2-ethylhexyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a heneicosyl group, a docosyl group, a toicosyl group, a tetracosyl group, a phenyl group, a benzyl group, a cresyl group, a butylphenyl group, a dodecylphenyl group, and a trityl group, with the hydrocarbon group having 1 to 10 carbon atoms being preferred, the methyl group or ethyl group being more preferred, and the methyl group being still more preferred.

Here, the protecting group is a component that prevents or inhibits reaction of a particular chemically reactive functional group in a molecule under a certain reaction condition. The protecting group varies depending on a type of the chemically reactive functional group to be protected, a condition when being used, and presence of other functional group or protecting group in the molecule. Specific examples of the protecting group can be found in many general textbooks, for example, "Wuts, P. G. M.; Greene, T. W. Protective Groups in Organic Synthesis, 4th ed.; Wiley-Interscience: New York, 2007". In the present invention, examples of the amino group protected by a protecting group include an amino group or an azido group protected by an acyl protecting group or a carbamate protecting group, and specific examples of the acyl protecting group or the carbamate protecting group include a trifluoroacetyl group, a 9-fluorenylmethyloxycarbonyl group, and a 2-(trimethylsilyl)ethyloxycarbonyl group.

Examples of the group capable of reacting with a biofunctional molecule include a formyl group, an epoxy group, a maleimidyl group, a vinylsulfone group, an acryl group, a sulfonyloxy group, a carboxy group, a dithiopyridyl group, an α-haloacetyl group, an alkynyl group, an allyl group, a vinyl group, and an azido group.

More specifically, examples of a functional group capable of reacting with an amino group of a biofunctional molecule to form a covalent bond include a formyl group, an epoxy group, a maleimidyl group, a vinylsulfone group, an acryl group, a sulfonyloxy group, and a carboxy group. Examples of a functional group capable of reacting with a thiol group of a biofunctional molecule to form a covalent bond include a formyl group, an epoxy group, a maleimidyl group, a vinylsulfone group, an acryl group, a sulfonyloxy group, a carboxy group, a dithiopyridyl group, an α-haloacetyl group, an alkynyl group, an allyl group, and a vinyl group. A functional group capable of reacting with an alkynyl group of a biofunctional molecule to form a covalent bond may be an azido group. A functional group capable of reacting with an azido group of a biofunctional molecule to form a covalent bond may be an alkynyl group or a functional group containing a triple

bond.

In a preferred embodiment of this aspect, the group capable of reacting with a biofunctional molecule is a group expressed by Group (II), Group (III), Group (IV) or Group (V). Note that the symbol "**" represents a bonding point with $Z^1$.

Group (II): functional groups capable of reacting with an amino group of a biofunctional molecule to form a covalent bond
The following (a), (b), (e) and (f)
Group (III): functional groups capable of reacting with a thiol group of a biofunctional molecule to form a covalent bond
The following (a), (b), (c), (d), (e), (f) and (h)
Group (IV): functional groups capable of reacting with an alkynyl group of a biofunctional molecule to form a covalent bond
The following (c), (d) and (g)
Group (V): functional groups capable of reacting with an azido group of a biofunctional molecule to form a covalent bond
the following (i) and (j)

[Chem. 23]

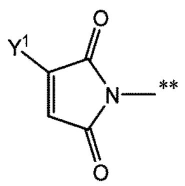
(a)

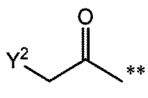
(b)

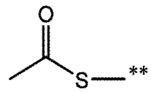
(c)

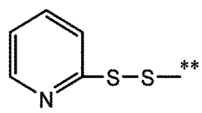
(d)

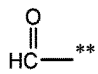
(e)

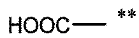
(f)

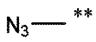
(g)

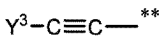
(h)

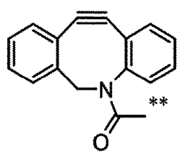
(i)

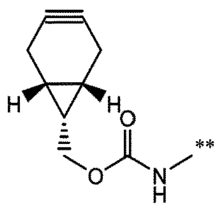
(j)

In the formulas, $Y^1$ and $Y^3$ each independently represent a hydrogen atom or a hydrocarbon group having 1 to 5 carbon atoms, and specific examples of the hydrocarbon group include a methyl group, an ethyl group, a propyl group, an isopropyl group, a butyl group, a t-butyl group, and a pentyl group. $Y^2$ represents a halogen atom selected from a chlorine atom, a bromine atom, and an iodine atom.

In Formulas (p2), (p4) and (p8), s represents 0 or 1, t represents 2 or 3, and v represents 0 or 2, and preferred embodiments are residues expressed by the following Formulas (p14) to (p19).

[Chem. 24]

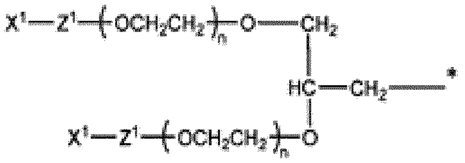

(p14)

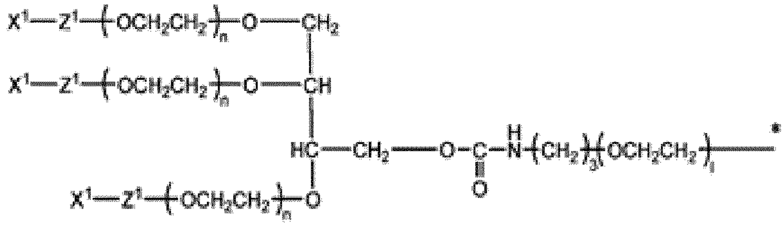

(p15)

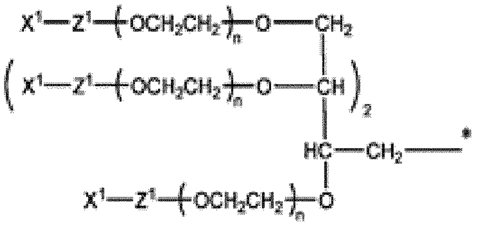

(p16)

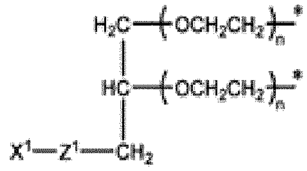

(p17)

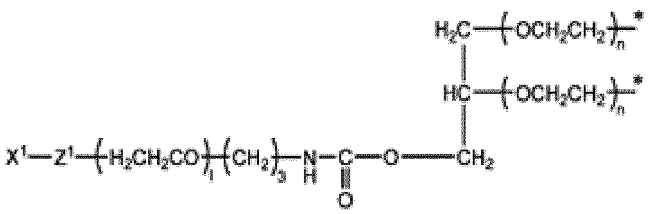

(p18)

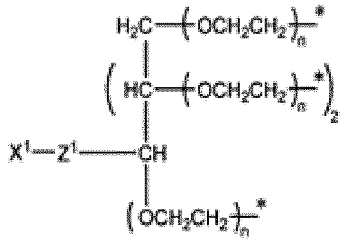

(p19)

In Formulas (p14) to (p19), $X^1$, $Z^1$, n and I are as defined above.

In Formulas (1) to (4), $R^1$, $R^2$, $R^3$, $R^4$, $R^5$ and $R^{11}$ each independently represent a hydrocarbon group having 1 to 10 carbon atoms or a hydrogen atom, and specific examples of the hydrocarbon group include a methyl group, an ethyl group, a propyl group, an isopropyl group, a t-butyl group, a phenyl group, and a benzyl group, with the hydrogen atom or methyl group being more preferred.

In Formulas (1) to (4), $R^6$, $R^7$, $R^8$, $R^9$ and $R^{10}$ each independently represent an electron-withdrawing substituent, an electron-donating substituent, or a hydrogen atom. Examples of the electron-withdrawing substituent include an acyl group having 2 to 5 carbon atoms, an alkoxycarbonyl group having 2 to 5 carbon atoms, a carbamoyl group having 2 to 5 carbon atoms, an acyloxy group having 2 to 5 carbon atoms, an acylamino group having 2 to 5 carbon atoms, an alkoxycarbonylamino group having 2 to 5 carbon atoms, a fluorine atom, a chlorine atom, a bromine atom, an iodine atom, an alkylsulfanyl group having 1 to 4 carbon atoms, an alkylsulfonyl group having 1 to 4 carbon atoms, an arylsulfonyl group having 6 to 10 carbon atoms, a nitro group, a trifluoromethyl group, and a cyano group, with the acetyl group, methoxycarbonyl group, methylcarbamoyl group, acetoxy group, acetamide group, methoxycarbonylamino group,

fluorine atom, chlorine atom, bromine atom, iodine atom, methylsulfanyl group, phenylsulfonyl group, nitro group, trifluoromethyl group, and cyano group being preferred.

The electron-donating substituent may be an alkyl group having 1 to 4 carbon atoms, and preferred examples thereof include a methyl group, an ethyl group, a propyl group, an isopropyl group, and a t-butyl group. Examples of a substituent which is electron-withdrawing at the meta position of a phenyl group, that is, $R^7$ or $R^8$, and electron-donating at the para position and ortho position, that is, $R^6$, $R^9$ or $R^{10}$, include an alkoxy group having 1 to 4 carbon atoms, an aryl group having 6 to 10 carbon atoms, and an aryloxy group having 6 to 10 carbon atoms, and preferred examples thereof include a methoxy group, an ethoxy group, a propoxy group, an isopropoxy group, a t-butoxy group, a phenyl group, and a phenoxy group.

As a more preferred embodiment of the present invention, it is more preferable that, in Formulas (1) to (4), m represents 0, and $R^1$ and $R^2$ represent a hydrogen atom, $R^3$, $R^4$, $R^5$ and $R^{11}$ represent a hydrogen atom or a methyl group having 1 carbon atom, and $R^3$, $R^4$ and $R^5$ represent a hydrogen atom, and $R^{11}$ represents a methyl group.

Next, a production method of the acetal-type releasable polyoxyethylene derivative according to the present invention will be described. To produce the acetal-type releasable polyoxyethylene derivative according to the present invention, a coupling step of coupling a polyoxyethylene derivative with a hydroxybenzaldehyde derivative to obtain a coupling product expressed by the following Formula (5) or (6); an acetalization step of, after the coupling step, reacting the coupling product expressed by Formula (5) or (6) with a phenol having a hydroxymethyl group at 2-position and having a substituent ($-CH=CB^1)_mC(R^1)(R^2)-OH$ ($B^1$, m, $R^1$, and $R^2$ are as described above) at 4-position or 6-position under an acidic condition to obtain an acetal structure; and a leaving group structure introduction step of introducing a leaving group structure ($-OC(O)E^1$) to a terminal of the substituent at the 4-position or the 6-position after the acetalization step, are performed.

[Chem. 25]

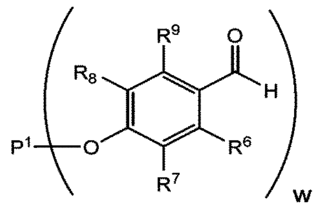

(5)

[Chem. 26]

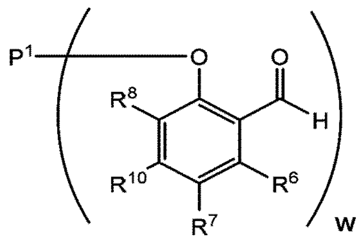

(6)

(In Formula (5) or (6), $P^1$, w, $R^6$, $R^7$, $R^8$, $R^9$ and $R^{10}$ are as described above.)

The coupling step of a polyoxyethylene derivative and a hydroxybenzaldehyde derivative is a step of obtaining a coupling product of Formula (5) or (6) via a reaction step expressed by Reaction 1-1 or Reaction 1-2, and a purification step may be performed after the reaction step.

[0065] (Reaction 1-1)

[Chem. 27]

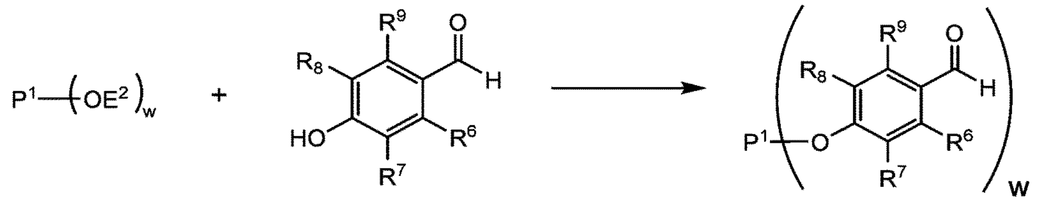

(11) (12) (5)

(Reaction 1-2)

[Chem. 28]

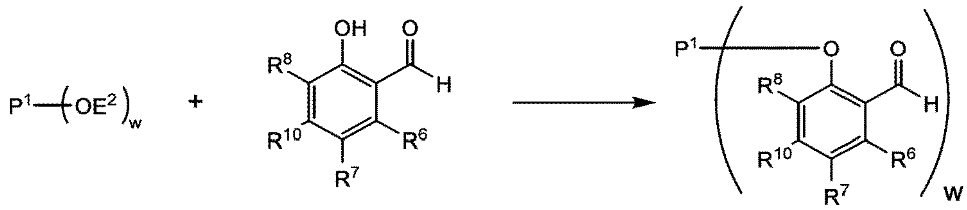

(11) (13) (6)

The polyoxyethylene derivative used in the coupling reaction is a compound expressed by Formula (11), and in Formula (11), detailed definitions of $P^1$ and w are the same as those above, and $E^2$ represents a leaving group or a hydrogen atom. The leaving group is not particularly limited as long as it is a group that has reactivity in a coupling reaction, and examples thereof include a chloro group, a bromo group, an iodo group, a mesylate group, a tosylate group, and a chloromethanesulfonate group, with the mesylate group or tosylate group being preferred, and the mesylate group being more preferred. The hydroxybenzaldehyde derivative is a compound expressed by Formula (12) or (13), in which detailed definitions of $R^6$ to $R^{10}$ are the same as those above.

When $E^2$ in Formula (11) represents a leaving group, Reaction 1-1 or Reaction 1-2 is a coupling reaction of the compound of Formula (11) with a hydroxybenzaldehyde derivative in an aprotic solvent such as toluene, benzene, xylene, acetonitrile, ethyl acetate, diethyl ether, t-butyl methyl ether, tetrahydrofuran, chloroform, dichloromethane, dimethylsulfoxide, dimethylformamide, or dimethylacetamide, or with no solvent, and in the presence an organic base such as triethylamine, N-methylmorpholine, potassium t-butoxide, or sodium hexamethyldisilazide, or an inorganic base such as potassium carbonate, potassium hydroxide, or sodium hydride. Proportions of the hydroxybenzaldehyde derivative, the organic base and the inorganic base used are not particularly limited, but are preferably equimolar or more relative to chemically reactive functional groups of the compound of Formula (11). An organic base may also be used as a solvent.

When $E^2$ in Formula (11) represents a hydrogen atom, Reaction 1-1 or Reaction 1-2 is a coupling reaction of Formula (11) with a hydroxybenzaldehyde derivative in the presence of an azo reagent such as diethyl azodicarboxylate or diisopropyl azodicarboxylate and a phosphine such as triphenylphosphine, tri-n-octylphosphine or tributylphosphine, in an organic solvent such as tetrahydrofuran, dioxane or dichloromethane. Proportions of the hydroxybenzaldehyde derivative, the azo reagent and the phosphine used are not particularly limited, but are preferably equimolar or more relative to the chemically reactive functional groups of the compound of Formula (11).

After the reaction step of Reaction 1-1 and Reaction 1-2, impurities produced as by-products in the reaction, remaining compounds that are not consumed in the reaction, and base catalysts are preferably removed by a purification step, and a purification method is not particularly limited, and purification can be performed by extraction, recrystallization, adsorption treatment, reprecipitation, column chromatography, supercritical extraction, or the like.

After the coupling step, the acetalization step of reacting, under an acidic condition, the coupling product expressed by Formula (5) or (6) with a phenol having a hydroxymethyl group at 2-position and having a substituent $(-CH=CB^1)_mC(R^1)(R^2)-OH$ ($B^1$, m, $R^1$, $R^2$ are as described above) at 4-position or 6-position, is a step of obtaining an acetal structure expressed by Formula (15), (16), (18) or (19) through a reaction step expressed by any one of Reactions 2-1 to 2-4, and a purification step may be performed after the reaction step.

(Reaction 2-1)

[Chem. 29]

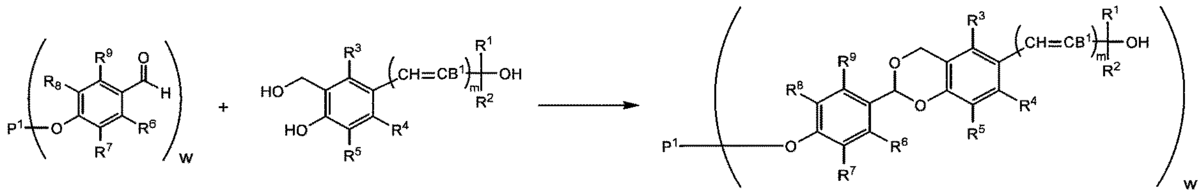

(5) (14) (15)

(Reaction 2-2)

[Chem. 30]

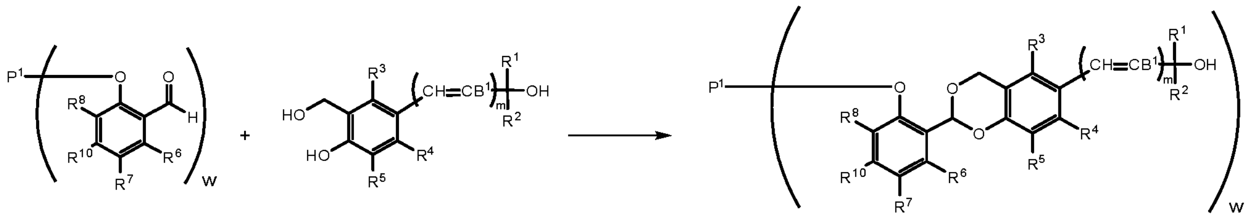

(6) (14) (16)

(Reaction 2-3)

[Chem. 31]

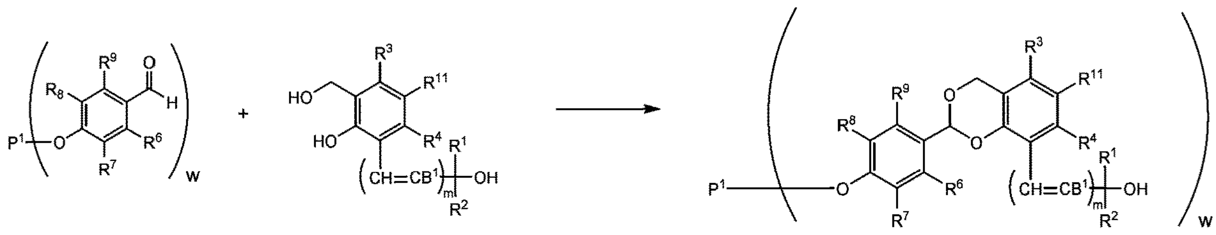

(5) (17) (18)

(Reaction 2-4)

[Chem. 32]

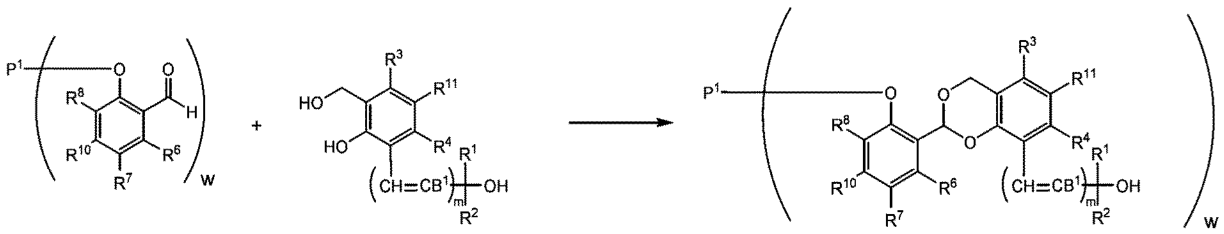

(6) (17) (19)

In Formulas (14) to (19), details of $P^1$, $B^1$, m, $R^1$, $R^2$, $R^3$, $R^4$, $R^5$ and $R^{11}$ are the same as those in the above description of the acetal-type releasable polyoxyethylene derivative.

Each of Reactions 2-1 to 2-4 is a step of reacting the coupling product expressed by Formula (5) or Formula (6) with the phenol expressed by Formula (14) or (17) in an aprotic solvent such as toluene, benzene, xylene, acetonitrile, ethyl acetate, diethyl ether, t-butyl methyl ether, tetrahydrofuran, chloroform, dichloromethane, dimethylsulfoxide, dimethylformamide, or dimethylacetamide, or with no solvent and in the presence of an acid catalyst, to obtain an acetal structure expressed by Formula (15), (16), (18), or (19). The acid catalyst may be either an organic acid or an inorganic acid, and is not particularly limited, and specific examples thereof include p-toluenesulfonic acid, pyridinium p-toluenesulfonate, methanesulfonic acid, 10-camphorsulfonic acid, hydrogen chloride, iodine, ammonium chloride, oxalic acid, and boron trifluoride diethyl ether complex. Furthermore, a dehydrating agent may be added to a reaction system to remove water molecules generated in the reaction, and a type of the dehydrating agent is not particularly limited as long as it does not interfere with the reaction, and examples of the dehydrating agent include sodium sulfate, magnesium sulfate, alumina, silica gel, and molecular sieves, with the molecular sieves being preferred.

After the reaction step of Reactions 2-1 to 2-4, impurities produced as by-products in the reaction, remaining compounds that are not consumed in the reaction, and base catalysts are preferably removed by a purification step, and a purification method is not particularly limited, and purification can be performed by extraction, recrystallization, adsorption treatment, reprecipitation, column chromatography, supercritical extraction, or the like.

Especially, between the acetalization step and the step of introducing a leaving group structure to the hydroxy group, a deprotection step of deprotecting an amino group protected by a protecting group in the coupling product of Formulas (5) and (6) and a step of introducing a group capable of reacting with a biofunctional molecule into the amino group deprotected after the deprotection step may be provided. Conditions for the deprotection step of the protecting group and the step of introducing a group capable of reacting with a biofunctional molecule into the deprotected amino group can be found in many general textbooks. The deprotection step can be performed, for example, according to "Wuts, P. G. M.; Greene, T. W. Protective Groups in Organic Synthesis, 4th ed.; Wiley-Interscience: New York, 2007", and the step of introducing a group capable of reacting with a biofunctional molecule into the deprotected amino group can be performed, for example, according to "Greg T. Hermanso, Bioconjugate Techniques, 3rd ed.".

The leaving group structure introduction step of introducing a leaving group structure in place of a terminal hydroxy group after the acetalization step or the step of introducing a group capable of reacting with a biofunctional molecule into the deprotected amino group is a step of converting a hydroxy group in the polyoxyethylene derivative expressed by Formula (15), (16), (18), or (19) into a succinimidyloxycarbonyloxy group, a phthalimidyloxycarbonyloxy group, a 4-nitrophenoxycarbonyloxy group, a 1-imidazolylcarbonyloxy group, a pentafluorophenoxycarbonyloxy group, a benzotriazol-1-yloxycarbonyloxy group, or a 7-azabenzotriazol-1-yloxycarbonyloxy group, and a purification step may be performed after the reaction step.

The conversion of the hydroxy group is performed by condensing the polyoxyethylene derivative expressed by Formula (15), (16), (18), or (19) with a reagent for converting into, for example, each leaving group as described in Table 1, in an aprotic solvent such as toluene, benzene, xylene, acetonitrile, ethyl acetate, diethyl ether, t-butyl methyl ether, tetrahydrofuran, chloroform, dichloromethane, dimethyl sulfoxide, dimethylformamide, or dimethylacetamide, or with no solvent and in the presence of an organic base such as triethylamine, N-methylmorpholine, pyridine, or 4-dimethylaminopyridine or an inorganic base such as sodium carbonate, sodium hydrogencarbonate, sodium acetate, or potassium carbonate. Proportions of the reagents and base catalysts described in Table 1 to be used are not particularly limited, and are preferably equimolar or more relative to the hydroxy groups of the polyoxyethylene derivative of Formula (15), (16), (18) or (19). The reagents shown in Table 1 may be commercially available products or may be produced using known

reactions.

[Table 1]

| Leaving group structure (-OC(O)$E^1$) | Reagent |
|---|---|
| Succinimidyloxycarbonyloxy group | 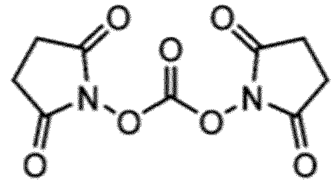 |
| Phthalimidyloxycarbonyloxy group | 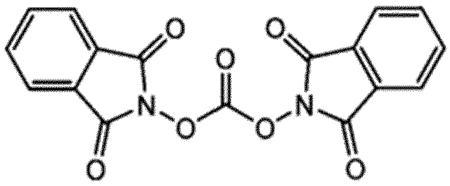 |
| 4-nitrophenoxycarbonyloxy group | 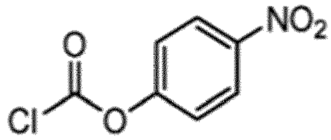 |
| 1-imidazolylcarbonyloxy group | 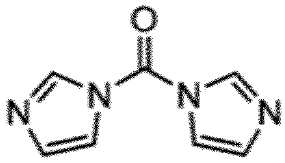 |
| Pentafluorophenoxycarbonyloxy group | 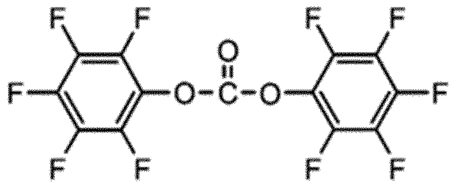 |
| Benzotriazol-1-yloxycarbonyloxy group | 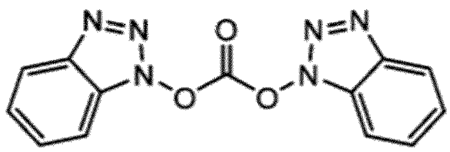 |
| 7-azabenzotriazol-1-yloxycarbonyloxy group | 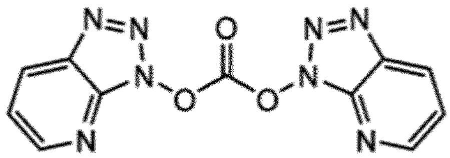 |

After the reaction step of the leaving group structure introduction step, impurities produced as by-products in the reaction, remaining compounds that are not consumed in the reaction, and base catalysts are preferably removed by a purification step, and a purification method is not particularly limited, and purification can be performed by extraction, recrystallization, adsorption treatment, reprecipitation, column chromatography, supercritical extraction, or the like.

An acetal-type releasable polyoxyethylene conjugate according to the present invention is obtained by reacting the -OC(O)$E^1$ group of the acetal-type releasable polyethylene glycol derivative with an amino group contained in a biofunctional molecule, and is expressed by the following Formula (7), (8), (9) or (10).

[Chem. 33]

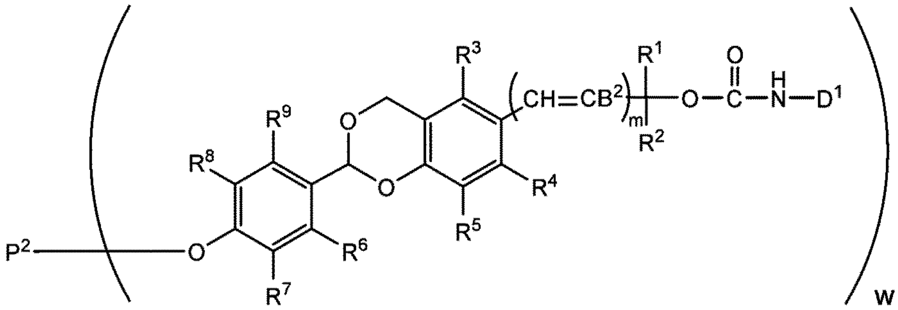

(7)

[Chem. 34]

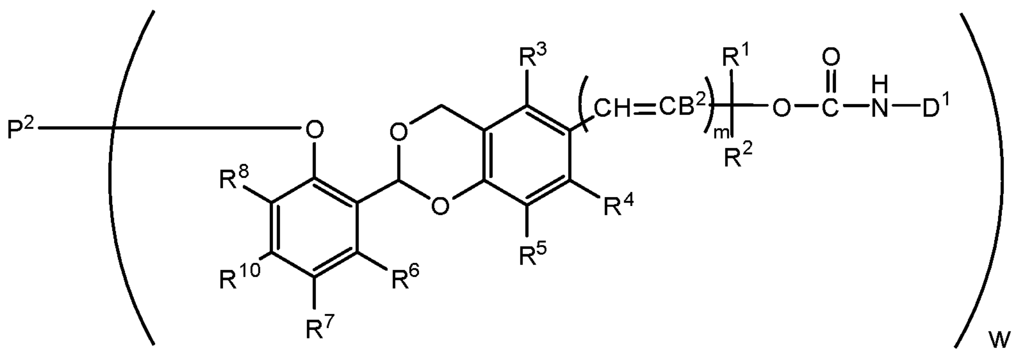

(8)

[Chem. 35]

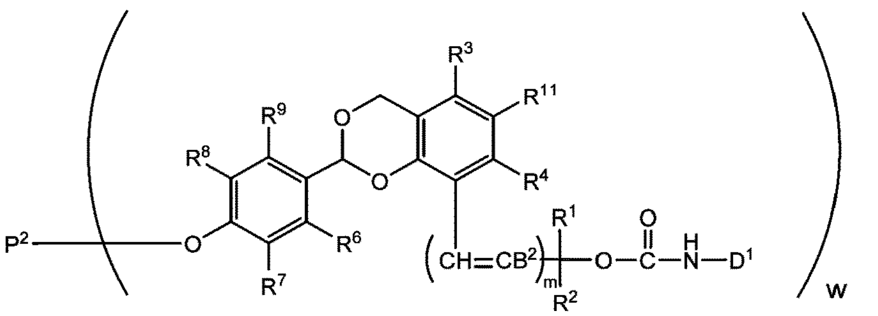

(9)

[Chem. 36]

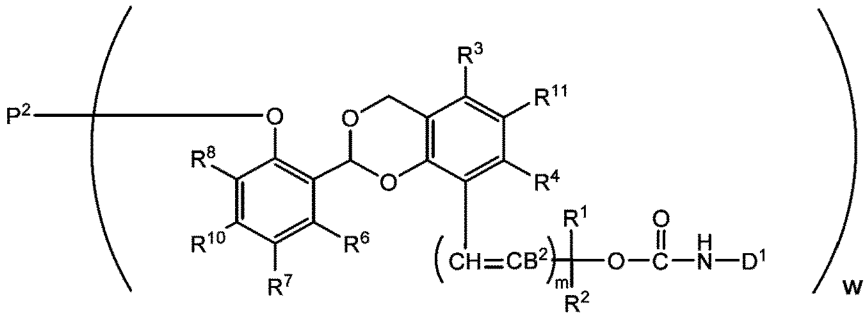

(10)

In Formulas (7), (8), (9) and (10),

$B^2$ represents a hydrogen atom or $-C(R^1)(R^2)OC(O)NHD^1$,
$D^1$ represents a residue obtained by removing an amino group that constitutes a carbamate bond from an amino group contained in a biofunctional molecule,
$P^2$ represents a polyoxyethylene derivative having a dehydroxylated group, or a conjugate of a biofunctional molecule and a polyoxyethylene derivative having a dehydroxylated group,
w represents an integer of 1 to 8,
$R^1$, $R^2$, $R^3$, $R^4$, $R^5$ and $R^{11}$ each independently represent a hydrocarbon group having 1 to 10 carbon atoms or a hydrogen atom,
$R^6$, $R^7$, $R^8$, $R^9$ and $R^{10}$ each independently represent an electron-withdrawing substituent, an electron-donating substituent, or a hydrogen atom, and
m represents 0 or 1.

In Formulas (7) to (10), details of $R^1$ to $R^{11}$ and m are the same as those described above.
In Formulas (7) to (10), $B^2$ represents a hydrogen atom or $-C(R^1)(R^2)OC(O)NHD^1$, and preferably represents a hydrogen atom.
In Formulas (7) to (10), $D^1$ represents a residue obtained by removing an amino group that constitutes a carbamate bond from amino groups contained in a biofunctional molecule.
The biofunctional molecule is not particularly limited as long as it is a substance involved in diagnosis, cure, mitigation, treatment or prevention of a disease in humans or other animals. Specific examples thereof include proteins, peptides, nucleic acids, cells, and viruses, and preferred examples of proteins or peptides include hormones, cytokines, antibodies, aptamers, and enzymes.
More specifically, examples of cytokines include interferon types I, II, and III, which regulate immunity, interleukins, tumor necrosis factors, and receptor antagonists thereof. Examples of growth factors include erythropoietin as a hematopoietic factor, and granulocyte-colony stimulating factor (GCSF) as a stimulating factor, and examples of blood coagulation factors include factor V, factor VII, factor VIII, factor IX, factor X, and factor XII. Examples of hormones include calcitonin, insulin and its analogs, exenatide, GLP-1, somatostatin, and human growth hormone. Examples of antibodies include full-length antibodies, examples of antibody fragments include Fab and svFV, examples of aptamers include DNA aptamers and RNA aptamers, and examples of enzymes include superoxide dismutase and uricase.
Examples of suitable proteins include interferons, interleukins, erythropoietin, GCSF, factor VIII, factor IX, human growth hormone, and antibody fragments, with the human growth hormone, interferon, GCSF, erythropoietin, or antibody fragments (particularly Fab) being more preferred.
Examples of suitable peptides include insulin, bivalirudin, teriparatide, exenatide, enfuvirtide, degarelix, mifamurtide, nesiritide, goserelin, glatiramer, octreotide, lanreotide, icatibant, zicotinide, pramlintide, romiplostim, calcitonin, oxytocin, leuprorelin, and glucagon, with the insulin, exenatide, and calcitonin (especially salmon calcitonin) being more preferred.
In Formulas (7) to (10), $P^2$ represents a polyoxyethylene derivative having a dehydroxylated group, or a conjugate of a polyoxyethylene derivative having a dehydroxylated group and a biofunctional molecule.
Detailed description of the polyoxyethylene derivative having a dehydroxylated group is the same as that for $P^1$ of the acetal-type releasable polyoxyethylene derivative.
The conjugate of the polyoxyethylene derivative having a dehydroxylated group and a biofunctional molecule is a

group in which $X^1$ of $P^1$ of the acetal-type releasable polyoxyethylene derivative is replaced with $D^2$, and $D^2$ is a group formed by the reaction of a group capable of reacting with a biofunctional molecule with a biofunctional molecule. Details of the group capable of reacting with a biofunctional molecule are the same as those for $X^1$ of the acetal-type releasable polyoxyethylene derivative, and the biofunctional molecule is not particularly limited as long as it is a substance involved in diagnosis, cure, mitigation, treatment or prevention of a disease in humans or other animals. Specific examples thereof include proteins, peptides, nucleic acids, cells, and viruses, and preferred examples of proteins or peptides include hormones, cytokines, antibodies, aptamers, and enzymes.

Suitable examples of the biofunctional molecule in $D^2$ include target-directed biofunctional molecules, such as antibodies and aptamers, examples of antibodies include antibody fragments such as Fab, Fab', and $F(ab')_2$, and examples of aptamers include a peptide aptamer, an RNA aptamer, and a DNA aptamer.

A production method of the acetal-type releasable polyoxyethylene conjugate according to the present invention will now be described. To produce the polyoxyethylene conjugate according to the present invention, a coupling step of reacting the acetal-type polyoxyethylene derivative with a biofunctional molecule in a neutral or basic buffer solution which may contain a water-soluble organic solvent such as acetonitrile, dimethylsulfoxide or N,N-dimethylformamide to obtain the polyoxyethylene conjugate, and a purification step of removing the unreacted polyoxyethylene derivative, biofunctional molecule or by-products under a basic condition after the coupling step are performed. Neutral or basic means a pH of 6.5 to 11.0, preferably a pH of 7.0 to 10.5, more preferably a pH of 7.0 to 10.0, and particularly preferably a pH of 7.0 to 9.0.

A neutral or basic buffer solution is an aqueous solution that has a buffer action that mitigates effects of adding a small amount of acid or base from outside, or changing of concentration by diluting, thereby keeping the pH (hydrogen ion exponent) at a nearly constant neutral or basic level.

A water-soluble organic solvent may also be added to the buffer solution. In this case, examples of the water-soluble organic solvent include methanol, ethanol, propanol, isopropanol, tetrahydrofuran, acetone, acetonitrile, dimethyl sulfoxide, N,N-dimethylformamide, triethylamine, pyridine, and hexamethylphosphoric triamide, with the methanol, ethanol, propanol, isopropanol, tetrahydrofuran, acetone, acetonitrile, dimethyl sulfoxide, or N,N-dimethylformamide being preferred, and the acetonitrile, dimethyl sulfoxide, and N,N-dimethylformamide being more preferred.

Examples of a specific method of the purification step performed after the coupling step include ion exchange chromatography, gel filtration chromatography, hydrophobic interaction chromatography, reverse phase chromatography, and affinity chromatography.

In the purification step, the unreacted polyoxyethylene derivative, the biofunctional molecule or the by-product can be removed under a neutral or basic condition under which the acetal structure in the acetal-type releasable polyoxyethylene conjugate is not easily hydrolyzed, and the neutral or basic condition refers to a pH of 6.5 to 11.0, preferably a pH of 7.0 to 10.5, more preferably a pH of 7.0 to 10.0, and particularly preferably a pH of 7.0 to 9.0.

## EXAMPLES

The present invention will be described in more detail below based on Examples, but the present invention is not limited to the following Examples.

In the following Examples, $^1$H-NMR was obtained from JNM-ECP400 or JNM-ECA600 manufactured by JEOL Ltd. A φ5 mm tube was used for measurement, and a deuterated solvent was $D_2O$ or $CDCl_3$ or d6-DMSO containing tetramethylsilane (TMS) as an internal standard substance. A molecular weight and a purity of the terminal functional group of the obtained acetal-type releasable polyoxyethylene conjugate were calculated by liquid chromatography (GPC and HPLC). A system of liquid chromatography used was "HLC-8320GPC EcoSEC" manufactured by Tosoh Corporation for GPC and "Thermo Ultimate 3000" manufactured by Thermo Fisher Scientific for HPLC.

### (Example 1-1)

To a 300 mL four-neck flask equipped with a thermometer, a nitrogen inlet tube, a stirrer, a Dean-Stark tube, and a cooling tube, SUNBRIGHT MEH-20T (40 g, 2.0 mmol) manufactured by NOF Corporation, toluene (120 g), and 2,6-di-tert-butyl-p-cresol (8 mg) were added, and water was removed azeotropically with toluene. After being cooled to 45°C, chloroform (200 g) was added, and then cooled to 25°C, and 4-hydroxybenzaldehyde (977 mg, 8.0 mmol) and triphenylphosphine (2.10 g, 8.0 mmol) were added. After stirring for a while, diisopropyl azodicarboxylate (1.51 g, 7.0 mmol) was added in three times so that an internal temperature did not exceed 35°C. After reacting at 25°C for 1 hour, methanol (224 mg, 7.0 mmol) was added, and the mixture was stirred for 30 minutes, and then the solvent was distilled off under reduced pressure. The residue was dissolved in ethyl acetate (320 g), and hexane (240 g) was added to cause crystallization, followed by suction filtration to obtain crystals. Furthermore, the obtained crystals were dissolved in ethyl acetate (320 g) containing 2,6-di-tert-butyl-p-cresol (64 mg), and then hexane (16 g) was added to repeat the crystallization operation to remove low molecular weight impurities. The crystals were washed with hexane (160 g), filtered, and dried under reduced pressure to obtain the compound of Formula (20).

$^1$H-NMR ($CDCl_3$, internal standard TMS); δ (ppm):

3.38 (3H, s, $-(OCH_2CH_2)_nOCH_3$), 3.52 to 4.23 (m, $-(OCH_2CH_2)_n-$), 7.03 (2H, d, arom.H), 7.83 (2H, d, arom.H), 9.89 (1H, s, -COH)
Number average molecular weight (Mn): 20,451

[Chem. 37]

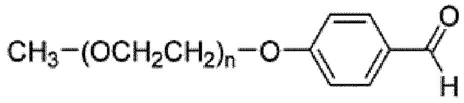

n = APPROXIMATELY 455 (20)

(Example 1-2)

To a 50 mL three-neck flask equipped with a thermometer, a nitrogen inlet tube, a stirrer and a cooling tube, the compound of Formula (20) (1.0 g, 0.05 mmol), 2,4-bis(hydroxymethyl)-p-cresol (336 mg, 2.0 mmol), tetrahydrofuran (5.0 g) and 2,6-di-tert-butyl-p-cresol (1.1 mg) were added, and after dissolution, a molecular sieve 5A (1.0 g) and p-toluenesulfonic acid monohydrate (11.4 mg, 0.06 mmol) were added and reacted at 40°C for 4 hours. N-methylmorpholine (12.1 mg, 0.12 mmol) was added and stirred for a while, and then filtered, and the filtrate was diluted with chloroform (10 g). After repeatedly washing with 20% saline (10 g) of pH 12, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added to repeat the crystallization operation to remove low molecular weight impurities, and then the compound of Formula (21) was obtained after drying under reduced pressure.
$^1$H-NMR ($CDCl_3$, internal standard TMS); δ (ppm):

2.27 (1H, t, -OH), 2.36 (3H, s, $-CH_3$), 3.38 (3H, s, $-(OCH_2CH_2)_nOCH_3$), 3.52 to 4.17 (m, $-(OCH_2CH_2)_n-$), 4.68 (2H, dd, $-CH_2OH$), 4.94 (1H, d, $-CH_2O-$), 5.15 (1H, d, $-CH_2O-$), 5.96 (1H, s, -CH<), 6.77 (1H, s, arom.H), 6.97 (2H, d, arom.H), 7.04 (1H, s, arom.H), 7.50 (2H, d, arom.H)
Number average molecular weight (Mn): 20,435

[Chem. 38]

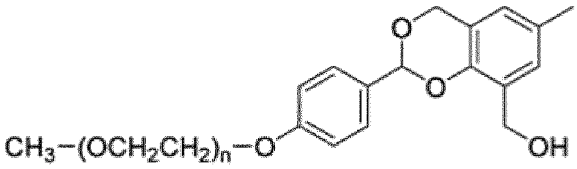

n = APPROXIMATELY 455 (21)

(Example 1-3)

To a 50 mL three-neck flask equipped with a thermometer, a nitrogen inlet tube, and a stirrer, the compound of Formula (21) (250 mg, 0.0125 mmol) and dichloromethane (3.6 g) were added and dissolved, and then pyridine (23.7 mg, 0.30 mmol) and p-nitrophenyl chloroformate (40.3 mg, 0.20 mmol) were added and reacted at 25°C for 3 hours. After repeatedly washing with 20% saline (2.5 g) of pH 12 to remove some impurities, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added for crystallization, followed by filtration and drying under reduced pressure, and then the compound of Formula (22) was obtained.
$^1$H-NMR ($CDCl_3$, internal standard TMS); δ (ppm):

2.31 (3H, s, $-CH_3$), 3.38 (3H, s, $-(OCH_2CH_2)_nOCH_3$), 3.52 to 4.17 (m, $-(OCH_2CH_2)_n-$), 4.96 (1H, d, $-CH_2O-$), 5.16 (1H, d, $-CH_2O-$), 5.35 (2H, dd, $-CH_2OCO-$), 5.98 (1H, s, -CH<), 6.87 (1H, s, arom.H), 6.94 (2H, d, arom.H), 7.11 (1H, s, arom.H), 7.26 to 7.29 (2H, m, arom.H), 7.52 (2H, d, arom.H), 8.24 (2H, d, arom.H)
Number average molecular weight (Mn): 20,204

[Chem. 39]

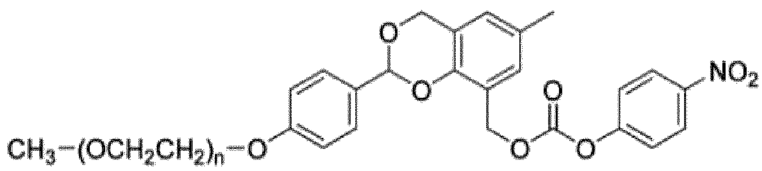

n = APPROXIMATELY 455 (22)

(Example 2-1)

To a 300 mL four-neck flask equipped with a thermometer, a nitrogen inlet tube, a stirrer, a Dean-Stark tube, and a cooling tube, SUNBRIGHT MEH-20T (40 g, 2.0 mmol) manufactured by NOF Corporation, toluene (120 g), and 2,6-di-tert-butyl-p-cresol (8 mg) were added, and water was removed azeotropically with toluene. After being cooled to 45°C, chloroform (200 g) was added, and then cooled to 25°C, and 3-fluoro-4-hydroxybenzaldehyde (1.12 g, 8.0 mmol) and triphenylphosphine (2.62 g, 10.0 mmol) were added. After stirring for a while, diisopropyl azodicarboxylate (1.84 g, 8.6 mmol) was added in three times so that an internal temperature did not exceed 35°C. After reacting at 25°C for 2 hour, methanol (276 mg, 8.6 mmol) was added, and the mixture was stirred for 30 minutes, and then the solvent was distilled off under reduced pressure. The residue was dissolved in ethyl acetate (320 g), and hexane (240 g) was added to cause crystallization, followed by suction filtration to obtain crystals. Furthermore, the obtained crystals were dissolved in ethyl acetate (320 g) containing 2,6-di-tert-butyl-p-cresol (64 mg), and then hexane (160 g) was added to repeat the crystallization operation to remove low molecular weight impurities. The crystals were washed with hexane (160 g), filtered, and dried under reduced pressure to obtain the compound of Formula (23).

$^1$H-NMR ($CDCl_3$, internal standard TMS); δ (ppm):

3.38 (3H, s, $-(OCH_2CH_2)_nOCH_3$), 3.52 to 4.30 (m, $-(OCH_2CH_2)_n-$), 7.13 (1H, t, -CH<), 7.60 to 7.65 (2H, m, arom.H), 9.86 (1H, s, -COH)
Number average molecular weight (Mn): 20,201

[Chem. 40]

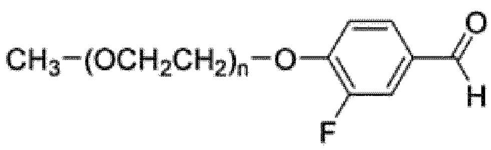

n = APPROXIMATELY 455 (23)

(Example 2-2)

To a 50 mL three-neck flask equipped with a thermometer, a nitrogen inlet tube, a stirrer and a cooling tube, the compound of Formula (23) (1.0 g, 0.05 mmol), 2,4-bis(hydroxymethyl)-p-cresol (336 mg, 2.0 mmol), tetrahydrofuran (5.0 g) and 2,6-di-tert-butyl-p-cresol (1.1 mg) were added, and after dissolution, a molecular sieve 5A (1.0 g) and p-toluenesulfonic acid monohydrate (11.4 mg, 0.06 mmol) were added and reacted at 40°C for 4 hours. N-methylmorpholine (12.1 mg, 0.12 mmol) was added and stirred for a while, and then filtered, and the filtrate was diluted with chloroform (10 g). After repeatedly washing with 20% saline (10 g) of pH 12, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added to repeat the crystallization operation to remove low

molecular weight impurities, and then the compound of Formula (24) was obtained after drying under reduced pressure.

$^1$H-NMR ($CDCl_3$, internal standard TMS); δ (ppm):

2.20 (1H, t, -OH), 2.29 (3H, s, $-CH_3$), 3.38 (3H, s, $-(OCH_2CH_2)_nOCH_3$), 3.52 to 4.24 (m, $-(OCH_2CH_2)_n-$), 4.68 (2H, dd, $-CH_2OH$), 4.94 (1H, d, $-CH_2O-$), 5.14 (1H, d, $-CH_2O-$), 5.93 (1H, s, -CH<), 6.77 (1H, s, arom.H), 7.04(1H, t, arom.H), 7.06 (1H, s, arom.H), 7.34 to 7.26 (2H, m, arom.H)
Number average molecular weight (Mn): 20,445

[Chem. 41]

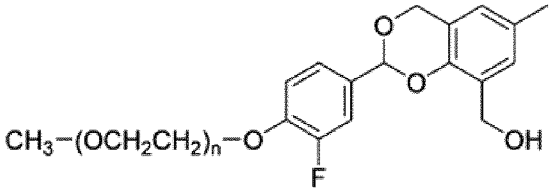

n = APPROXIMATELY 455 (24)

(Example 2-3)

To a 50 mL three-neck flask equipped with a thermometer, a nitrogen inlet tube, and a stirrer, the compound of Formula (24) (180 mg, 0.009 mmol) and dichloromethane (2.4 g) were added and dissolved, and then pyridine (17.1 mg, 0.216 mmol) and p-nitrophenyl chloroformate (29.0 mg, 0.144 mmol) were added and reacted at 25°C for 3 hours. After repeatedly washing with 20% saline (1.8 g) of pH 12 to remove some impurities, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added for crystallization, followed by filtration and drying under reduced pressure, and then the compound of Formula (25) was obtained.

$^1$H-NMR ($CDCl_3$, internal standard TMS); δ (ppm):

2.32 (3H, s, $-CH_3$), 3.38 (3H, s, $-(OCH_2CH_2)_nOCH_3$), 3.52 to 4.24 (m, $-(OCH_2CH_2)_n-$), 4.96 (1H, d, $-CH_2O-$), 5.15(1H, d, $-CH_2O-$), 5.34 (2H, dd, $-CH_2OCO-$), 5.96 (1H, s, -CH<), 6.87 (1H, s, arom.H), 7.01 (1H, t, arom.H), 7.12(1H, s, arom.H), 7.26 to 7.36 (4H, m, arom.H), 8.26 (2H, d, arom.H)
Number average molecular weight (Mn): 20,298

[Chem. 42]

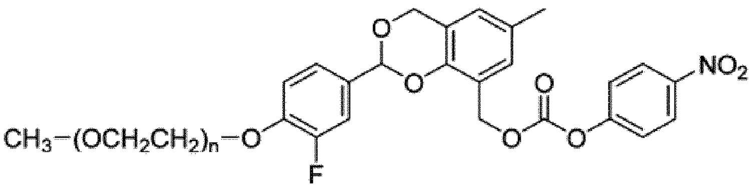

n = APPROXIMATELY 455 (25)

(Example 3-1)

To a 300 mL four-neck flask equipped with a thermometer, a nitrogen inlet tube, a stirrer, a Dean-Stark tube, and a cooling tube, SUNBRIGHT MEH-20T (40 g, 2.0 mmol) manufactured by NOF Corporation, toluene (120 g), and 2,6-di-tert-butyl-p-cresol (8 mg) were added, and water was removed azeotropically with toluene. After being cooled to 45°C, chloroform (200 g) was added, and then cooled to 25°C, and 4-hydroxy-2-methoxybenzaldehyde (1.22 g, 8.0 mmol) and triphenylphosphine (2.62 g, 10.0 mmol) were added. After stirring for a while, diisopropyl azodicarboxylate (1.84 g, 8.6 mmol) was added in three times so that an internal temperature did not exceed 35°C. After reacting at 25°C for 2 hour, methanol (276 mg, 8.6 mmol) was added, and the mixture was stirred for 30 minutes, and then the solvent was distilled off under reduced pressure. The residue was dissolved in ethyl acetate (320 g), and hexane (240 g) was added to cause

crystallization, followed by suction filtration to obtain crystals. The obtained crystals were dissolved in ethyl acetate (320 g) containing 2,6-di-tert-butyl-p-cresol (64 mg), and then hexane (160 g) was added to repeat the crystallization operation to remove low molecular weight impurities. The crystals were washed with hexane (160 g), filtered, and dried under reduced pressure to obtain the compound of Formula (26).

$^1$H-NMR ($CDCl_3$, internal standard TMS); δ (ppm):

3.38 (3H, s, $-(OCH_2CH_2)_nOCH_3$), 3.52 to 4.21 (m, $-(OCH_2CH_2)_n-$), 6.50 (1H, d, arom.H), 6.55 (1H, dd, arom.H), 7.79 (2H, d, arom.H), 10.3(1H, s, -COH)
Number average molecular weight (Mn): 20,216

[Chem. 43]

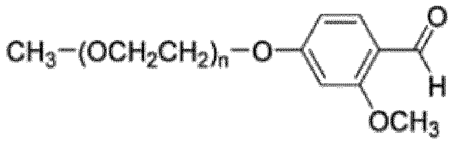

n = APPROXIMATELY 455 (26)

(Example 3-2)

To a 50 mL three-neck flask equipped with a thermometer, a nitrogen inlet tube, a stirrer and a cooling tube, the compound of Formula (26) (1.0 g, 0.05 mmol), 2,4-bis(hydroxymethyl)-p-cresol (336 mg, 2.0 mmol), tetrahydrofuran (5.0 g) and 2,6-di-tert-butyl-p-cresol (1.1 mg) were added, and after dissolution, a molecular sieve 5A (1.0 g) and p-toluenesulfonic acid monohydrate (11.4 mg, 0.06 mmol) were added and reacted at 40°C for 4 hours. N-methylmorpholine (12.1 mg, 0.12 mmol) was added and stirred for a while, and then filtered, and the filtrate was diluted with chloroform (10 g). After repeatedly washing with 20% saline (10 g) of pH 12 to remove some low molecular weight impurities, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added to repeat the crystallization operation to remove low molecular weight impurities, and then the compound of Formula (27) was obtained after drying under reduced pressure.

$^1$H-NMR ($CDCl_3$, internal standard TMS); δ (ppm):

2.28 (3H, s, $-CH_3$), 2.84 (1H, dd, -OH), 3.38 (3H, s, $-(OCH_2CH_2)_nOCH_3$), 3.52 to 4.17 (m, $-(OCH_2CH_2)_n-$), 4.47 (1H, dd, $-CH_2OH$), 4.75 (1H, dd, $-CH_2OH$), 4.98 (1H, d, $-CH_2O-$), 5.17 (1H, d, $-CH_2O-$), 6.20 (1H, s, -CH<), 6.57 to 6.55 (2H, m, arom.H), 6.78 (1H, s, arom.H), 6.97 (1H, s, arom.H), 7.58 (2H, d, arom.H)
Number average molecular weight (Mn): 20,215

[Chem. 44]

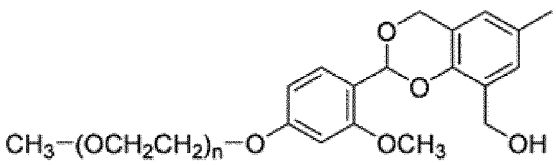

n = APPROXIMATELY 455 (27)

(Example 3-3)

To a 50 mL three-neck flask equipped with a thermometer, a nitrogen inlet tube, and a stirrer, the compound of Formula (27) (300 mg, 0.015 mmol) and dichloromethane (4.0 g) were added and dissolved, and then pyridine (28.5 mg, 0.360 mmol) and p-nitrophenyl chloroformate (48.4 mg, 0.240 mmol) were added and reacted at 25°C for 3 hours. After repeatedly washing with 20% saline (3.0 g) of pH 12 to remove some low molecular weight impurities, an organic layer was

dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added for crystallization, followed by filtration and drying under reduced pressure, and then the compound of Formula (28) was obtained.

$^1$H-NMR ($CDCl_3$, internal standard TMS); δ (ppm):

2.31 (3H, s, $-CH_3$), 3.38 (3H, s, $-(OCH_2CH_2)_nOCH_3$), 3.52 to 4.17 (m, $-(OCH_2CH_2)_n-$), 4.93 (1H, d, $-CH_2O-$), 5.18 (1H, d, $-CH_2O-$), 5.33 (2H, dd, $-CH_2OCO-$), 6.26 (1H, s, -CH<), 6.52 to 6.54 (2H, m, arom.H), 6.87 (1H, s, arom.H), 7.10 (1H, s, arom.H), 7.26 to 7.27 (2H, m, arom.H), 7.59 (1H, d, arom.H), 8.22 (2H, d, arom.H)

Number average molecular weight (Mn): 20,315

[Chem. 45]

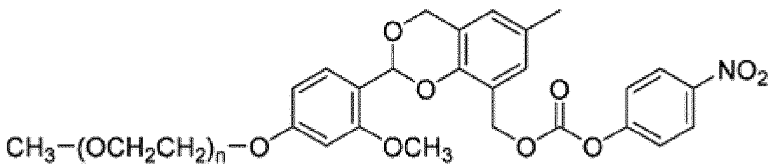

n = APPROXIMATELY 455 (28)

(Example 4-1)

To a 300 mL four-neck flask equipped with a thermometer, a nitrogen inlet tube, a stirrer, a Dean-Stark tube, and a cooling tube, SUNBRIGHT GL2-400HO (10 g, 0.25 mmol) manufactured by NOF Corporation, toluene (30 g), and 2,6-di-tert-butyl-p-cresol (2 mg) were added, and water was removed azeotropically with toluene. After being cooled to 45°C, chloroform (50 g) was added, and then cooled to 25°C, and 4-hydroxybenzaldehyde (244 mg, 2.0 mmol) and triphenylphosphine (524 mg, 2.0 mmol) were added. After stirring for a while, diisopropyl azodicarboxylate (376 g, 1.75 mmol) was added in three times so that an internal temperature did not exceed 35°C. After reacting at 25°C for 2 hour, methanol (56 mg, 1.75 mmol) was added, and the mixture was stirred for 30 minutes, and then the solvent was distilled off under reduced pressure. The residue was dissolved in ethyl acetate (80 g), and hexane (60 g) was added to cause crystallization, followed by suction filtration to obtain crystals. Furthermore, the obtained crystals were dissolved in ethyl acetate (80 g) containing 2,6-di-tert-butyl-p-cresol (16 mg), and then hexane (40 g) was added to repeat the crystallization operation to remove low molecular weight impurities. The crystals were washed with hexane (40 g), filtered, and dried under reduced pressure to obtain the compound of Formula (29).

$^1$H-NMR ($CDCl_3$, internal standard TMS); δ (ppm):

3.38 (6H, s, $-(OCH_2CH_2)_nOCH_3$), 3.52 to 3.92 (m, $-(OCH_2CH_2)_n-$), 4.13 (1H, dd, - $CH_2O-$), 4.21 (1H, dd, $-CH_2O-$), 7.03 (2H, d, arom.H), 7.83 (2H, d, arom.H), 9.89 (1H, s, -COH)

Number average molecular weight (Mn): 38,947

[Chem. 46]

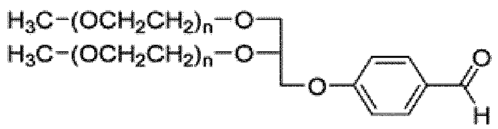

n = APPROXIMATELY 455 (29)

(Example 4-2)

To a 50 mL three-neck flask equipped with a thermometer, a nitrogen inlet tube, a stirrer and a cooling tube, the compound of Formula (29) (1.0 g, 0.025 mmol), 2,4-bis(hydroxymethyl)-p-cresol (336 mg, 2.0 mmol), tetrahydrofuran (5.0 g) and 2,6-di-tert-butyl-p-cresol (1.1 mg) were added, and after dissolution, a molecular sieve 5A (1.0 g) and p-toluenesulfonic acid monohydrate (17.1 mg, 0.09 mmol) were added and reacted at 40°C for 4 hours. N-methylmorpholine (12.1 mg, 0.12 mmol) was added and stirred for a while, and then filtered, and the filtrate was diluted with chloroform (20 g).

After repeatedly washing with 20% saline (20 g) of pH 12, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added to repeat the crystallization operation to remove low molecular weight impurities, and then the compound of Formula (30) was obtained after drying under reduced pressure.

$^1$H-NMR ($CDCl_3$, internal standard TMS); δ (ppm):

2.29 (3H, s, $-CH_3$), 2.35 (1H, t, -OH), 3.38 (6H, s, $-(OCH_2CH_2)_nOCH_3$), 3.52 to 3.91 (m, $-(OCH_2CH_2)_n-$), 4.07 (1H, dd, $-CH_2O-$), 4.14 (1H, dd, $-CH_2O-$), 4.68 (2H, dd, $-CH_2OH$), 4.94 (1H, d, $-CH_2O-$), 5.15 (1H, d, $-CH_2O-$), 5.96 (1H, s, -CH<), 6.77 (1H, s, arom.H), 6.97 (2H, d, arom.H), 7.04 (1H, s, arom.H), 7.50 (2H, d, arom.H)
Number average molecular weight (Mn): 39,110

[Chem. 47]

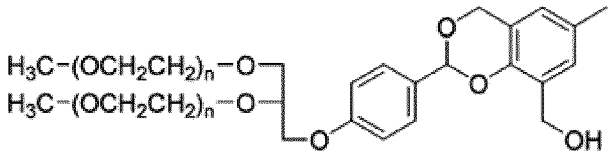

n = APPROXIMATELY 455 (30)

(Example 4-3)

To a 50 mL three-neck flask equipped with a thermometer, a nitrogen inlet tube, and a stirrer, the compound of Formula (30) (250 mg, 0.00625 mmol) and dichloromethane (3.6 g) were added and dissolved, and then pyridine (11.9 mg, 0.15 mmol) and p-nitrophenyl chloroformate (20.3 mg, 0.10 mmol) were added and reacted at 25°C for 3 hours. After repeatedly washing with 20% saline (2.5 g) of pH 12 to remove some impurities, an organic layer was dried with anhydrous sodium sulfate and filtered, and the solvent was distilled off under reduced pressure. The residue was dissolved in ethyl acetate (45 g) containing 2,6-di-tert-butyl-p-cresol (9 mg), and crystallization was performed by adding hexane (33 g), followed by suction filtration to obtain crystals. Furthermore, the crystals obtained were dissolved in ethyl acetate (90 g) containing 2,6-di-tert-butyl-p-cresol (9 mg), and then hexane (33 g) was added for crystallization, followed by filtration and drying under reduced pressure, and then the compound of Formula (31) was obtained.

$^1$H-NMR ($CDCl_3$, internal standard TMS); δ (ppm):

2.29 (3H, s, $-CH_3$), 3.38 (6H, s, $-(OCH_2CH_2)_nOCH_3$), 3.52 to 3.91 (m, $-(OCH_2CH_2)_n-$), 4.07 (1H, dd, $-CH_2O-$), 4.14 (1H, dd, $-CH_2O-$), 4.94 (1H, d, $-CH_2O-$), 5.16 (1H, d, $-CH_2O-$), 5.35 (1H, d, $-CH_2OCO-$), 5.98 (1H, s, -CH<), 6.87 (1H, s, arom.H), 6.94 (2H, d, arom.H), 7.11 (1H, s, arom.H), 7.26 to 7.29 (2H, m, arom.H), 7.52 (2H, d, arom.H), 8.24 (2H, d, arom.H)
Number average molecular weight (Mn): 39,456

[Chem. 48]

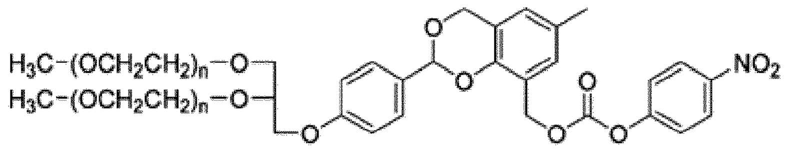

n = APPROXIMATELY 455 (31)

(Example 5-1)

To a 300 mL four-neck flask equipped with a thermometer, a nitrogen inlet tube, a stirrer, a Dean-Stark tube, and a cooling tube, SUNBRIGHT PTE-200HO (10 g, 0.5 mmol) manufactured by NOF Corporation, toluene (30 g), and 2,6-di-tert-butyl-p-cresol (2 mg) were added, and water was removed azeotropically with toluene. After being cooled to 45°C,

chloroform (50 g) was added, and then cooled to 25°C, and 4-hydroxybenzaldehyde (977 mg, 8.0 mmol) and triphenylphosphine (2.10 g, 8.0 mmol) were added. After stirring for a while, diisopropyl azodicarboxylate (1.51 g, 7.0 mmol) was added in three times so that an internal temperature did not exceed 35°C. After reacting at 25°C for 1 hour, methanol (224 mg, 7.0 mmol) was added, and the mixture was stirred for 30 minutes, and then the solvent was distilled off under reduced pressure. The residue was dissolved in ethyl acetate (320 g), and hexane (240 g) was added to cause crystallization, followed by suction filtration to obtain crystals. Furthermore, the obtained crystals were dissolved in ethyl acetate (80 g) containing 2,6-di-tert-butyl-p-cresol (64 mg), and then hexane (160 g) was added to repeat the crystallization operation to remove low molecular weight impurities. The crystals obtained after filtration were washed with hexane (160 g), filtered, and then dried under reduced pressure to obtain the compound of Formula (32).

$^1$H-NMR ($CDCl_3$, internal standard TMS); δ (ppm):
3.41 to 4.23 (m, $-(OCH_2CH_2)_n-$), 7.03 (8H, d, arom.H), 7.83 (8H, d, arom.H), 9.89 (4H, s, -COH)

[Chem. 49]

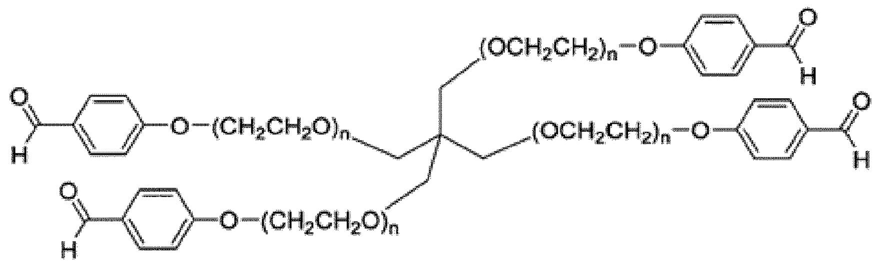

n = APPROXIMATELY 114 (32)

(Example 5-2)

To a 50 mL three-neck flask equipped with a thermometer, a nitrogen inlet tube, a stirrer and a cooling tube, the compound of Formula (32) (1.0 g, 0.05 mmol), 2,4-bis(hydroxymethyl)-p-cresol (1.34 g, 8 mmol), tetrahydrofuran (5.0 g) and 2,6-di-tert-butyl-p-cresol (1.1 mg) were added, and after dissolution, a molecular sieve 5A (1.0 g) and p-toluenesulfonic acid monohydrate (45.6 mg, 0.24 mmol) were added and reacted at 40°C for 4 hours. N-methylmorpholine (48.4 mg, 0.48 mmol) was added and stirred for a while, and then filtered, and the filtrate was diluted with chloroform (10 g). After repeatedly washing with 20% saline (10 g) of pH 12, an organic layer was dried with anhydrous sodium sulfate and filtered, and the solvent was distilled off under reduced pressure. The residue was dissolved in ethyl acetate (360 g) containing 2,6-di-tert-butyl-p-cresol (18 mg), and crystallization was performed by adding hexane (264 g), followed by suction filtration to obtain crystals. Furthermore, the crystals obtained were dissolved in ethyl acetate (360 g) containing 2,6-di-tert-butyl-p-cresol (72 mg), and then hexane (264 g) was added to repeat the crystallization operation to remove low molecular weight impurities, and then the compound of Formula (33) was obtained after drying under reduced pressure.

$^1$H-NMR ($CDCl_3$, internal standard TMS); δ (ppm):
2.27 (4H, t, -OH), 2.36 (12H, s, $-CH_3$), 3.41 to 4.23 (m, $-(OCH_2CH_2)_n-$), 4.68 (8H, dd, $-CH_2OH$), 4.94 (4H, d, $-CH_2O-$), 5.15 (4H, d, $-CH_2O-$), 5.96 (4H, s, -CH<), 6.77 (4H, s, arom.H), 6.97 (8H, d, arom.H), 7.04 (4H, s, arom.H), 7.50 (8H, d, arom.H)

[Chem. 50]

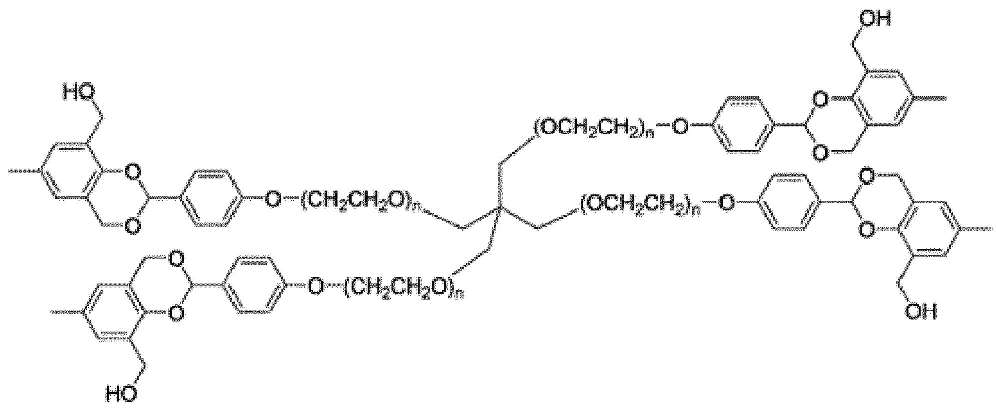

n = APPROXIMATELY 114 (33)

(Example 5-3)

To a 50 mL three-neck flask equipped with a thermometer, a nitrogen inlet tube, and a stirrer, the compound of Formula (33) (250 mg, 0.0125 mmol) and dichloromethane (3.6 g) were added and dissolved, and then pyridine (94.8 mg, 1.20 mmol) and p-nitrophenyl chloroformate (161 mg, 0.80 mmol) were added and reacted at 25°C for 3 hours. After repeatedly washing with 20% saline (2.5 g) of pH 12 to remove some low molecular weight impurities, an organic layer was dried with anhydrous sodium sulfate and filtered, and the solvent was distilled off under reduced pressure. The residue was dissolved in ethyl acetate (360 g) containing 2,6-di-tert-butyl-p-cresol (72 mg), and crystallization was performed by adding hexane (264 g), followed by suction filtration to obtain crystals. Furthermore, the crystals obtained were dissolved in ethyl acetate (360 g) containing 2,6-di-tert-butyl-p-cresol (72 mg), and then hexane (264 g) was added for crystallization, followed by filtration and drying under reduced pressure, and then the compound of Formula (34) was obtained.

$^1$H-NMR ($CDCl_3$, internal standard TMS); δ (ppm):
2.31 (12H, s, $-CH_3$), 3.41 to 4.23 (m, $-(OCH_2CH_2)_n-$), 4.96 (4H, d, $-CH_2O-$), 5.16 (4H, d, $-CH_2O-$), 5.35 (8H, dd, $-CH_2OCO-$), 5.98 (4H, s, -CH<), 6.87 (4H, s, arom.H), 6.94 (8H, d, arom.H), 7.11 (4H, s, arom.H), 7.26 to 7.29 (8H, m, arom.H), 7.52 (8H, d, arom.H), 8.24 (8H, d, arom.H)

[Chem. 51]

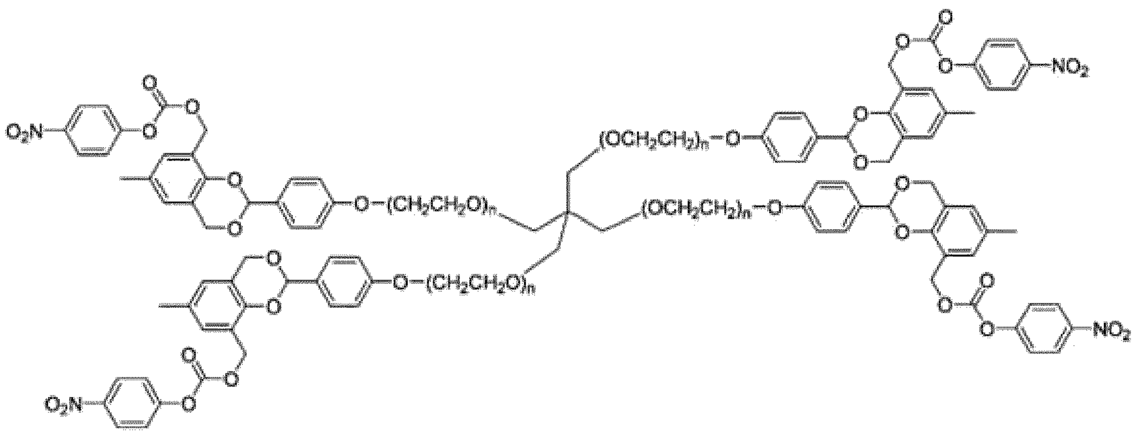

n = APPROXIMATELY 114 (34)

(Example 6-1)

To a 300 mL four-neck flask equipped with a thermometer, a nitrogen inlet tube, a stirrer, a Dean-Stark tube, and a cooling tube, SUNBRIGHT HGEO-400HO (10 g, 0.25 mmol) manufactured by NOF Corporation, toluene (30 g), and 2,6-di-tert-butyl-p-cresol (2 mg) were added, and water was removed azeotropically with toluene. After being cooled to 45°C,

chloroform (50 g) was added, and then cooled to 25°C, and 4-hydroxybenzaldehyde (977 mg, 8.0 mmol) and triphenylphosphine (2.10 g, 8.0 mmol) were added. After stirring for a while, diisopropyl azodicarboxylate (1.51 g, 7.0 mmol) was added in three times so that an internal temperature did not exceed 35°C. After reacting at 25°C for 1 hour, methanol (224 mg, 7.0 mmol) was added, and the mixture was stirred for 30 minutes, and then the solvent was distilled off under reduced pressure. The residue was dissolved in ethyl acetate (320 g), and hexane (240 g) was added to cause crystallization, followed by suction filtration to obtain crystals. Furthermore, the obtained crystals were dissolved in ethyl acetate (320 g) containing 2,6-di-tert-butyl-p-cresol (64 mg), and then hexane (160 g) was added to repeat the crystallization operation to remove low molecular weight impurities. The crystals were washed with hexane (160 g), filtered, and dried under reduced pressure to obtain the compound of Formula (35).

$^1$H-NMR ($CDCl_3$, internal standard TMS); δ (ppm):
2.98 to 4.23 (m, $-(OC\underline{H_2}C\underline{H_2})_n-$), 7.03 (16H, d, arom.H), 7.83 (16H, d, arom.H), 9.89 (8H, s, -CO$\underline{H}$)

[Chem. 52]

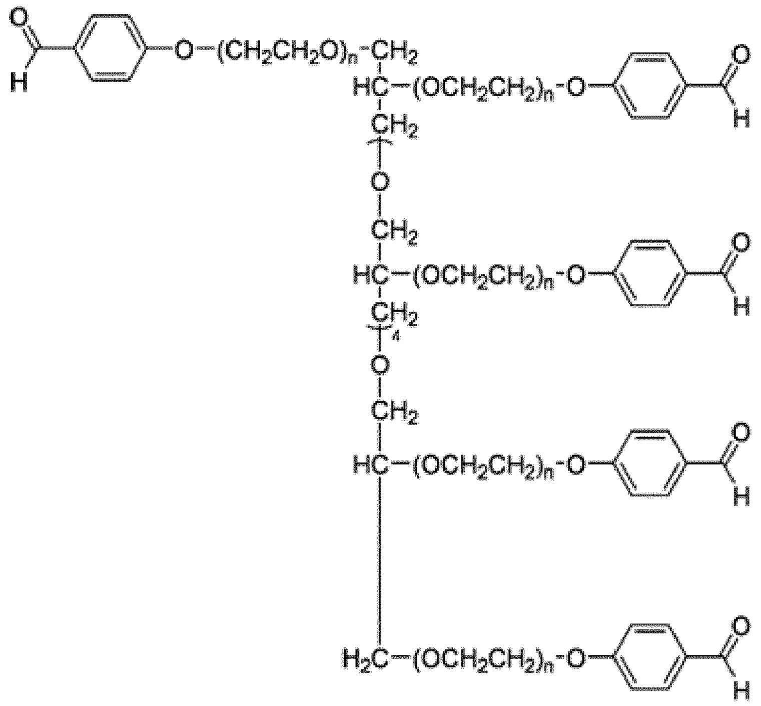

n = APPROXIMATELY 114 (35)

(Example 6-2)

To a 50 mL three-neck flask equipped with a thermometer, a nitrogen inlet tube, a stirrer and a cooling tube, the compound of Formula (35) (1.0 g, 0.025 mmol), 2,4-bis(hydroxymethyl)-p-cresol (1.34 g, 8.0 mmol), tetrahydrofuran (5.0 g) and 2,6-di-tert-butyl-p-cresol (1.1 mg) were added, and after dissolution, a molecular sieve 5A (1.0 g) and p-toluenesulfonic acid monohydrate (45.6 mg, 0.24 mmol) were added and reacted at 40°C for 4 hours. N-methylmorpholine (48.4 mg, 0.48 mmol) was added and stirred for a while, and then filtered, and the filtrate was diluted with chloroform (10 g). After repeatedly washing with 20% saline (10 g) of pH 12 to remove some low molecular weight impurities, an organic layer was dried with anhydrous sodium sulfate and filtered, and the solvent was distilled off under reduced pressure. The residue was dissolved in ethyl acetate (360 g) containing 2,6-di-tert-butyl-p-cresol (72 mg), and crystallization was performed by adding hexane (264 g), followed by suction filtration to obtain crystals. Furthermore, the crystals obtained were dissolved in ethyl acetate (360 g) containing 2,6-di-tert-butyl-p-cresol (72 mg), and then hexane (264 g) was added to repeat the crystallization operation to remove low molecular weight impurities, and then the compound of Formula (36) was obtained after drying under reduced pressure.

$^1$H-NMR ($CDCl_3$, internal standard TMS); δ (ppm):
2.27 (8H, t, -OH), 2.36 (24H, s, $-CH_3$), 2.98 to 4.23 (m, $-(OCH_2CH_2)_n-$), 4.68 (16H, dd, $-CH_2OH$), 4.94 (8H, d, $-CH_2O-$), 5.15 (8H, d, $-C\underline{H_2}O-$), 5.96 (8H, s, -CH<), 6.77 (8H, s, arom.H), 6.97 (16H, d, arom.H), 7.04 (8H, s, arom.H), 7.50 (16H, d,

arom.H)

[Chem. 53]

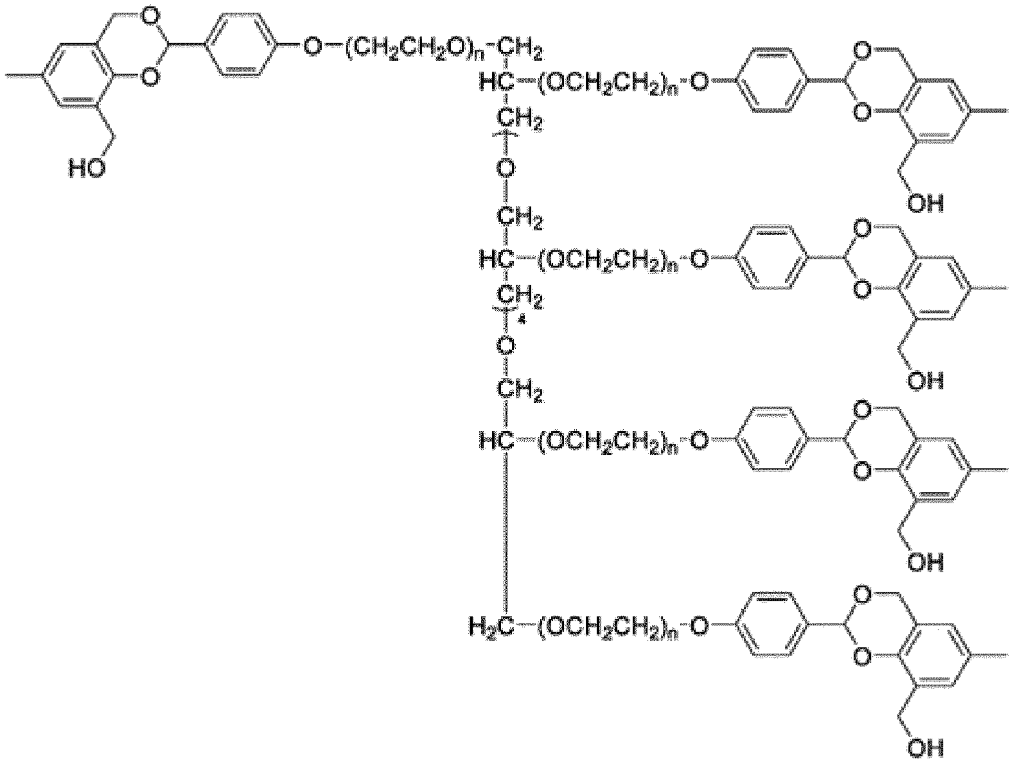

n = APPROXIMATELY 114 (36)

(Example 6-3)

To a 50 mL three-neck flask equipped with a thermometer, a nitrogen inlet tube, and a stirrer, the compound of Formula (36) (250 mg, 0.00625 mmol) and dichloromethane (3.6 g) were added and dissolved, and then pyridine (94.8 mg, 1.20 mmol) and p-nitrophenyl chloroformate (161.2 mg, 0.80 mmol) were added and reacted at 25°C for 3 hours. After repeatedly washing with 20% saline (2.5 g) of pH 12 to remove some low molecular weight impurities, an organic layer was dried with anhydrous sodium sulfate and filtered, and the solvent was distilled off under reduced pressure. The residue was dissolved in ethyl acetate (360 g) containing 2,6-di-tert-butyl-p-cresol (72 mg), and crystallization was performed by adding hexane (264 g), followed by suction filtration to obtain crystals. Furthermore, the crystals obtained were dissolved in ethyl acetate (360 g) containing 2,6-di-tert-butyl-p-cresol (72 mg), and then hexane (263 g) was added for crystallization, followed by filtration and drying under reduced pressure, and then the compound of Formula (37) was obtained.

$^1$H-NMR ($CDCl_3$, internal standard TMS); $\delta$ (ppm):
2.31 (24H, s, $-CH_3$), 2.98 to 4.23 (m, $-(OCH_2CH_2)_n-$), 4.96 (8H, d, $-CH_2O-$), 5.16 (8H, d, $-CH_2O-$), 5.35 (16H, dd, $-CH_2OCO-$), 5.98 (8H, s, -CH<), 6.87 (8H, s, arom.H), 6.94 (16H, d, arom.H), 7.11 (8H, s, arom.H), 7.26 to 7.29 (16H, m, arom.H), 7.52 (16H, d, arom.H), 8.24 (16H, d, arom.H)

[Chem. 54]

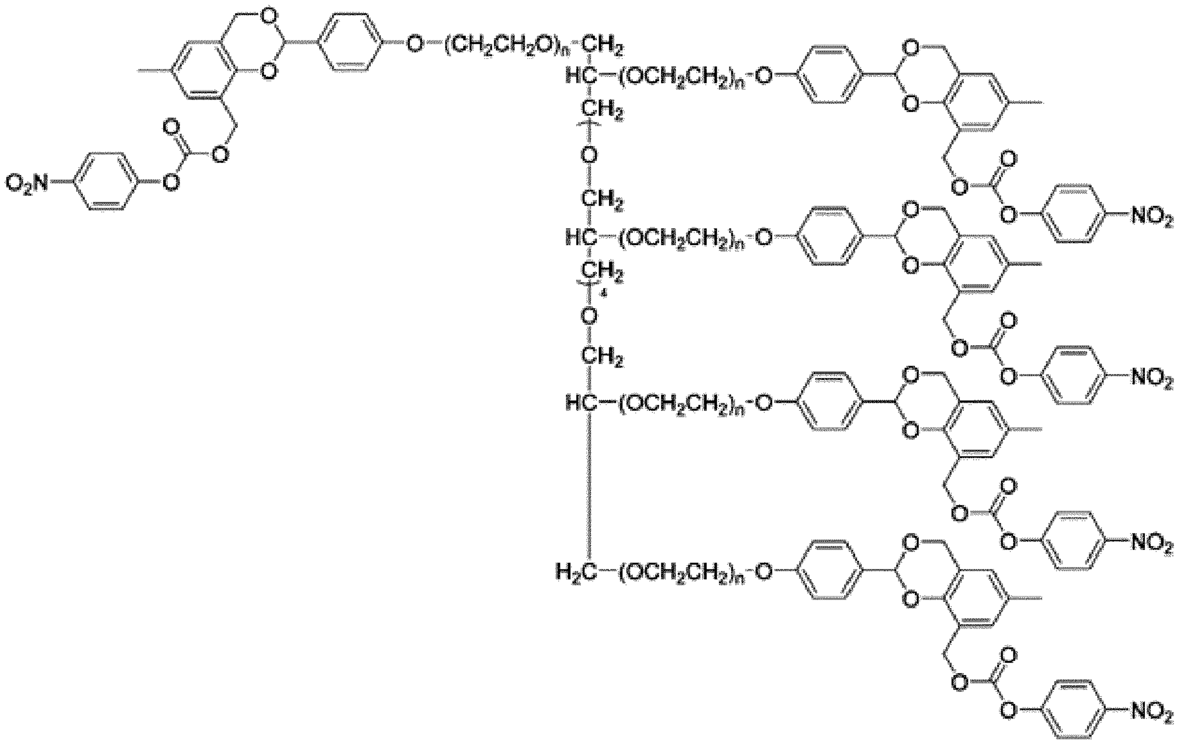

n = APPROXIMATELY 114 (37)

(Example 7-1)

To a 300 mL four-neck flask equipped with a thermometer, a nitrogen inlet tube, a stirrer, a Dean-Stark tube, and a cooling tube, SUNBRIGHT DKH-20T (20 g, 1.0 mmol) manufactured by NOF Corporation, toluene (60 g), and 2,6-di-tert-butyl-p-cresol (4 mg) were added, and water was removed azeotropically with toluene. After being cooled to 45°C, chloroform (100 g) was added, and then cooled to 25°C, and 4-hydroxybenzaldehyde (977 mg, 8.0 mmol) and triphenylphosphine (2.10 g, 8.0 mmol) were added. After stirring for a while, diisopropyl azodicarboxylate (1.51 g, 7.0 mmol) was added in three times so that an internal temperature did not exceed 35°C. After reacting at 25°C for 1 hour, methanol (224 mg, 7.0 mmol) was added, and the mixture was stirred for 30 minutes, and then the solvent was distilled off under reduced pressure. The residue was dissolved in ethyl acetate (320 g), and hexane (240 g) was added to cause crystallization, followed by suction filtration to obtain crystals. Furthermore, the obtained crystals were dissolved in ethyl acetate (320 g) containing 2,6-di-tert-butyl-p-cresol (64 mg), and then hexane (160 g) was added to repeat the crystallization operation to remove low molecular weight impurities. The crystals were washed with hexane (160 g), filtered, and dried under reduced pressure to obtain the compound of Formula (38).

$^1$H-NMR ($CDCl_3$, internal standard TMS); δ (ppm):
3.52 to 4.23 (m, $-(OC\underline{H_2}C\underline{H_2})_n-$), 7.03 (4H, d, arom.H), 7.83 (4H, d, arom.H), 9.89 (2H, s, -COH)

[Chem. 55]

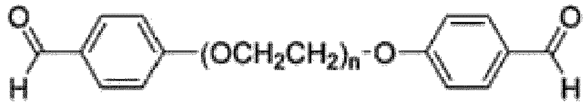

n = APPROXIMATELY 455 (38)

(Example 7-2)

To a 50 mL three-neck flask equipped with a thermometer, a nitrogen inlet tube, a stirrer and a cooling tube, the

compound of Formula (38) (1.0 g, 0.05 mmol), 2,4-bis(hydroxymethyl)-p-cresol (672 mg, 4.0 mmol), tetrahydrofuran (5.0 g) and 2,6-di-tert-butyl-p-cresol (1.1 mg) were added, and after dissolution, a molecular sieve 5A (1.0 g) and p-toluenesulfonic acid monohydrate (22.8 mg, 0.12 mmol) were added and reacted at 40°C for 4 hours. N-methylmorpholine (24.2 mg, 0.24 mmol) was added and stirred for a while, and then filtered, and the filtrate was diluted with chloroform (10 g). After repeatedly washing with 20% saline (10 g) of pH 12, an organic layer was dried with anhydrous sodium sulfate and filtered, and the solvent was distilled off under reduced pressure. The residue was dissolved in ethyl acetate (180 g) containing 2,6-di-tert-butyl-p-cresol (36 mg), and crystallization was performed by adding hexane (132 g), followed by suction filtration to obtain crystals. Furthermore, the crystals obtained were dissolved in ethyl acetate (180 g) containing 2,6-di-tert-butyl-p-cresol (36 mg), and then hexane (132 g) was added to repeat the crystallization operation to remove low molecular weight impurities, and then the compound of Formula (39) was obtained after drying under reduced pressure.

$^1$H-NMR ($CDCl_3$, internal standard TMS); δ (ppm):
2.27 (2H, t, -OH), 2.36 (6H, s, $-CH_3$), 3.52 to 4.17 (m, $-(OCH_2CH_2)_n-$), 4.68 (4H, dd, - $CH_2OH$), 4.94 (2H, d, $-CH_2O-$), 5.15 (2H, d, $-CH_2O-$), 5.96 (2H, s, -CH<), 6.77 (2H, s, arom.H), 6.97 (4H, d, arom.H), 7.04 (2H, s, arom.H), 7.50 (4H, d, arom.H)

[Chem. 56]

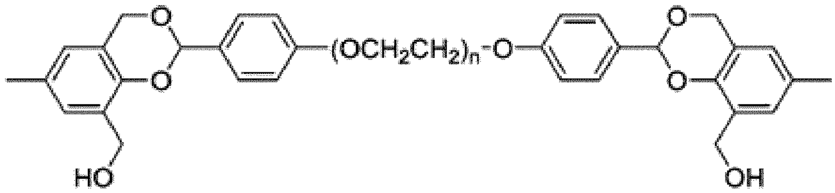

n = APPROXIMATELY 455 (39)

(Example 7-3)

To a 50 mL three-neck flask equipped with a thermometer, a nitrogen inlet tube, and a stirrer, the compound of Formula (39) (250 mg, 0.0125 mmol) and dichloromethane (3.6 g) were added and dissolved, and then pyridine (47.4 mg, 0.60 mmol) and p-nitrophenyl chloroformate (80.6 mg, 0.40 mmol) were added and reacted at 25°C for 3 hours. After repeatedly washing with 20% saline (2.5 g) of pH 12, an organic layer was dried with anhydrous sodium sulfate and filtered, and the solvent was distilled off under reduced pressure. The residue was dissolved in ethyl acetate (180 g) containing 2,6-di-tert-butyl-p-cresol (36 mg), and crystallization was performed by adding hexane (132 g), followed by suction filtration to obtain crystals. Furthermore, the crystals obtained were dissolved in ethyl acetate (180 g) containing 2,6-di-tert-butyl-p-cresol (36 mg), and then hexane (132 g) was added for crystallization, followed by filtration and drying under reduced pressure, and then the compound of Formula (40) was obtained.

$^1$H-NMR ($CDCl_3$, internal standard TMS); δ (ppm):
2.31 (6H, s, $-CH_3$), 3.52 to 4.17 (m, $-(OCH_2CH_2)_n-$), 4.96 (2H, d, $-CH_2O-$), 5.16 (2H, d, $-CH_2O-$), 5.35 (4H, dd, $-CH_2OCO-$), 5.98 (2H, s, -CH<), 6.87 (2H, s, arom.H), 6.94 (4H, d, arom.H), 7.11 (2H, s, arom.H), 7.26 to 7.29 (4H, m, arom.H), 7.52 (4H, d, arom.H), 8.24 (4H, d, arom.H)

[Chem. 57]

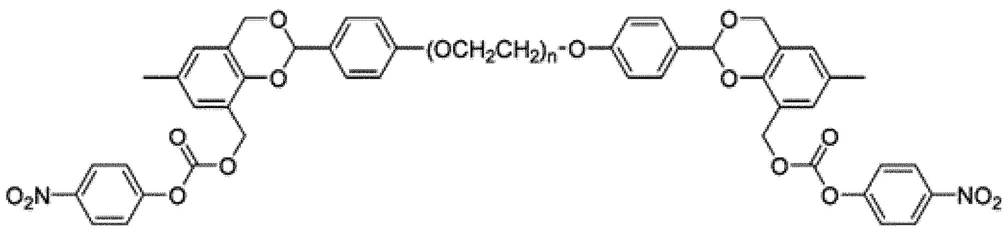

n = APPROXIMATELY 455 (40)

(Comparative Example 1-1)

To a 50 mL three-neck flask equipped with a thermometer, a nitrogen inlet tube, a stirrer, and a cooling tube, 3-hydroxybenzaldehyde (2.00 g, 16.4 mmol), trimethyl orthoformate (3.48 g, 32.8 mmol), and methanol (17 g) were added,

and then p-toluenesulfonic acid monohydrate (0.312 mg, 1.64 mmol) was added and reacted at 25°C for 2 hours. Sodium hydroxide was added and stirred for a while, and then the solvent was distilled off under reduced pressure. The residue was dissolved in dichloromethane and washed with a 5 wt% aqueous solution of sodium hydrogen carbonate and then with 25 wt% saline, and an organic layer was dried with anhydrous sodium sulfate. After filtration, the solvent was removed under reduced pressure to obtain the compound of Formula (41).

$^1$H-NMR (CDCl3, internal standard TMS); δ (ppm):
3.33 (6H, s, $-OCH_3$), 5.35 (1H, s, -CH<), 6.81 (1H, d, arom.H), 6.95 (1H, d, arom.H), 7.23 to 7.26 (1H, m, arom.H)

[Chem. 58]

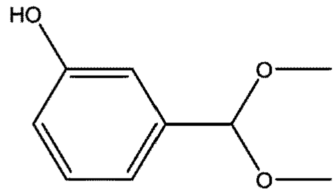

(41)

(Comparative Example 1-2)

To a 50 mL three-neck flask equipped with a thermometer, a nitrogen inlet tube, a stirrer and a cooling tube, 2,4-di(hydroxymethyl)phenol (50.0 mg, 0.324 mmol) synthesized according to a literature (Freeman, J.H.; JAm. Chem. Soc. 1952, 74, 6257-6260), the compound of Formula (41) (217 mg, 1.29 mmol), 2,6-di-tert-butyl-p-cresol (7.14 mg, 0.0324 mmol), anhydrous sodium sulfate (1 g) and cyclopentyl methyl ether (10 g) were added, and then p-toluenesulfonic acid monohydrate (4.10 mg, 0.0212 mmol) was added and reacted at 40°C for 2 hours. N-methylmorpholine was added and stirred for a while, followed by filtration. After washing with 10 wt% saline, an organic layer was dried with anhydrous sodium sulfate. After filtration, the solvent was removed under reduced pressure to obtain the compound of Formula (42).

$^1$H-NMR (d6-DMSO, internal standard TMS); δ (ppm):
4.42 (2H, d, $-CH_2OH$), 4.93 (1H, d, $-CH_2O-$), 5.10 (1H, t, $-CH_2OH$), 5.15 (1H, d, - $CH_2O-$), 6.01 (1H, s, -CH<), 6.80 to 7.21 (7H, m, arom.H), 9.53 (1H, bs, >C-OH)

[Chem. 59]

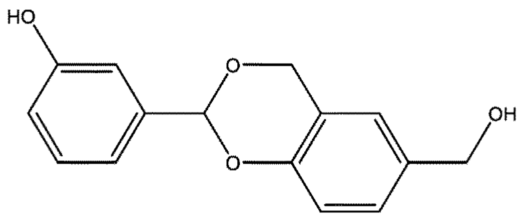

(42)

(Comparative Example 1-3)

To a 50 mL three-neck flask equipped with a thermometer, a nitrogen inlet tube, a stirrer, and a cooling tube, the compound of Formula (42) (37.0 mg, 0.141 mmol), ME-200MS (α-methyl-ω-[(methylsulfonyl)oxy]poly(oxyethylene), 705 mg, 0.0353 mmol) manufactured by NOF Corporation, potassium carbonate (97.0 mg, 0.705 mmol), and acetonitrile (3.5 g) were added and reacted at 80°C for 4 hours. After filtration, the solvent was removed under reduced pressure and the residue was dissolved in dichloromethane. After washing with 10 wt% saline, an organic layer was dried with anhydrous sodium sulfate. After filtration, the solvent was removed under reduced pressure and the residue was dissolved in toluene (50 g). Hexane (50 g) was added to cause crystallization, followed by filtration and drying under reduced pressure to obtain the compound of Formula (43).

$^1$H-NMR ($CDCl_3$, internal standard TMS); δ (ppm):
3.38 (3H, s, $-OCH_3$), 3.52 to 4.18 (m, $-(OCH_2CH_2)_n-$), 4.62 (2H, s, $-CH_2OH$), 4.98 (1H, d, $-CH_2O-$), 5.18 (1H, d, $-CH_2O-$), 5.95 (1H, s, -CH<), 6.87 to 7.34 (7H, m, arom.H)

[Chem. 60]

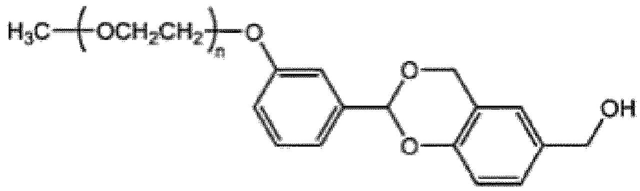

n = APPROXIMATELY 455 (43)

(Comparative Examples 1 to 4)

To a 50 mL three-neck flask equipped with a thermometer, a nitrogen inlet tube, a stirrer, and a cooling tube, the compound of Formula (43) (300 mg, 0.0150 mmol), di(N-succinimidyl) carbonate (46.0 mg, 0.180 mmol), triethylamine (21.0 mg, 0.208 mmol), and dichloromethane (5 g) were added and reacted at 25°C for 12 hours. After filtration, the mixture was washed with 5 wt% saline, and the solvent in an organic layer was distilled off under reduced pressure. The residue was dissolved in ethyl acetate (6 g), dried with anhydrous sodium sulfate, and then filtered. After adding ethyl acetate (44 g), hexane (50 g) was added to cause crystallization, followed by filtration and drying under reduced pressure to obtain the compound of Formula (44).

$^1$H-NMR ($CDCl_3$, internal standard TMS); δ (ppm):

2.85 (4H, s, $-COCH_2CH_2CO-$), 3.38 (3H, s, $-OCH_3$), 3.52 to 4.18 (m, $-(OCH_2CH_2)_n-$), 5.00 (1H, d, $-CH_2OCH<$), 5.18 (1H, d, $-CH_2O-$), 5.25 (2H, s, $-CH_2OCO-$), 5.97 (1H, s, -CH<), 6.96 to 7.35 (7H, m, arom.H)

[Chem. 61]

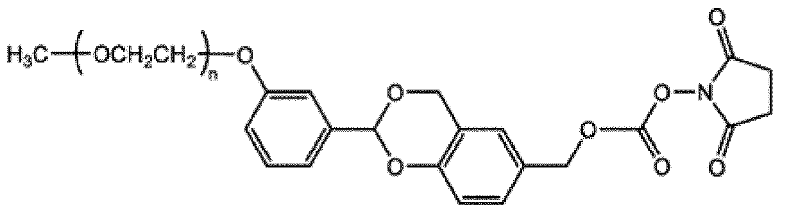

n = APPROXIMATELY 455 (44)

(Example 8-1)

To a 50 mL three-neck flask equipped with a thermometer, a nitrogen inlet tube, a stirrer and a cooling tube, the compound of Formula (20) (1.0 g, 0.05 mmol), 2,4-di(hydroxymethyl)phenol (336 mg, 2.0 mmol) synthesized according to the literature (Freeman, J.H.; JAm. Chem. Soc. 1952, 74, 6257-6260), tetrahydrofuran (5.0 g) and 2,6-di-tert-butyl-p-cresol (1.1 mg) were added and dissolved, and then a molecular sieve 5A (1.0 g) and p-toluenesulfonic acid monohydrate (11.4 mg, 0.06 mmol) were added and reacted at 40°C for 4 hours. N-methylmorpholine (12.1 mg, 0.12 mmol) was added and stirred for a while, and then filtered, and the filtrate was diluted with chloroform (10 g). After repeatedly washing with 20% saline (10 g) of pH 12, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added to repeat the crystallization operation to remove low molecular weight impurities, and then the compound of Formula (45) was obtained after drying under reduced pressure.

$^1$H-NMR ($CDCl_3$, internal standard TMS); δ (ppm):

3.38 (3H, s, $-(OCH_2CH_2)_nOCH_3$), 3.52 to 4.17 (m, $-(OCH_2CH_2)_n-$), 4.62 (2H, s, $-CH_2OH$), 5.00 (1H, d, $-CH_2OCH<$), 5.18 (1H, d, $-CH_2O-$), 5.25 (2H, s, $-CH_2OCO-$), 5.97 (1H, s, -CH<), 6.96 to 7.35 (7H, m, arom.H)

[Chem. 62]

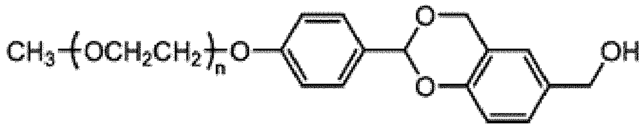

n = APPROXIMATELY 455 (45)

(Example 8-2)

To a 50 mL three-neck flask equipped with a thermometer, a nitrogen inlet tube, and a stirrer, the compound of Formula (45) (250 mg, 0.0125 mmol) and dichloromethane (3.6 g) were added and dissolved, and then pyridine (23.7 mg, 0.30 mmol) and p-nitrophenyl chloroformate (40.3 mg, 0.20 mmol) were added and reacted at 25°C for 3 hours. After repeatedly washing with 20% saline (2.5 g) of pH 12 to remove some impurities, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added for crystallization, followed by filtration and drying under reduced pressure, and then the compound of Formula (46) was obtained.

$^1$H-NMR ($CDCl_3$, internal standard TMS); δ (ppm):
3.38 (3H, s, $-(OCH_2CH_2)_nOCH_3$), 3.52 to 4.17 (m, $-(OCH_2CH_2)_n-$), 4.62 (2H, s, - $CH_2OH$), 5.00 (1H, d, $-CH_2OCH<$), 5.18 (1H, d, $-CH_2O-$), 5.25 (2H, s, $-CH_2OCO-$), 5.97 (1H, s, -CH<), 6.96 to 7.35 (11H, m, arom.H)

[Chem. 63]

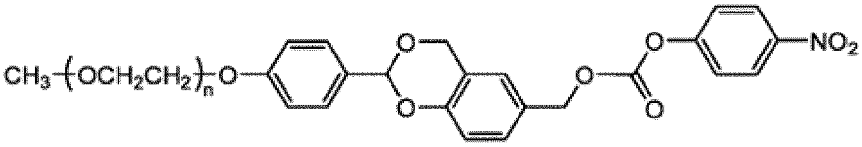

n = APPROXIMATELY 455 (46)

(Example 9-1)

To a 300 mL four-neck flask equipped with a thermometer, a nitrogen inlet tube, a stirrer, a Dean-Stark tube, and a cooling tube, SUNBRIGHT MEH-40T (40 g, 2.0 mmol) manufactured by NOF Corporation, toluene (120 g), and 2,6-di-tert-butyl-p-cresol (8 mg) were added, and water was removed azeotropically with toluene. After being cooled to 45°C, chloroform (200 g) was added, and then cooled to 25°C, and 2-hydroxybenzaldehyde (977 mg, 8.0 mmol) and triphenylphosphine (2.10 g, 8.0 mmol) were added. After stirring for a while, diisopropyl azodicarboxylate (1.51 g, 7.0 mmol) was added in three times so that an internal temperature did not exceed 35°C. After reacting at 25°C for 1 hour, methanol (224 mg, 7.0 mmol) was added, and the mixture was stirred for 30 minutes, and then the solvent was distilled off under reduced pressure. The residue was dissolved in ethyl acetate (320 g), and hexane (240 g) was added to cause crystallization, followed by suction filtration to obtain crystals. Furthermore, the obtained crystals were dissolved in ethyl acetate (320 g) containing 2,6-di-tert-butyl-p-cresol (64 mg), and then hexane (160 g) was added to repeat the crystallization operation to remove low molecular weight impurities. The crystals were washed with hexane (160 g), filtered, and dried under reduced pressure to obtain the compound of Formula (47).

$^1$H-NMR ($CDCl_3$, internal standard TMS); δ (ppm):
3.38 (3H, s, $-(OCH_2CH_2)_nOCH_3$), 3.52 to 4.23 (m, $-(OCH_2CH_2)_n-$), 9.89 (1H, s, -COH)

[Chem. 64]

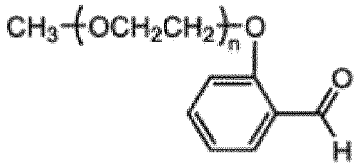

n = APPROXIMATELY 909 (47)

(Example 9-2)

To a 50 mL three-neck flask equipped with a thermometer, a nitrogen inlet tube, a stirrer and a cooling tube, the compound of Formula (47) (1.0 g, 0.05 mmol), 2,4-di(hydroxymethyl)phenol (336 mg, 2.0 mmol) synthesized according to the literature (Freeman, J.H.; JAm. Chem. Soc. 1952, 74, 6257-6260), tetrahydrofuran (5.0 g) and 2,6-di-tert-butyl-p-cresol (1.1 mg) were added and dissolved, and then a molecular sieve 5A (1.0 g) and p-toluenesulfonic acid monohydrate (11.4 mg, 0.06 mmol) were added and reacted at 40°C for 4 hours. N-methylmorpholine (12.1 mg, 0.12 mmol) was added and stirred for a while, and then filtered, and the filtrate was diluted with chloroform (10 g). After repeatedly washing with 20% saline (10 g) of pH 12, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added to repeat the crystallization operation to remove low molecular weight impurities, and then the compound of Formula (48) was obtained after drying under reduced pressure.

$^1$H-NMR ($CDCl_3$, internal standard TMS); δ (ppm): 3.38 (3H, s, $-(OCH_2CH_2)_nOCH_3$), 3.52 to 4.17 (m, $-(OCH_2CH_2)_n-$), 4.96 (1H, d, - $C\underline{H_2}O$-), 5.16 (1H, d, $-C\underline{H_2}O-$), 5.25 (2H, dd, $-C\underline{H_2}OH$), 5.97 (1H, s, -C<u>H</u><), 6.96 to 7.35 (7H, m, arom.H)

[Chem. 65]

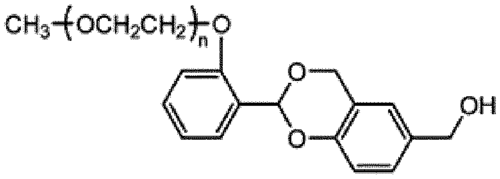

n = APPROXIMATELY 909 (48)

(Example 9-3)

To a 50 mL three-neck flask equipped with a thermometer, a nitrogen inlet tube, and a stirrer, the compound of Formula (48) (250 mg, 0.0125 mmol) and dichloromethane (3.6 g) were added and dissolved, and then pyridine (23.7 mg, 0.30 mmol) and p-nitrophenyl chloroformate (40.3 mg, 0.20 mmol) were added and reacted at 25°C for 3 hours. After repeatedly washing with 20% saline (2.5 g) of pH 12 to remove some impurities, an organic layer was dried with anhydrous sodium sulfate and filtered, and the solvent was distilled off under reduced pressure. The residue was dissolved in ethyl acetate (90 g) containing 2,6-di-tert-butyl-p-cresol (18 mg), and crystallization was performed by adding hexane (66 g), followed by suction filtration to obtain crystals. Furthermore, the crystals obtained were dissolved in ethyl acetate (90 g) containing 2,6-di-tert-butyl-p-cresol (18 mg), and then hexane (66 g) was added for crystallization, followed by filtration and drying under reduced pressure, and then the compound of Formula (49) was obtained.

$^1$H-NMR ($CDCl_3$, internal standard TMS); δ (ppm):
3.38 (3H, s, $-(OCH_2CH_2)_nOCH_3$), 3.52 to 4.17 (m, $-(OCH_2CH_2)_n-$), 4.96 (1H, d, - $C\underline{H_2}O$-), 5.16 (1H, d, $-C\underline{H_2}O-$), 5.35 (2H, dd, $-C\underline{H_2}OCO-$), 5.97 (1H, s, -C<u>H</u><), 6.96 to 7.35 (11H, m, arom.H)

[Chem. 66]

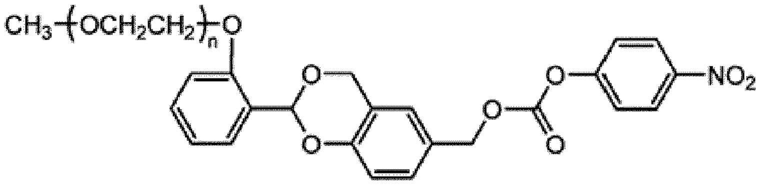

n = APPROXIMATELY 909 (49)

(Example 10)

A 20 mg/mL buffer solution of β-alanine was prepared using 0.1 M sodium phosphate buffer solution (pH 8.5). To a 50 mL three-neck flask equipped with a thermometer, a nitrogen inlet tube, and a stirrer, the compound of Formula (22) (70 mg, 0.0035 mmol) and the 20 mg/mL buffer solution of β-alanine (1.5 g) were added and dissolved, and then reacted at 25°C for 6 hours. After dissolving salt (500 mg), extraction was performed using chloroform (3.0 g), and an organic layer was dried with anhydrous sodium sulfate and then filtered. The filtrate was diluted with ethyl acetate (45 g), and then hexane (33 g) was added to cause crystallization, and after filtration, the crystals were dried under reduced pressure to obtain the compound of Formula (50).

$^1$H-NMR ($CDCl_3$, internal standard TMS); δ (ppm):
2.28 (3H, s, $-CH_3$), 2.48 (2H, t, $-CH_2COOH$), 3.38 (3H, s, $-(OCH_2CH_2)_nOCH_3$), 3.39 to 3.45 (2H, m, $-CH_2NH-$), 3.52 to 4.17 (m, $-(OCH_2CH_2)_n-$), 4.92 (1H, d, $-CH_2O-$), 5.08 (1H, d, $-CH_2O-$), 5.15 (2H, dd, $-CH_2OCONH-$), 5.96 (1H, s, -CH<), 6.79 (1H, s, arom.H), 6.96 (2H, d, arom.H), 7.04 (1H, s, arom.H), 7.50 (2H, d, arom.H)

[Chem. 67]

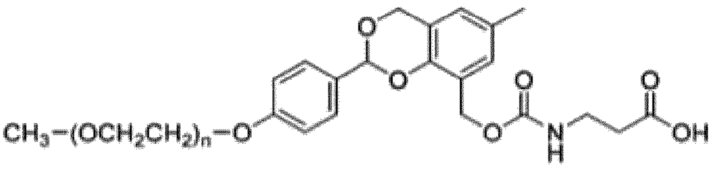

n = APPROXIMATELY 455 (50)

(Example 11)

A 20 mg/mL buffer solution of β-alanine was prepared using 0.1 M sodium phosphate buffer solution (pH 8.5). To a 50 mL three-neck flask equipped with a thermometer, a nitrogen inlet tube, and a stirrer, the compound of Formula (25) (70 mg, 0.0035 mmol) and the 20 mg/mL buffer solution of β-alanine (1.5 g) were added and dissolved, and then reacted at 25°C for 6 hours. After dissolving salt (500 mg), extraction was performed using chloroform (3.0 g), and an organic layer was dried with anhydrous sodium sulfate and then filtered. The filtrate was diluted with ethyl acetate (45 g), and then hexane (33 g) was added to cause crystallization, and after filtration, the crystals were dried under reduced pressure to obtain the compound of Formula (51).

$^1$H-NMR ($CDCl_3$, internal standard TMS); δ (ppm):
2.28 (3H, s, $-CH_3$), 2.50 (2H, t, $-CH_2COOH$), 3.38 (3H, s, $-(OCH_2CH_2)_nOCH_3$), 3.52 to 4.25 (m, $-(OCH_2CH_2)_n-$), 3.41 to 3.45 (2H, m, $-CH_2NH-$), 4.91 (1H, d, $-CH_2O-$), 5.08 (1H, d, $-CH_2O-$), 5.14 (2H, dd, $-CH_2OCONH-$), 5.94 (1H, s, -CH<), 6.79 (1H, s, arom.H), 7.03 to 7.06 (2H, m, arom.H), 7.30 to 7.34 (2H, m, arom.H)

[Chem. 68]

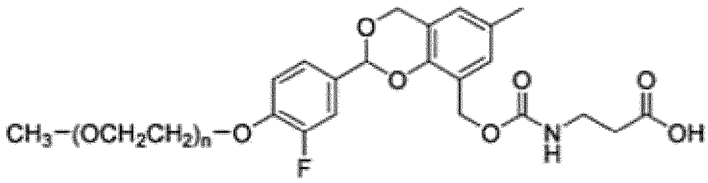

n = APPROXIMATELY 455 (51)

(Example 12)

A 20 mg/mL buffer solution of β-alanine was prepared using 0.1 M sodium phosphate buffer solution (pH 8.5). To a 50 mL three-neck flask equipped with a thermometer, a nitrogen inlet tube, and a stirrer, the compound of Formula (28) (14 mg, 0.0072 mmol) and the 20 mg/mL buffer solution of β-alanine (3.0 g) were added and dissolved, and then reacted at 25°C for 6 hours. After dissolving salt (750 mg), extraction was performed using chloroform (4.5 g), and an organic layer was dried with anhydrous sodium sulfate and then filtered. The filtrate was diluted with ethyl acetate (45 g), and then hexane (33 g) was added to cause crystallization, and after filtration, the crystals were dried under reduced pressure to obtain the compound of Formula (52).

$^1H$-NMR ($CDCl_3$, internal standard TMS); δ (ppm):
2.28 (3H, s, $-CH_3$), 2.47 (2H, t, $-CH_2COOH$), 3.38 (3H, s, $-(OCH_2CH_2)_nOCH_3$), 3.37 to 3.43 (2H, m, $-CH_2NH-$), 3.52 to 4.21 (m, $-(OCH_2CH_2)_n-$), 4.89 (1H, d, $-CH_2O-$), 5.05 (1H, d, $-CH_2O-$), 5.14 (2H, dd, $-CH_2OCONH-$), 6.22 (1H, s, -CH<), 6.52 to 7.57 (5H, arom.H)

[Chem. 69]

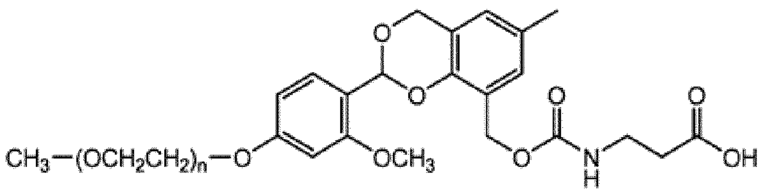

n = APPROXIMATELY 455 (52)

(Comparative Example 2)

A 20 mg/mL buffer solution of β-alanine was prepared using 0.1 M sodium phosphate buffer solution (pH 8.5). To a 50 mL three-neck flask equipped with a thermometer, a nitrogen inlet tube, and a stirrer, the compound of Formula (44) (143 mg, 0.0072 mmol) and the 20 mg/mL buffer solution of β-alanine (3.0 g) were added and dissolved, and then reacted at 25°C for 6 hours. After dissolving salt (750 mg), extraction was performed using chloroform (4.5 g), and an organic layer was dried with anhydrous sodium sulfate and then filtered. The filtrate was diluted with ethyl acetate (45 g), and then hexane (33 g) was added to cause crystallization, and after filtration, the crystals were dried under reduced pressure to obtain the compound of Formula (53).

$^1H$-NMR ($CDCl_3$, internal standard TMS); δ (ppm):
2.47 (2H, t, $-CH_2COOH$), 3.38 (3H, s, $-(OCH_2CH_2)_nOCH_3$), 3.37 to 3.43 (2H, m, - $CH_2NH-$), 3.52 to 4.21 (m, $-(OCH_2CH_2)_n-$), 4.89 (1H, d, $-CH_2O-$), 5.05 (1H, d, $-CH_2O-$), 5.14 (2H, dd, $-CH_2OCONH-$), 6.22 (1H, s, -CH<), 6.52 to 7.57 (7H, arom.H)

[Chem. 70]

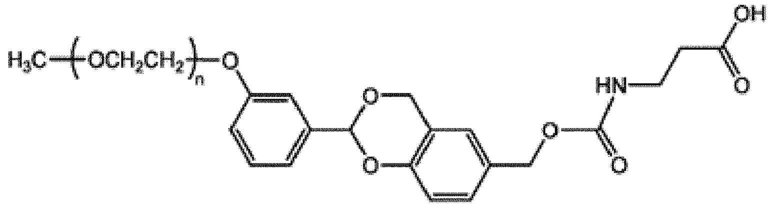

n = APPROXIMATELY 455 (53)

(Degradability Test)

Each of the compounds of Formulas (50), (51), (52) and (53) (20 mg) was dissolved in sodium phosphate deuterium oxide buffer solution (0.7 mL) having a pD of 7.4, and then placed in a thermostatic bath at 40°C, and NMR measurement was performed at a random timing. Fig. 1 shows a β-alanine adduct amount at a random timing assuming a β-alanine adduct amount at 0 hour of standing as 100%. A half-life (t1/2) was calculated from a formula (in which In represents a logarithm with the Napier's number as a base, and λ represents a product of an exponent of the Napier's number in an approximation formula of the graph and -1) based on the approximation formula.

[Math. 1]

$$t_{1/2} = In(2)/\lambda$$

The half-life (t1/2) calculated above was 3.6 days for the compound of Formula (50), 31.5 days for the compound of Formula (51), 0.55 days for the compound of Formula (52), and 231 days for the compound of Formula (53), which indicates that the compounds according to the present invention expressed by Formulas (50), (51), and (52) have a desired half-life under a physiological condition, as compared with the compound exemplified in Patent Literature 3 expressed by Formula (53), which is in the prior art.

(Example 13)

The compound of Formula (22) was used to modify RNase A (manufactured by Roche, RNase A from bovine pancreas, Mw = 13,700). A 10 mg/mL buffer solution of RNase A was prepared using 0.1 M borate buffer solution (pH = 8.5), and the 10 mg/mL buffer solution of RNase A (636.3 μL) and the 0.1 M borate buffer solution (863.7 μL) were mixed, and then the compound of Formula (22) (6.0 mg) was added and reacted at 20°C for 20 hours. The reaction solution was filtered, and then subjected to HPLC measurement using a cation exchange column under the following conditions. An eluate was monitored by UV and fractionated to obtain mono PEG RNase A with a purity of 96.1% modified at one site with the compound of Formula (22). Fig. 2 shows results of the HPLC measurement of the fractionated mono PEG RNase A.

HPLC equipment: Thermo Ultimate3000
Column: TSKgel SP-5PW (7.5 × 75 mm, 10 um; Tosoh Corporation)
Flow rate: 1 mL/min
Analysis time: 25 minutes (including equilibration)
Column temperature: 25°C
Injection volume: 20 μL
Detector: PDA (measurement wavelength: 280 nm)
Mobile phase A: 5 mM sodium phosphate buffer solution (pH 8.5)
Mobile phase B: 5 mM sodium phosphate buffer solution + 0.5 mol/L NaCl (pH 8.5)
Gradient program (A/B): 100/0 (- 15 min) → 100/0 (0 min) → 33/67 (10 min)

(Example 14-1)

To a four-neck flask equipped with a thermometer, a nitrogen inlet tube, and a stirrer, ME-200MS (α-methyl-ω-[(methylsulfonyl)oxy]poly(oxyethylene), 10 g, 0.5 mmol) manufactured by NOF Corporation, 3-methyl-4-hydroxybenzaldehyde (272 mg, 2 mmol), potassium carbonate (0.69 g, 5 mmol), and acetonitrile (50 g) were added and reacted at 80°C for 6 hours. After the reaction, the mixture was filtered, concentrated, and redissolved in dichloromethane (80 g).

Thereafter, an aqueous solution containing 1 wt% of potassium carbonate and 10 wt% of sodium chloride was added, and the mixture was stirred at 25°C for 10 minutes under a nitrogen atmosphere. After stirring, the mixture was allowed to stand for 10 minutes, and an organic layer was recovered. Magnesium sulfate was added to the recovered organic layer, and the mixture was stirred at 25°C for 10 minutes under a nitrogen atmosphere and then filtered by suction. The obtained filtrate was concentrated and then dissolved in ethyl acetate, and hexane was added thereto to precipitate crystals at 25°C under a nitrogen atmosphere. The precipitated crystals were recovered by suction filtration and dried under reduced pressure to obtain the compound of Formula (54).

$^1$H-NMR ($CDCl_3$, internal standard TMS); δ (ppm):
2.28 (3H, s, $-CH_3$), 3.38 (3H, s, $-(OCH_2CH_2)_nOCH_3$), 3.52 to 4.17 (m, $-(OCH_2CH_2)_n-$), 6.93 (1H, d, arom.H), 7.69 (2H, d, arom.H), 9.85 (1H, s, -CHO)

[Chem. 71]

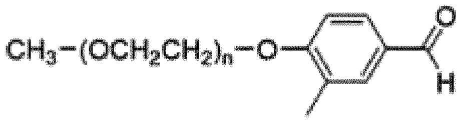

n = APPROXIMATELY 455 (54)

(Example 14-2)

To a three-neck flask equipped with a thermometer, a nitrogen inlet tube, a stirrer and a cooling tube, the compound of Formula (54) (3.0 g, 0.15 mmol), 2,4-bis(hydroxymethyl)-p-cresol (1.0 g, 6.0 mmol), tetrahydrofuran (15.0 g) and 2,6-di-tert-butyl-p-cresol (3.3 mg) were added, and after dissolution, a molecular sieve 5A (3.0 g) and p-toluenesulfonic acid monohydrate (34.2 mg, 0.18 mmol) were added and reacted at 40°C for 4 hours. N-methylmorpholine (36.4 mg, 0.36 mmol) was added and stirred for a while, and then filtered, and the filtrate was diluted with toluene (33.0 g). After repeatedly washing with 20% saline (30 g) of pH 12, an organic layer was dried with anhydrous sodium sulfate and filtered, and the solvent was distilled off under reduced pressure. The residue was dissolved in toluene containing 2,6-di-tert-butyl-p-cresol, and hexane was added to cause crystallization, followed by suction filtration and drying under reduced pressure to obtain the compound of Formula (55).

$^1$H-NMR ($CDCl_3$, internal standard TMS); δ (ppm):
2.23 (3H, s, $-CH_3$), 2.28 (3H, s, $-CH_3$), 3.38 (3H, s, $-(OCH_2CH_2)_nOCH_3$), 3.52 to 4.17 (m, $-(OCH_2CH_2)_n-$), 4.62 (2H, s, $-CH_2OH$), 4.95 (1H, d, $-CH_2OCH<$), 5.16 (1H, d, $-CH_2O-$), 5.92 (1H, s, -CH <), 6.8 to 7.3 (5H, arom.H)

[Chem. 72]

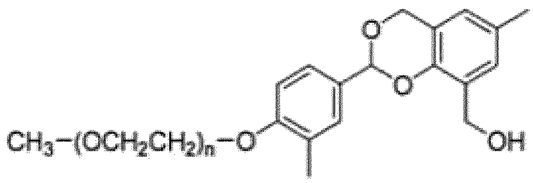

n = APPROXIMATELY 455 (55)

(Example 14-3)

To a three-neck flask equipped with a thermometer, a nitrogen inlet tube, and a stirrer, the compound of Formula (55) (500 mg, 0.025 mmol), 2,6-di-tert-butyl-p-cresol (0.5 mg), and toluene (6.0 g) were added and dissolved, and then triethylamine (38 mg, 0.375 mmol) and p-nitrophenyl chloroformate (50.4 mg, 0.25 mmol) were added and reacted at 60°C for 3 hours. After the reaction, the mixture was diluted with a mixed solvent of ethyl acetate (100 g) containing 2,6-di-tert-butyl-p-cresol (20 mg) and acetonitrile (1.25 g), and crystals were precipitated by adding hexane (50 g), and the crystals were then obtained by suction filtration. The obtained crystals were dried under reduced pressure to obtain the compound of Formula (56).

$^1$H-NMR ($CDCl_3$, internal standard TMS); δ (ppm):

2.23 (3H, s, $-CH_3$), 2.31 (3H, s, $-CH_3$), 3.38 (3H, s, $-(OCH_2CH_2)_nOCH_3$), 3.52 to 4.24 (m, $-(OCH_2CH_2)_n-$), 4.96 (1H, d,

$-CH_2O-$), 5.15 (1H, d, $-CH_2O-$), 5.34 (2H, dd, $-CH_2OCO-$), 5.96 (1H, s, -CH <), 6.8 to 7.4 (5H, arom.H), 8.23 (2H, d, arom.H)
Number average molecular weight (Mn): 20,223

[Chem. 73]

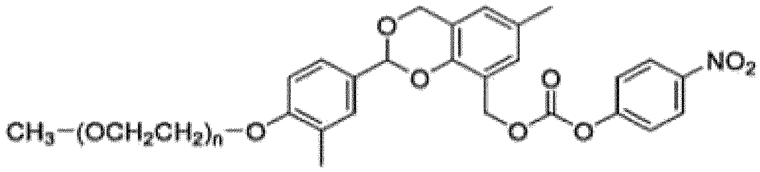

n = APPROXIMATELY 455 (56)

(Example 15-1)

The compound expressed by Formula (57) was obtained in the same manner as in Example 14-1, except that 4-hydroxy-3,5-dimethylbenzaldehyde (300 mg, 2.0 mmol) was used instead of 3-methyl-4-hydroxybenzaldehyde.
$^1$H-NMR ($CDCl_3$, internal standard TMS); δ (ppm):
2.36 (6H, s), 3.38 (3H, s, $-(OCH_2CH_2)_nOCH_3$), 3.52 to 4.17 (m, $-(OCH_2CH_2)_n-$), 7.54 (2H, s, arom.H), 9.88 (1H, s, -CHO)

[Chem. 74]

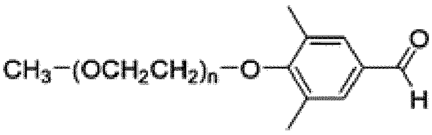

n = APPROXIMATELY 455 (57)

(Example 15-2)

The compound expressed by Formula (58) was obtained in the same manner as in Example 14-2, except that the compound expressed by Formula (57) (3.0 g, 0.15 mmol) was used instead of the compound expressed by Formula (54).
$^1$H-NMR ($CDCl_3$, internal standard TMS); δ (ppm):
2.29 (3H, s, $-CH_3$), 2.36 (6H, s), 3.38 (3H, s, $-(OCH_2CH_2)_nOCH_3$), 3.52 to 4.17 (m, - $(OCH_2CH_2)_n-$), 4.68 (2H, s, $-CH_2OH$), 4.95 (1H, d, $-CH_2OCH<$), 5.16 (1H, d, $-CH_2O-$), 5.89 (1H, s, -CH<), 6.8 to 7.3 (4H, arom.H)

[Chem. 75]

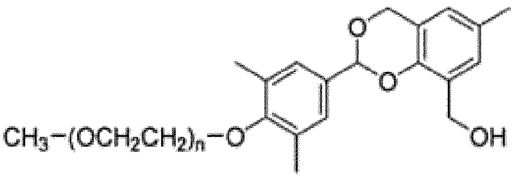

n = APPROXIMATELY 455 (58)

(Example 15-3)

The compound expressed by Formula (59) was obtained in the same manner as in Example 14-3, except that the compound expressed by Formula (58) (500 mg, 0.025 mmol) was used instead of the compound expressed by Formula (55).
$^1$H-NMR ($CDCl_3$, internal standard TMS); δ (ppm):

2.29 (3H, s, $-CH_3$), 2.36 (6H, s), 3.38 (3H, s, $-(OCH_2CH_2)_nOCH_3$), 3.52 to 4.17 (m, - $(OCH_2CH_2)_n-$), 4.68 (2H, s,

$-CH_2O-$), 4.95 (1H, d, $-CH_2OCH<$), 5.16 (1H, d, $-CH_2O-$), 5.89 (1H, s, -CH<), 6.8 to 7.3 (4H, arom.H), 8.23 (2H, d, arom.H)
Number average molecular weight (Mn): 20,072

[Chem. 76]

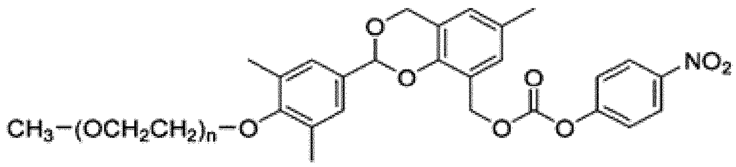

n = APPROXIMATELY 455 (59)

(Example 16-1)

The compound expressed by Formula (60) was obtained in the same manner as in Example 14-1, except that 2-hydroxybenzaldehyde (244 mg, 2.0 mmol) was used instead of 3-methyl-4-hydroxybenzaldehyde.
$^1H$-NMR ($CDCl_3$, internal standard TMS); δ (ppm):
3.38 (3H, s, $-(OCH_2CH_2)_nOCH_3$), 3.52 to 4.23 (m, $-(OCH_2CH_2)_n-$), 6.9 to 7.8 (4H, arom.H), 10.52 (1H, s, -COH)

[Chem. 77]

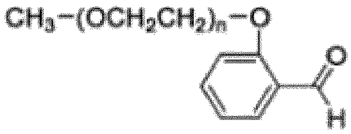

n = APPROXIMATELY 455 (60)

(Example 16-2)

The compound expressed by Formula (61) was obtained in the same manner as in Example 14-2, except that the compound expressed by Formula (60) (3.0 g, 0.15 mmol) was used instead of the compound expressed by Formula (54).
$^1H$-NMR ($CDCl_3$, internal standard TMS); δ (ppm):
2.29 (3H, s, $-CH_3$), 3.38 (3H, s, $-(OCH_2CH_2)_nOCH_3$), 3.52 to 4.17 (m, $-(OCH_2CH_2)_n-$), 4.49 (1H, d, $-CH_2OH$), 4.78 (1H, d, $-CH_2OH$), 4.99 (1H, d, $-CH_2OCH<$), 5.19 (1H, d, $-CH_2O-$), 6.32 (1H, s, -CH<), 6.8 to 7.7 (6H, arom.H)

[Chem. 78]

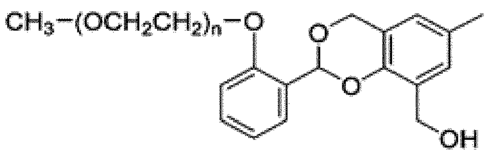

n = APPROXIMATELY 455 (61)

(Example 16-3)

The compound expressed by Formula (62) was obtained in the same manner as in Example 14-3, except that the compound expressed by Formula (61) (500 mg, 0.025 mg) was used instead of the compound expressed by Formula (55).
$^1H$-NMR ($CDCl_3$, internal standard TMS); δ (ppm):

2.29 (3H, s, $-CH_3$), 3.38 (3H, s, $-(OCH_2CH_2)_nOCH_3$), 3.52 to 4.17 (m, $-(OCH_2CH_2)_n-$), 4.22 (2H, m, $-CH_2O-$), 4.95 (1H, d, $-CH_2OCH<$), 5.16 (1H, d, $-CH_2O-$), 6.37 (1H, s, -CH<), 6.8 to 7.3 (8H, arom.H), 8.23 (2H, d, arom.H)
Number average molecular weight (Mn): 19,942

[Chem. 79]

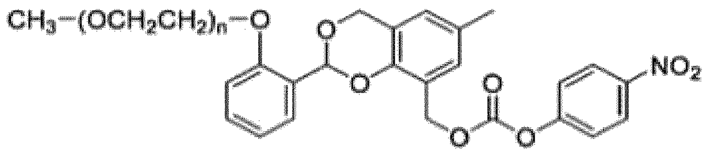

n = APPROXIMATELY 455 (62)

(Example 17-1)

The compound expressed by Formula (63) was obtained in the same manner as in Example 14-1, except that 2-hydroxy-6-methoxybenzaldehyde (304 mg, 2.0 mmol) was used instead of 3-methyl-4-hydroxybenzaldehyde.

$^1$H-NMR ($CDCl_3$, internal standard TMS); δ (ppm):
3.38 (3H, s, $-(OCH_2CH_2)_nOCH_3$), 3.52 to 4.23 (m, $-(OCH_2CH_2)_n-$), 6.6 to 7.8 (3H, arom.H), 10.53 (1H, s, -COH)

[Chem. 80]

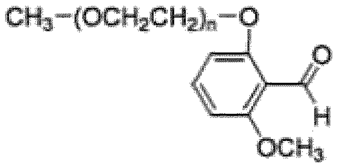

n = APPROXIMATELY 455 (63)

(Example 17-2)

The compound expressed by Formula (64) was obtained in the same manner as in Example 14-2, except that the compound expressed by Formula (63) (3.0 g, 0.15 mmol) was used instead of the compound expressed by Formula (54).

$^1$H-NMR ($CDCl_3$, internal standard TMS); δ (ppm):
2.29 (3H, s, $-CH_3$), 3.38 (3H, s, $-(OCH_2CH_2)_nOCH_3$), 3.52 to 4.17(m, $-(OCH_2CH_2)_n-$), 4.49 (1H, d, $-CH_2OH$), 4.78 (1H, d, $-CH_2OH$), 4.90 (1H, d, $-CH_2OCH$<), 5.15 (1H, d, $-CH_2O-$), 6.8 to 7.7 (5H, arom.H)

[Chem. 81]

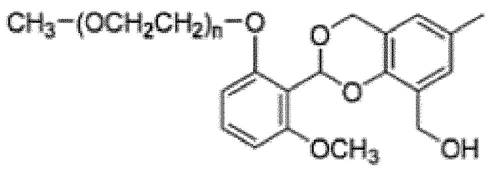

n = APPROXIMATELY 455 (64)

(Example 17-3)

The compound expressed by Formula (65) was obtained in the same manner as in Example 14-3, except that the compound expressed by Formula (64) (500 mg, 0.025 mmol) was used instead of the compound expressed by Formula (55).

$^1$H-NMR ($CDCl_3$, internal standard TMS); δ (ppm):

2.30 (3H, s, $-CH_3$), 3.38 (3H, s, $-(OCH_2CH_2)_nOCH_3$), 3.52 to 4.17 (m, $-(OCH_2CH_2)_n-$), 4.22 (2H, m, $-CH_2O-$), 4.95 (1H, d, $-CH_2OCH$<), 5.16 (1H, d, $-CH_2O-$), 6.6 to 7.1 (5H, arom.H), 8.23 (2H, d, arom.H)
Number average molecular weight (Mn): 20,915

[Chem. 82]

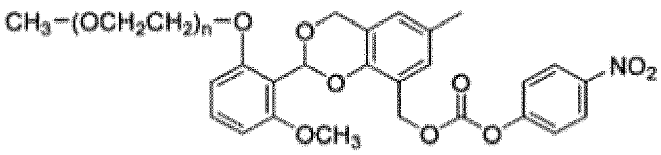

n = APPROXIMATELY 455 (65)

(Example 18-1)

The compound expressed by Formula (66) was obtained in the same manner as in Example 17-1, except that ME-050MS (2.5 g, 0.5 mmol) manufactured by NOF Corporation was used instead of ME-200MS manufactured by NOF Corporation.

$^1$H-NMR ($CDCl_3$, internal standard TMS); δ (ppm):
3.38 (3H, s, $-(OCH_2CH_2)_nOC\underline{H_3}$), 3.52 to 4.23 (m, $-(OC\underline{H_2}C\underline{H_2})_n-$), 6.6 to 7.8 (3H, arom.H), 10.53 (1H, s, -CO<u>H</u>)

[Chem. 83]

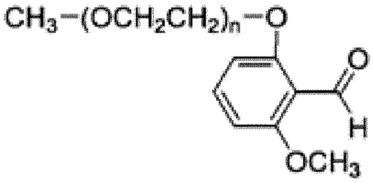

n = APPROXIMATELY 114 (66)

(Example 18-2)

The compound expressed by Formula (67) was obtained in the same manner as in Example 17-2, except that the compound expressed by Formula (66) (0.75 g, 0.15 mmol) was used instead of the compound expressed by Formula (63).

$^1$H-NMR ($CDCl_3$, internal standard TMS); δ (ppm):
2.29 (3H, s, $-CH_3$), 3.38 (3H, s, $-(OCH_2CH_2)_nOC\underline{H_3}$), 3.52 to 4.17(m, $-(OCH_2CH_2)_n-$), 4.49 (1H, d, $-C\underline{H_2}OH$), 4.78 (1H, d, $-C\underline{H_2}OH$), 4.90 (1H, d, $-C\underline{H_2}OCH<$), 5.15 (1H, d, $-C\underline{H_2}O-$), 6.8 to 7.7 (5H, arom.H)

[Chem. 84]

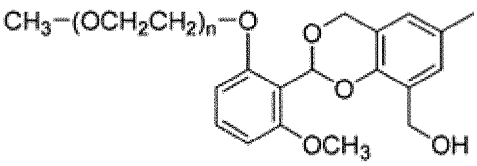

n = APPROXIMATELY 114 (67)

(Example 18-3)

The compound expressed by Formula (68) was obtained in the same manner as in Example 17-3, except that the compound expressed by Formula (67) (125 mg, 0.025 mmol) was used instead of the compound expressed by Formula (64).

$^1$H-NMR ($CDCl_3$, internal standard TMS); δ (ppm):

2.30 (3H, s, $-CH_3$), 3.38 (3H, s, $-(OCH_2CH_2)_nOCH_3$), 3.52 to 4.17 (m, $-(OCH_2CH_2)_n-$), 4.22 (2H, m, $-C\underline{H_2}O-$), 4.95 (1H, d, $-C\underline{H_2}OCH<$), 5.16 (1H, d, $-C\underline{H_2}O-$), 6.6 to 7.1 (5H, arom.H), 8.23 (2H, d, arom.H)
Number average molecular weight (Mn): 5,400

[Chem. 85]

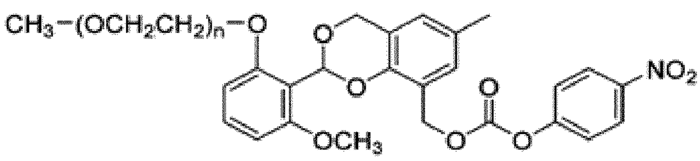

n = APPROXIMATELY 114 (68)

(Example 19-1)

The compound expressed by Formula (69) was obtained in the same manner as in Example 17-1, except that ME-020MS (1 g, 0.5 mmol) manufactured by NOF Corporation was used instead of ME-200MS manufactured by NOF Corporation.

$^1$H-NMR ($CDCl_3$, internal standard TMS); δ (ppm):
3.38 (3H, s, $-(OCH_2CH_2)_nOCH_3$), 3.52 to 4.23 (m, $-(OCH_2CH_2)_n-$), 6.6 to 7.8 (3H, arom.H), 10.53 (1H, s, -COH)

[Chem. 86]

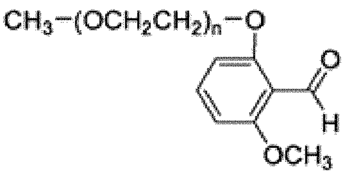

n = APPROXIMATELY 46 (69)

(Example 19-2)

The compound expressed by Formula (70) was obtained in the same manner as in Example 17-2, except that the compound expressed by Formula (69) (300 mg, 0.15 mmol) was used instead of the compound expressed by Formula (63).

$^1$H-NMR ($CDCl_3$, internal standard TMS); δ (ppm):
2.29 (3H, s, $-CH_3$), 3.38 (3H, s, $-(OCH_2CH_2)_nOCH_3$), 3.52 to 4.17(m, $-(OCH_2CH_2)_n-$), 4.49 (1H, d, $-CH_2OH$), 4.78 (1H, d, $-CH_2OH$), 4.90 (1H, d, $-CH_2OCH<$), 5.15 (1H, d, $-CH_2O-$), 6.8 to 7.7 (5H, arom.H)

[Chem. 87]

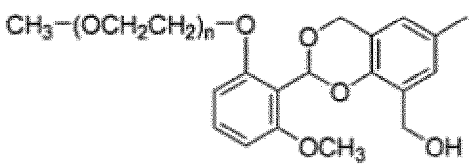

n = APPROXIMATELY 46 (70)

(Example 19-3)

The compound expressed by Formula (71) was obtained in the same manner as in Example 17-3, except that the compound expressed by Formula (70) (50 mg, 0.025 mmol) was used instead of the compound expressed by Formula (64).

$^1$H-NMR ($CDCl_3$, internal standard TMS); δ (ppm):

2.30 (3H, s, $-CH_3$), 3.38 (3H, s, $-(OCH_2CH_2)_nOCH_3$), 3.52 to 4.17 (m, $-(OCH_2CH_2)_n-$), 4.22 (2H, m, $-CH_2O-$), 4.95 (1H, d, $-CH_2OCH<$), 5.16 (1H, d, $-CH_2O-$), 6.6 to 7.1 (5H, arom.H), 8.23 (2H, d, arom.H)

Number average molecular weight (Mn): 2,615

[Chem. 88]

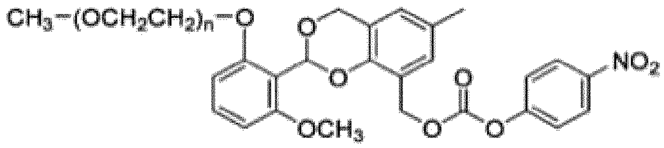

n = APPROXIMATELY 46 (71)

(Example 20-1)

The compound expressed by Formula (72) was obtained in the same manner as in Example 14-1, except that 4-hydroxy-2,6-dimethoxybenzaldehyde (364 mg, 2.0 mmol) was used instead of 3-methyl-4-hydroxybenzaldehyde.

$^1$H-NMR ($CDCl_3$, internal standard TMS); δ (ppm):
3.38 (3H, s, $-(OCH_2CH_2)_nOCH_3$), 3.52 to 4.23 (m, $-(OCH_2CH_2)_n-$), 6.12 (2H, arom.H), 10.35 (1H, s, -COH)

[Chem. 89]

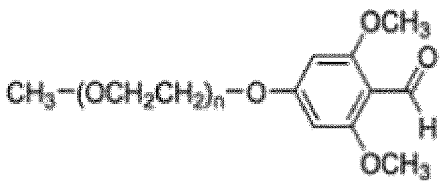

n = APPROXIMATELY 455 (72)

(Example 20-2)

The compound expressed by Formula (73) was obtained in the same manner as in Example 14-2, except that the compound expressed by Formula (72) (3.0 g, 0.15 mmol) was used instead of the compound expressed by Formula (54).

$^1$H-NMR ($CDCl_3$, internal standard TMS); δ (ppm):
2.28 (3H, s, $-CH_3$), 2.84 (1H, dd, -OH), 3.38 (3H, s, $-(OCH_2CH_2)_nOCH_3$), 3.52 to 4.17 (m, $-(OCH_2CH_2)_n-$), 4.47 (1H, dd, $-CH_2OH$), 4.75 (1H, dd, $-CH_2OH$), 4.98 (1H, d, $-CH_2O-$), 5.17 (1H, d, $-CH_2O-$), 6.20 (1H, s, -CH<), 6.57 to 6.55 (2H, m, arom.H), 6.78 (1H, s, arom.H), 6.97 (1H, s, arom.H), 7.58 (2H, d, arom.H)

[Chem. 90]

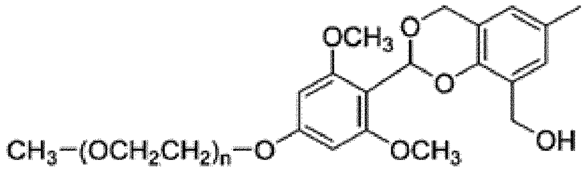

n = APPROXIMATELY 455 (73)

(Example 20-3)

The compound expressed by Formula (74) was obtained in the same manner as in Example 14-3, except that the compound expressed by Formula (73) (500 mg, 0.025 mmol) was used instead of the compound expressed by Formula (55).

$^1$H-NMR ($CDCl_3$, internal standard TMS); δ (ppm):

2.31 (3H, s, $-CH_3$), 3.38 (3H, s, $-(OCH_2CH_2)_nOCH_3$), 3.52 to 4.17 (m, $-(OCH_2CH_2)_n-$), 4.93 (1H, d, $-CH_2O-$), 5.18 (1H,

d, $-CH_2O-$), 5.33 (2H, dd, $-CH_2OCO-$), 6.26 (1H, s, -CH<), 6.52 to 6.54 (2H, m, arom.H), 6.87 (1H, s, arom.H), 7.10 (1H, s, arom.H), 7.26 to 7.27 (2H, m, arom.H), 7.59 (1H, d, arom.H), 8.22 (2H, d, arom.H)
Number average molecular weight (Mn): 20,560

[Chem. 91]

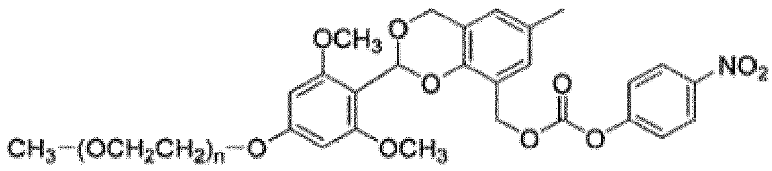

n = APPROXIMATELY 455 (74)

(Example 21)

A 20 mg/mL buffer solution of β-alanine was prepared using 0.1 M sodium phosphate buffer solution (pH 8.5). The compound of Formula (56) (74 mg, 0.0037 mmol) was dissolved in the 20 mg/mL buffer solution of β-alanine (1.5 mL), and then reacted at 25°C for 6 hours. After the reaction, the mixture was diluted with 0.1 M sodium phosphate buffer solution (pH 8.5) containing 20 wt% sodium chloride, and extracted with chloroform (3 g). An organic layer was dried with anhydrous sodium sulfate and then filtered. The filtrate was diluted with ethyl acetate (45 g), and then hexane (33 g) was added to cause crystallization, and after filtration, the crystals were dried under reduced pressure to obtain the compound of Formula (75).

$^1H$-NMR ($CDCl_3$, internal standard TMS); δ (ppm):
2.23 (3H, s, $-CH_3$), 2.28 (3H, s, $-CH_3$), 2.47 (2H, t, $-CH_2COOH$), 3.38 (3H, s, - $(OCH_2CH_2)_nOCH_3$), 3.37 to 3.43 (2H, m, $-CH_2NH-$), 3.52 to 4.21 (m, $-(OCH_2CH_2)_n-$), 4.89 (1H, d, $-CH_2O-$), 5.05 (1H, d, $-CH_2O-$), 5.14 (2H, dd, $-CH_2OCONH-$), 5.93 (1H, s, -CH<), 6.8 to 7.7 (5H, arom.H)

[Chem. 92]

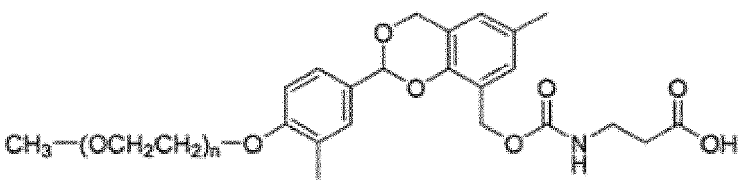

n = APPROXIMATELY 455 (75)

(Example 22)

The compound of Formula (76) was obtained in the same manner as in Example 21, except that the compound of Formula (59) (74 mg, 0.0037 mmol) was used instead of the compound of Formula (56).

$^1H$-NMR ($CDCl_3$, internal standard TMS); δ (ppm):
2.28 (3H, s, $-CH_3$), 2.31 (6H, s), 2.52 (2H, t, $-CH_2COOH$), 3.38 (3H, s, - $(OCH_2CH_2)_nOCH_3$), 3.52 to 4.21 (m, $-(OCH_2CH_2)_n-$), 3.93 to 3.97 (2H, m, $-CH_2NH-$), 4.92 (1H, d, $-CH_2O-$), 5.09 (1H, d, $-CH_2O-$), 5.13 (2H, dd, $-CH_2OCONH-$), 5.89 (1H, s, -CH<), 6.8 to 7.2 (4H, arom.H)

[Chem. 93]

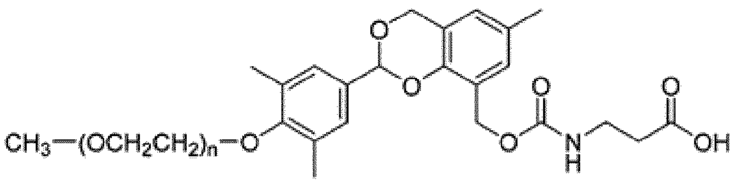

n = APPROXIMATELY 455 (76)

(Example 23)

The compound of Formula (77) was obtained in the same manner as in Example 21, except that the compound of Formula (62) (74 mg, 0.0037 mmol) was used instead of the compound of Formula (56).

$^1$H-NMR ($CDCl_3$, internal standard TMS); δ (ppm):
2.28 (3H, s, $-CH_3$), 2.43 (2H, t, $-CH_2COOH$), 3.38 (3H, s, $-(OCH_2CH_2)_nOCH_3$), 3.37 to 3.43 (2H, m, $-CH_2NH-$), 3.52 to 4.21 (m, $-(OCH_2CH_2)_n-$), 4.92 (1H, d, $-CH_2O-$), 4.97 (1H, d, $-CH_2O-$), 5.19 (2H, dd, $-CH_2OCONH-$), 6.32 (1H, s, -CH<), 6.8 to 7.7 (6H, arom.H)

[Chem. 94]

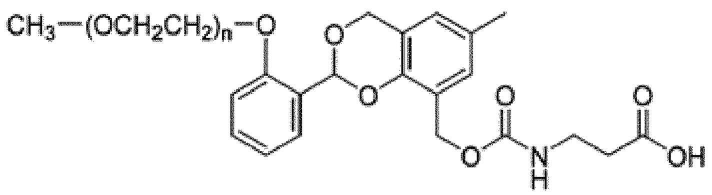

n = APPROXIMATELY 455 (77)

(Example 24)

The compound of Formula (78) was obtained in the same manner as in Example 21, except that the compound of Formula (65) (74 mg, 0.0037 mmol) was used instead of the compound of Formula (56).

$^1$H-NMR ($CDCl_3$, internal standard TMS); δ (ppm):
2.28 (3H, s, $-CH_3$), 2.47 (2H, t, $-CH_2COOH$), 3.38 (3H, s, $-(OCH_2CH_2)_nOCH_3$), 3.52 to 4.21 (m, $-(OCH_2CH_2)_n-$), 4.89 (1H, d, $-CH_2O-$), 5.03 (2H, dd, $-CH_2OCONH-$), 5.14 (1H, d, - $CH_2O-$), 6.53 (1H, s, -CH<), 6.8 to 7.7 (5H, arom.H)

[Chem. 95]

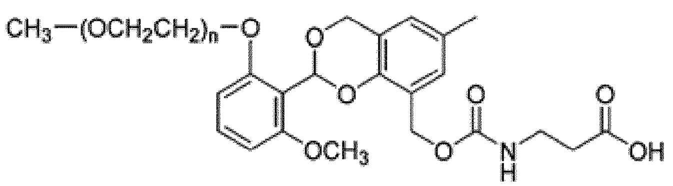

n = APPROXIMATELY 455 (78)

(Degradability Test 2)

Each of the compounds of Formulas (75), (76), (77) and (78) (20 mg) was dissolved in sodium phosphate deuterium oxide buffer solution (0.7 mL) having a pD of 7.4, and then placed in a thermostatic bath at 40°C, and NMR measurement was performed at a random timing. Fig. 3 shows a β-alanine adduct amount at a random timing assuming a β-alanine adduct amount at 0 hour of standing as 100%.

The half-life (t1/2) calculated from the calculation formula [Math. 1] described in the degradability test based on

the approximation formula of the graph was 2.3 days for the compound of Formula (75), 30.1 days for the compound of Formula (76), 23.1 days for the compound of Formula (77), and 1.4 days for the compound of Formula (78), which indicates that the compounds according to the present invention have a desired half-life under a physiological condition, as compared with the compound exemplified in Patent Literature 3 expressed by Formula (53), which is in the prior art.

(Comparative Example 3)

SUNBRIHGT MENP-20T (22.5 mg, 1.13 mmol) manufactured by NOF Corporation and a compound $NH_2$-C6-(Apt-TNFα) (1.8 mg, 141.8 nmol) having a C6 amino linker at 5' terminal of an aptamer represented by SEQ ID NO: 1, which is reported in WO2019203904A1 to have a TNFα inhibitory activity and to have a reduced TNFα inhibitory activity upon PEGylation, were dissolved in 125 μL of 100 mM sodium bicarbonate buffer solution (pH 8.3) and reacted at 40°C for 24 hours under a nitrogen atmosphere. After the reaction, the reaction solution was filtered and then subjected to HPLC measurement using a reverse phase column under the following conditions. The fractionated solution was substituted with PBS by ultrafiltration while monitoring the eluate at 260 nm, to obtain PEG(20k)-OCO-NH-C6-(Apt-TNFα) in which the compound of Formula (28) having a purity of 98.9% and a GPC peak top molecular weight of 33,101 was bonded to Apt-TNFα in a molar ratio of 1:1. In Fig. 5, (A) shows a result of the HPLC measurement of the fractionated PEG(20k)-OCO-NH-C6-(Apt-TNFα).

HPLC equipment: Thermo Ultimate3000
Column: Xbridge Oligonucleotide BEH C18 (4.6 × 50mm, 2.5 um, manufactured by Waters)
Flow rate: 1 mL/min
Analysis time: 25 minutes (including equilibration)
Column temperature: 35°C
Injection volume: 30 μL
Detector: UV (measurement wavelength: 260 nm)
Mobile phase A: 25 mM hexafluoroisopropanol-15 mM dibutylamine/water
Mobile phase B: 25 mM hexafluoroisopropanol-15 mM dibutylamine/methanol
Gradient program (A/B): 70/30 (0 min) → 10/90 (20 min) → 10/90 (30 min)

(Example 25)

In the same manner as in Comparative Example 3, except for using the compound of Formula (28) (22.5 mg, 1.13 mmol) instead of SUNBRIGHT MENP-20T manufactured by NOF Corporation, 2MPEG(20k)-OCO-NH-C6-(Apt-TNFα) was obtained in which the compound of Formula (28) having a purity of 84.7% and a GPC peak top molecular weight of 30,632 was bonded to Apt-TNFα in a molar ratio of 1:1. In Fig. 5, (B) shows a result of the HPLC measurement of the fractionated 2MPEG(20k)-OCO-NH-C6-(Apt-TNFα).

(Evaluation of TNFα Inhibitory Activity of PEGylated Aptamers)

To each well of a 96-well plate (Thermo Fisher Scientific), 80 μL of each of NF-kB Reporter with a viable cell concentration of $4 \times 10^5$ cells/mL, Luciferase, and HEK293 cells (BPS Bioscience Inc.) were added and cultured for 22 hours. Then, 10 μL of 5 μM $NH_2$-C6-(Apt-TNFα) solution, 500 nM PEG(20k)-OCO-NH-C6-(Apt-TNFα) solution or 500 nM 2MPEG(20k)-OCO-NH-C6-(Apt-TNFα) solution was added to each independent well. After the addition, 10 μL of a 50 ng/mL TNFα solution was added and cultured for 0.5 hours, after which luciferase activity was measured using Bio-Glo Luciferase Assay System (Promega).

The activity was calculated as a relative value, with the system without TNFα treatment set as 0% and the system with TNFα treatment set as 100%.

The results are expressed as a mean value ± standard error, and P values were calculated by Student's t-test, with a two-tailed P value of < 0.05 defined as statistically significant.

As a result of the evaluation, while the TNFα inhibitory activity was confirmed for $NH_2$-C6-(Apt-TNFα) and 2MPEG(20k)-OCO-NH-C6-(Apt-TNFα), the TNFα inhibitory activity was not confirmed for PEG(20k)-OCO-NH-C6-(Apt-TNFα). It indicates that 2MPEG(20k)-OCO-NH-C6-(Apt-TNFα) according to the present invention can sustained-release a physiologically functional substance and can improve pharmacological activity, as compared with PEG(20k)-OCO-NH-C6-(Apt-TNFα) in the conventional art.

Fig. 6 shows evaluation results of TNFα inhibitory activity using PEG(20k)-OCO-NH-C6-(Apt-TNFα), 2MPEG(20k)-OCO-NH-C6-(Apt-TNFα), and $NH_2$-C6-(Apt-TNFα).

(Comparative Example 4)

To 0.25 mL of a 100 mg/mL solution of SUNBRIGHT MENP-20T manufactured by NOF Corporation dissolved in acetonitrile, 0.25 mL of a 29 mg/mL insulin solution dissolved in 0.2 M borate buffer solution (pH 10.5) was added and reacted at 25°C for 3 hours under a nitrogen atmosphere. After the reaction, the solution was replaced with 10 mM ammonium formate buffer solution (pH 9.0) by ultrafiltration, and then PEG(20k)-OCO-NH-insulin was obtained by anion exchange chromatography under the following conditions. In Fig. 7, (A) shows a result of HPLC analysis of purified PEG(20k)-OCO-NH-insulin.

HPLC equipment: Nexera (Shimadzu Corporation)
Column: Asahipack ES-502N 7C (7.5 × 100 mm, 9 um, Showa Denko)
Flow rate: 1 mL/min
Analysis time: 30 minutes
Column temperature: 25°C
Injection volume: 50 μL
Detector: PDA (measurement wavelength: 280 nm)
Mobile phase A: 10 mM ammonium formate buffer solution (pH 8.5)
Mobile phase B: 10 mM ammonium formate buffer solution (pH 8.5) containing 0.25 M ammonium sulfate
Gradient program (A/B): 100/0 (0 min) → 67/33 (20 min) → 0/100 (30 min)

(Example 26)

2MPEG(20k)-OCO-NH-insulin was obtained in the same manner as in Comparative Example 4, except that the compound of Formula (28) was used instead of SUNBRIGHT MENP-20T manufactured by NOF Corporation. In Fig. 7, (B) shows a result of the HPLC analysis of purified 2MPEG(20k)-OCO-NH-insulin.

(Evaluation of Stability of PEGylated Insulin)

With 100 μL of 100 mM phosphate buffer solution (pH 6.0), 100 μL of 100 mM phosphate buffer solution (pH 7.0), 100 μL of 100 mM ammonium formate buffer solution (pH 8.0), or 100 μL of 100 mM ammonium formate buffer solution (pH 9.0), 100 μL of 0.5 mg/mL PEG(20k)-OCO-NH-insulin prepared in Comparative Example 4 or 100 μL of 0.5 mg/mL 2MPEG(20k)-OCO-NH-insulin prepared in Example 26 was mixed , and then allowed to stand at 25°C and subjected to HPLC analysis at a random timing.

For PEG(20k)-OCO-NH-insulin, no decrease in PEG(20k)-OCO-NH-insulin over time was observed at any of the measured pH values. On the other hand, for 2MPEG(20k)-OCO-NH-insulin, 2MPEG(20k)-OCO-NH-insulin decreased over time at all measured pH values, and the rate of decrease was particularly high at pH 6.0, confirming a remarkable increase in insulin. From the above, it was shown that in the 2MPEG(20k)-OCO-NH-insulin according to the present invention, the acetal is hydrolyzed over time to release insulin. Furthermore, it was shown that hydrolysis of the acetal structure could be reduced under a neutral to basic condition.

In Fig. 8, (A) shows an amount of PEG(20k)-OCO-NH-insulin at random timings with the amount of PEG(20k)-OCO-NH-insulin at 0 hour of standing set to 100%, and in Fig. 8, (B) shows an amount of 2MPEG(20k)-OCO-NH-insulin at random timings with the amount of 2MPEG(20k)-OCO-NH-insulin at 0 hour of standing set to 100%.

INDUSTRIAL APPLICABILITY

The acetal-type releasable polyoxyethylene derivative of the present invention is capable of gradually releasing a biofunctional molecule through hydrolysis of the acetal structure under a physiological condition, thereby improving the pharmacological action of the biofunctional molecule modified with the polyoxyethylene derivative.

## Claims

1. An acetal-type releasable polyoxyethylene derivative, expressed by the following Formula (1), (2), (3) or (4), and cleaved under a physiological condition,

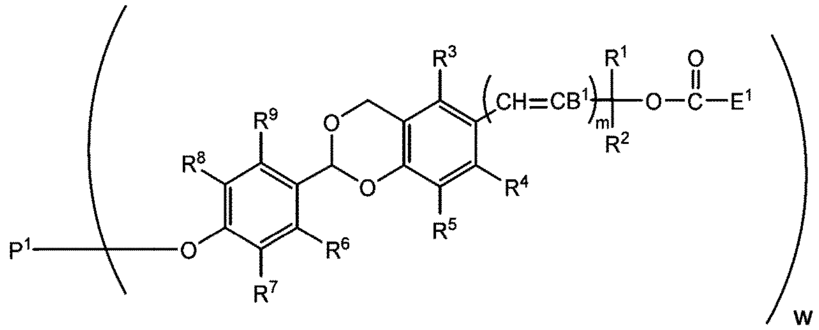

(1)

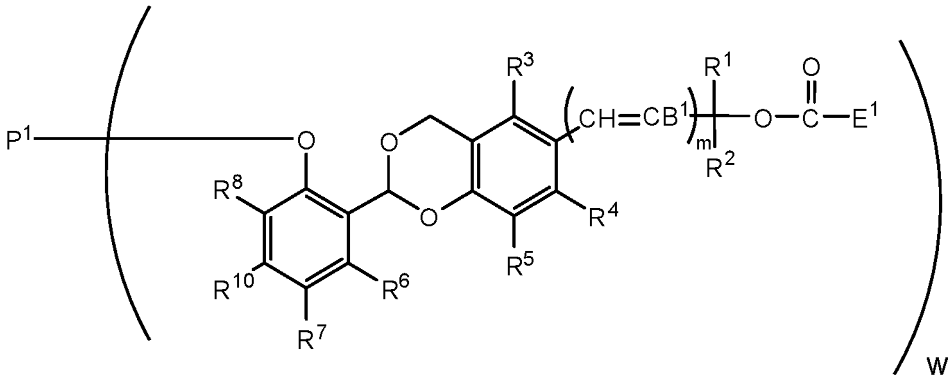

(2)

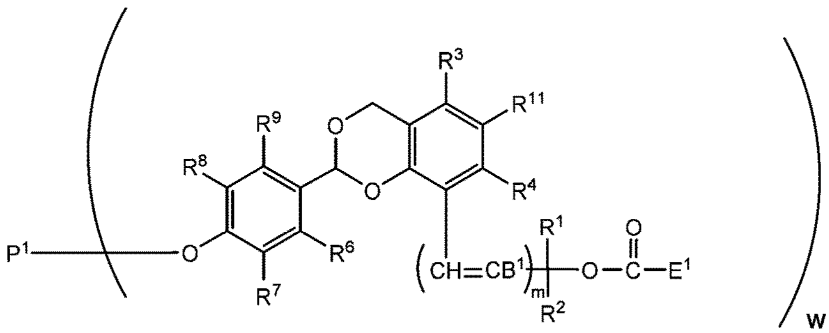

(3)

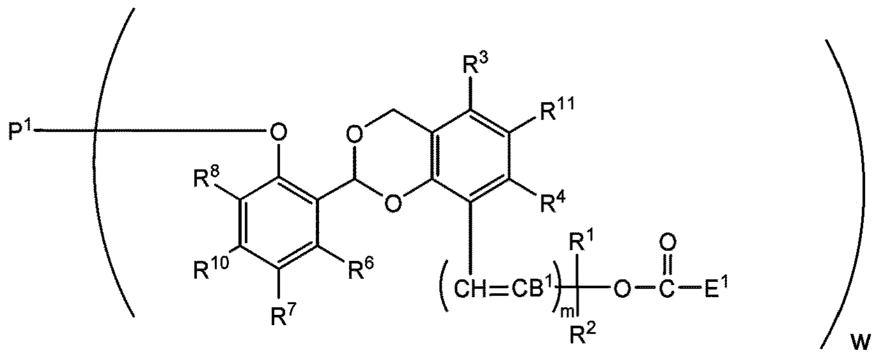

(4)

wherein

$B^1$ represents a hydrogen atom or $-C(R^1)(R^2)OC(O)E^1$,
$E^1$ represents a leaving group,
$P^1$ represents a polyoxyethylene derivative having a dehydroxylated group,
w represents an integer of 1 to 8,
$R^1$, $R^2$, $R^3$, $R^4$, $R^5$and $R^{11}$ each independently represent a hydrocarbon group having 1 to 10 carbon atoms or a

hydrogen atom,
$R^6$, $R^7$, $R^8$, $R^9$ and $R^{10}$ each independently represent an electron-withdrawing substituent, an electron-donating substituent, or a hydrogen atom, and
m represents 0 or 1.

**2.** The acetal-type releasable polyoxyethylene derivative according to claim 1, wherein m represents 0, $R^1$ and $R^2$ represent a hydrogen atom, and $R^3$, $R^4$, $R^5$ and $R^{11}$ each independently represent a hydrogen atom or a methyl group.

**3.** A method for producing the acetal-type releasable polyoxyethylene derivative according to claim 1 or 2, the method comprising:

a coupling step of coupling a polyoxyethylene derivative with a hydroxybenzaldehyde derivative to obtain a coupling product expressed by the following Formula (5) or (6);
an acetalization step of, after the coupling step, reacting the coupling product expressed by the Formula (5) or (6) with a phenol having a hydroxymethyl group at 2-position and having a substituent $(-CH=CB^1)_mC(R^1)(R^2)-OH$ ($B^1$, m, $R^1$, and $R^2$ are as described above) at 4-position or 6-position under an acidic condition to obtain an acetal structure; and
a leaving group structure introduction step of introducing a leaving group structure $(-OC(O)E^1)$ to a terminal of the substituent at the 4-position or the 6-position after the acetalization step,

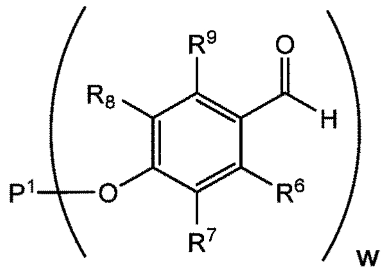
(5)

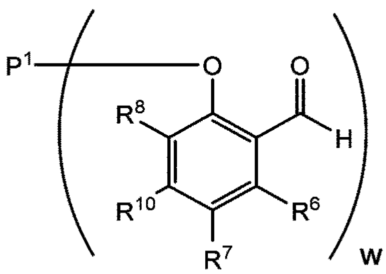
(6)

wherein $P^1$, w, $R^6$, $R^7$, $R^8$, $R^9$ and $R^{10}$ are as described above.

**4.** The method for producing the acetal-type releasable polyoxyethylene derivative according to claim 3, the method further comprising, between the acetalization step and the leaving group structure introduction step:

a deprotection step of deprotecting an amino group protected by a protecting group in the coupling product expressed by the Formulas (5) and (6); and
a step of introducing a group capable of reacting with a biofunctional molecule into the amino group deprotected after the deprotection step.

**5.** An acetal-type releasable polyoxyethylene conjugate, expressed by the following Formula (7), (8), (9) or (10), and cleaved under a physiological condition,

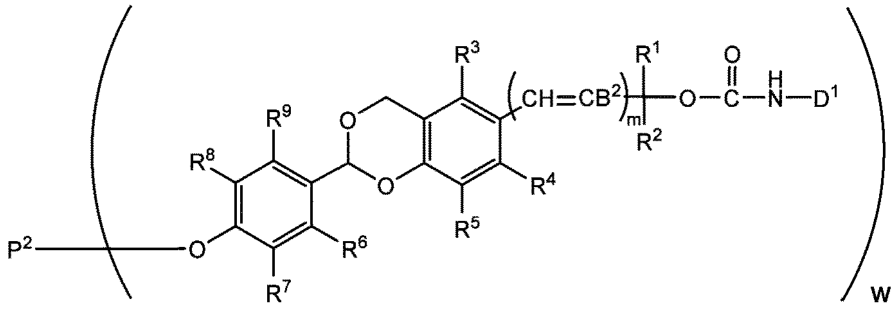

(7)

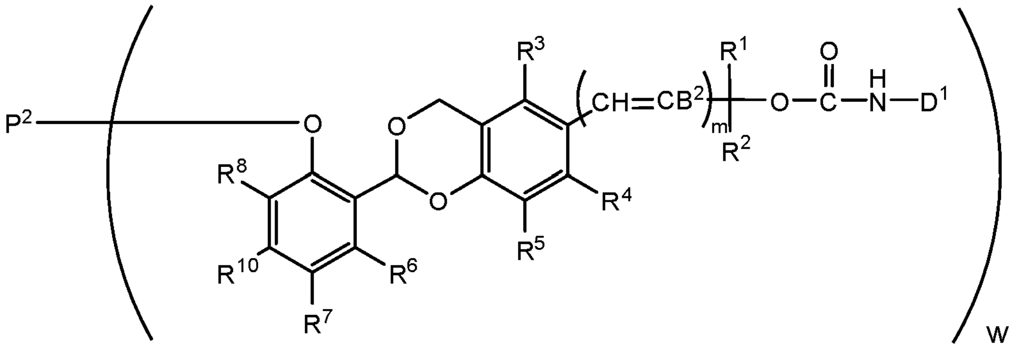

(8)

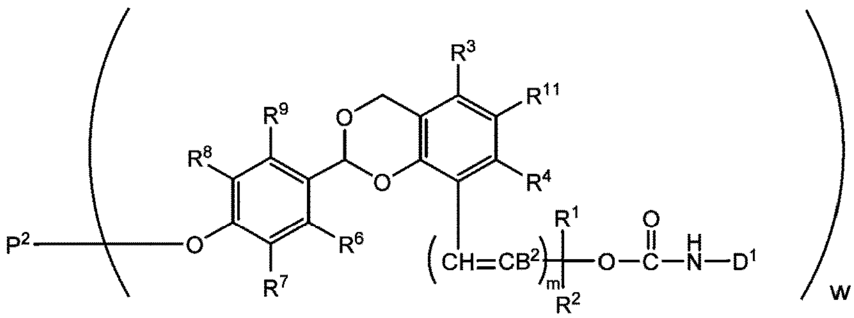

(9)

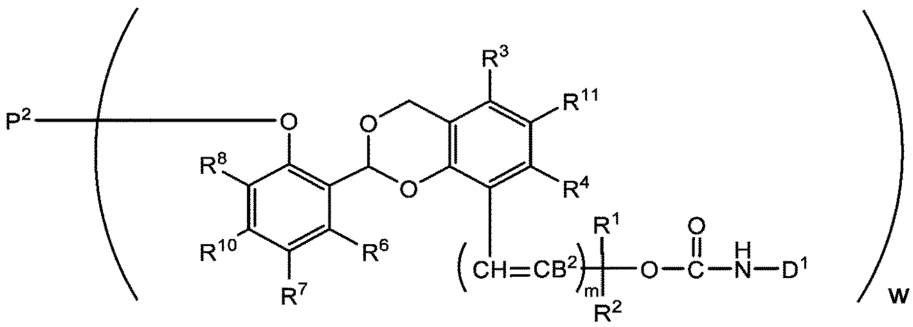

(10)

wherein

$B^2$ represents a hydrogen atom or $-C(R^1)(R^2)OC(O)NHD^1$,
$D^1$ represents a residue obtained by removing an amino group that constitutes a carbamate bond from an amino group contained in a biofunctional molecule,
$P^2$ represents a polyoxyethylene derivative having a dehydroxylated group, or a conjugate of a biofunctional molecule and a polyoxyethylene derivative having a dehydroxylated group,
w represents an integer of 1 to 8,
$R^1$, $R^2$, $R^3$, $R^4$, $R^5$ and $R^{11}$ each independently represent a hydrocarbon group having 1 to 10 carbon atoms or a hydrogen atom,

$R^6$, $R^7$, $R^8$, $R^9$ and $R^{10}$ each independently represent an electron-withdrawing substituent, an electron-donating substituent, or a hydrogen atom, and
m represents 0 or 1.

**6.** A method for producing the acetal-type releasable polyoxyethylene conjugate according to claim 5, the method comprising:

a coupling step of reacting the acetal-type releasable polyoxyethylene derivative according to claim 1 or 2 with a biofunctional molecule in a neutral or basic buffer solution which may contain a water-soluble organic solvent; and
a purification step under a neutral or basic condition after the coupling step.

**Patentansprüche**

**1.** Acetalartiges freisetzbares Polyoxyethylenderivat, das durch die folgende Formel (1), (2), (3) oder (4) ausgedrückt und unter physiologischen Bedingungen abgespalten wird,

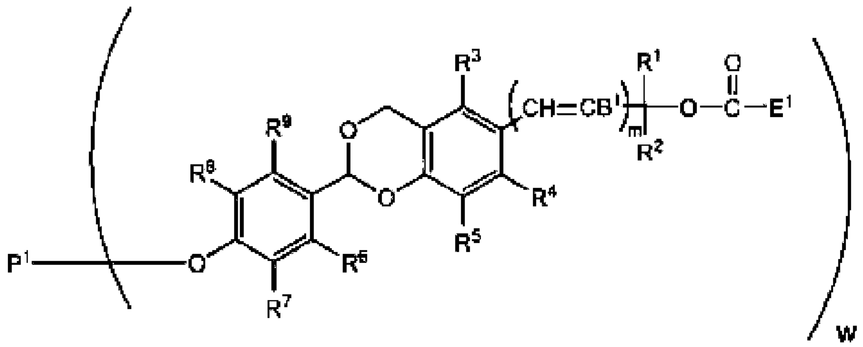
(1)

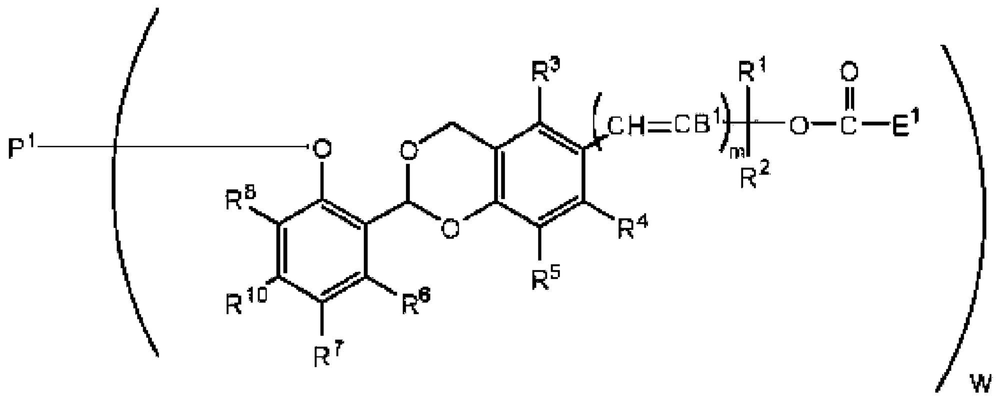
(2)

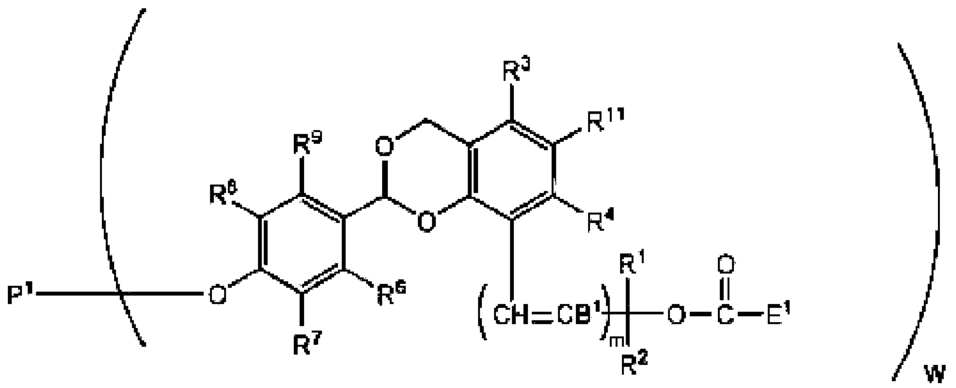
(3)

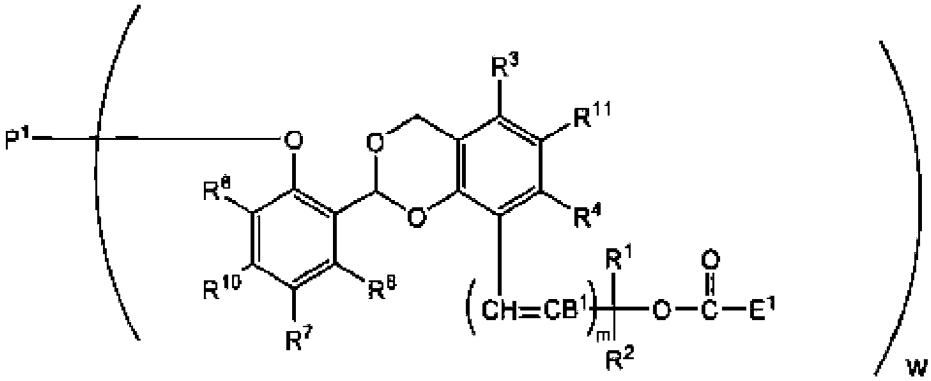

(4)

worin

$B^1$ ein Wasserstoffatom oder $-C(R^1)(R^2)OC(O)E^1$ darstellt,
$E^1$ eine Abgangsgruppe darstellt,
$P^1$ ein Polyoxyethylenderivat mit einer dehydroxylierten Gruppe darstellt,
w eine ganze Zahl von 1 bis 8 darstellt,
$R^1$, $R^2$, $R^3$, $R^4$, $R^5$ und $R^{11}$ jeweils unabhängig eine Kohlenwasserstoffgruppe mit 1 bis 10 Kohlenstoffatomen oder ein Wasserstoffatom darstellen,
$R^6$, $R^7$, $R^8$, $R^9$ und $R^{10}$ jeweils unabhängig einen elektronenziehenden Substituenten, einen elektronenspendenden Substituenten oder ein Wasserstoffatom darstellen und
m 0 oder 1 darstellt.

**2.** Acetalartiges freisetzbares Polyoxyethylenderivat gemäß Anspruch 1, wobei m 0 darstellt, $R^1$ und $R^2$ ein Wasserstoffatom darstellen und $R^3$, $R^4$, $R^5$ und $R^{11}$ jeweils unabhängig ein Wasserstoffatom oder eine Methylgruppe darstellen.

**3.** Verfahren zur Herstellung des acetalartigen freisetzbaren Polyoxyethylenderivats gemäß Anspruch 1 oder 2, wobei das Verfahren umfasst:

einen Kupplungsschritt zum Kuppeln eines Polyoxyethylenderivats mit einem Hydroxybenzaldehydderivat, um ein durch die folgende Formel (5) oder (6) ausgedrücktes Kupplungsprodukt zu erhalten,
einen Acetalisierungsschritt zur Reaktion des durch die Formel (5) oder (6) ausgedrückten Kupplungsprodukts mit einem Phenol mit einer Hydroxymethylgruppe an der 2-Position und mit einem Substituenten $(-CH=CB^1)_mC(R^1)(R^2)-OH$ ($B^1$, m, $R^1$ und $R^2$ sind so wie oben beschrieben) an der 4-Position oder 6-Position unter sauren Bedingungen unter Erhalt einer Acetalstruktur nach dem Kupplungsschritt und
einen Abgangsgruppenstruktureinführungsschritt zum Einführen einer Abgangsgruppenstruktur $(-OC(O)E^1)$ an ein Ende des Substituenten an der 4-Position oder der 6-Position nach dem Acetalisierungsschritt,

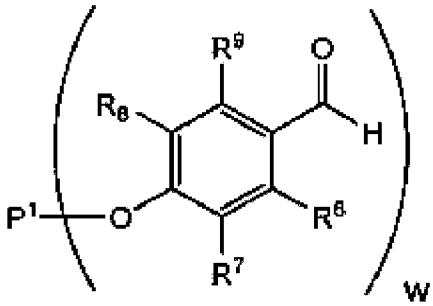

(5)

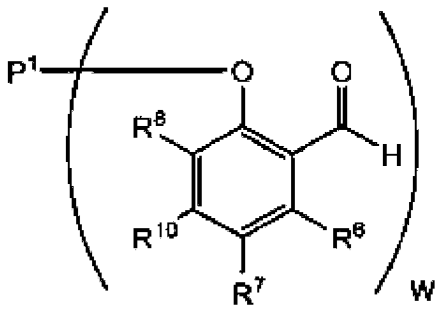

(6)

worin $P^1$, w, $R^6$, $R^7$, $R^8$, $R^9$ und $R^{10}$ so wie oben beschrieben sind.

**4.** Verfahren zur Herstellung des acetalartigen freisetzbaren Polyoxyethylenderivats gemäß Anspruch 3, wobei das Verfahren ferner zwischen dem Acetalisierungsschritt und dem Abgangsgruppenstruktureinführungsschritt folgendes umfasst:

einen Entschützungsschritt zum Entschützen einer durch eine Schutzgruppe geschützten Aminogruppe in dem durch die Formeln (5) und (6) ausgedrückten Kupplungsprodukt und
einen Schritt zum Einführen einer Gruppe, die zur Reaktion mit einem biofunktionellen Molekül geeignet ist, in die entschützte Aminogruppe nach dem Entschützungsschritt.

**5.** Acetalartiges freisetzbares Polyoxyethylenkonjugat, das durch die folgende Formel (7), (8), (9) oder (10) ausgedrückt und unter physiologischen Bedingungen gespalten wird,

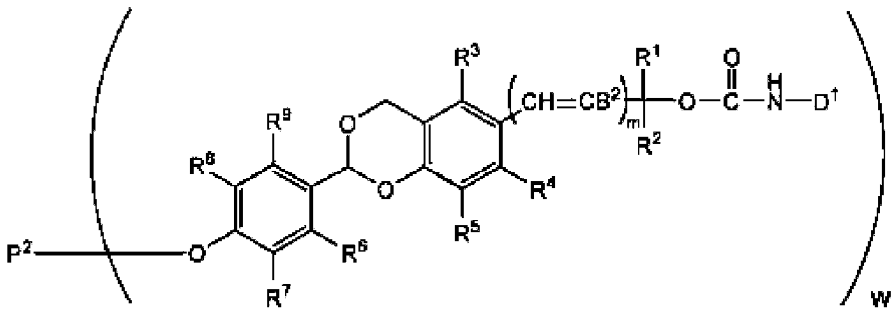

(7)

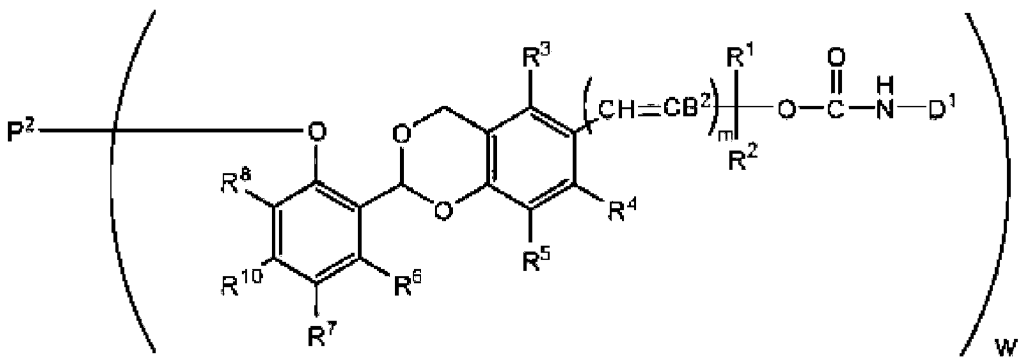

(8)

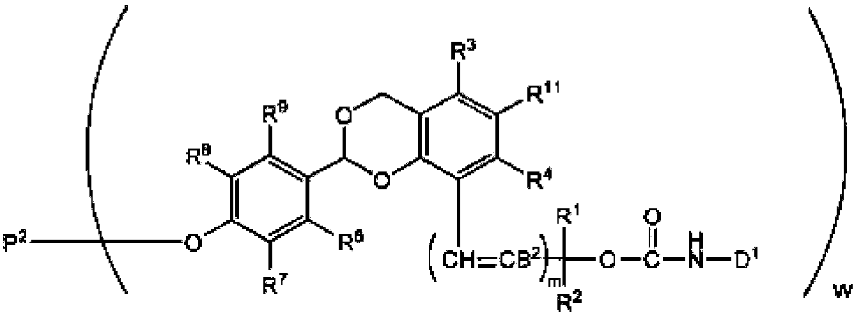

(9)

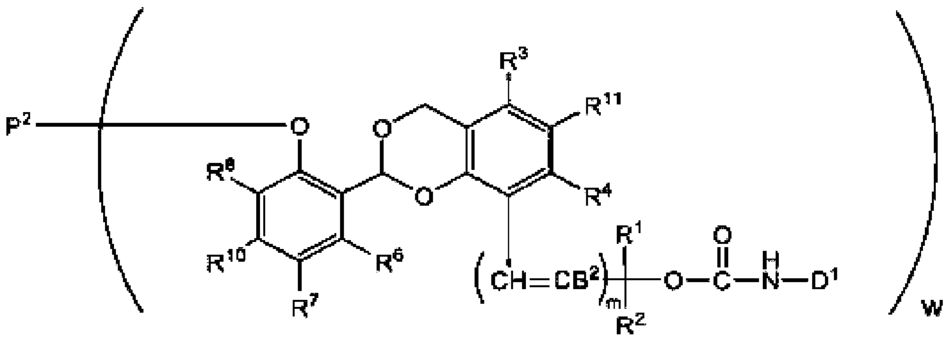

(10)

worin

$B^2$ ein Wasserstoffatom oder -C($R^1$)($R^2$)OC(O)NHD$^1$ darstellt,
$D^1$ einen Rest darstellt, der durch Entfernung einer Aminogruppe die eine Carbamatbindung bildet, von einer in einen biofunktionellen Molekül enthaltenen Aminogruppe erhalten wird,
$P^2$ ein Polyoxyethylenderivat mit einer dehydroxylierten Gruppe oder ein Konjugat aus einem biofunktionellen Molekül und einem Polyoxyethylenderviat mit einer dehydroxylierten Gruppe darstellt,
w eine ganze Zahl von 1 bis 8 darstellt,
$R^1$, $R^2$, $R^3$, $R^4$, $R^5$ und $R^{11}$ jeweils unabhängig eine Kohlenwasserstoffgruppe mit 1 bis 10 Kohlenstoffatomen oder ein Wasserstoffatom darstellen,
$R^6$, $R^7$, $R^8$, $R^9$ und $R^{10}$ jeweils unabhängig einen elektronenziehenden Substituenten, einen elektronenspendenden Substituenten oder ein Wasserstoffatom darstellen und
m 0 oder 1 darstellt.

**6.** Verfahren zur Herstellung des acetalartigen freisetzbaren Polyoxyethylenkonjugats gemäß Anspruch 5, wobei das Verfahren umfasst:

einen Kupplungsschritt zur Reaktion des acetalartigen freisetzbaren Polyoxyethylenderivats gemäß Anspruch 1 oder 2 mit einem biofunktionellen Molekül in einer neutralen oder basischen Pufferlösung, die ein wasserlösliches organisches Lösungsmittel enthält, und
einen Reinigungsschritt unter neutralen oder basischen Bedingungen nach dem Kupplungsschritt.

## Revendications

**1.** Dérivé de polyoxyéthylène libérable de type acétal, exprimé par la Formule (1), (2), (3) ou (4) suivante, et clivé dans une condition physiologique,

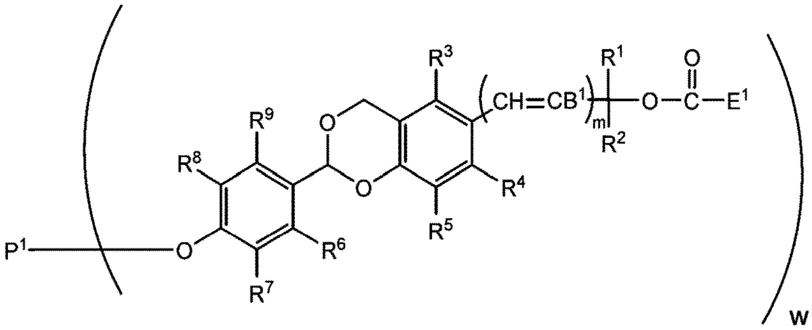
(1)

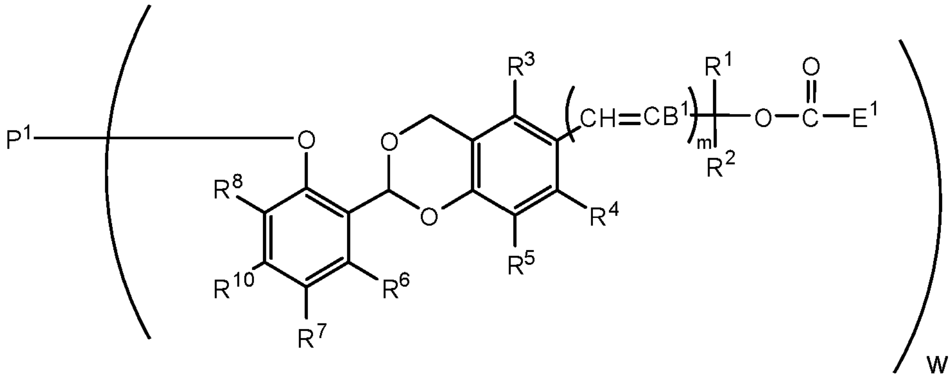
(2)

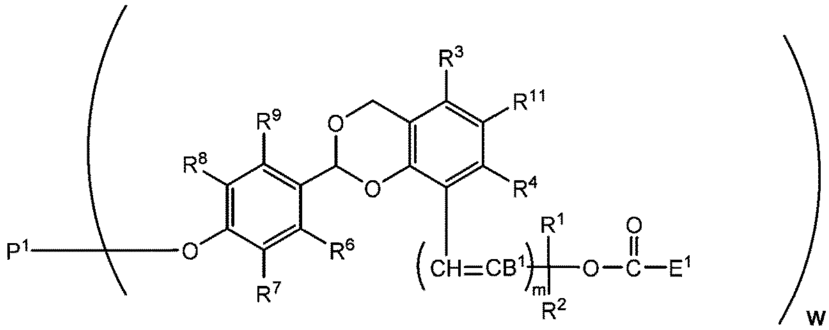
(3)

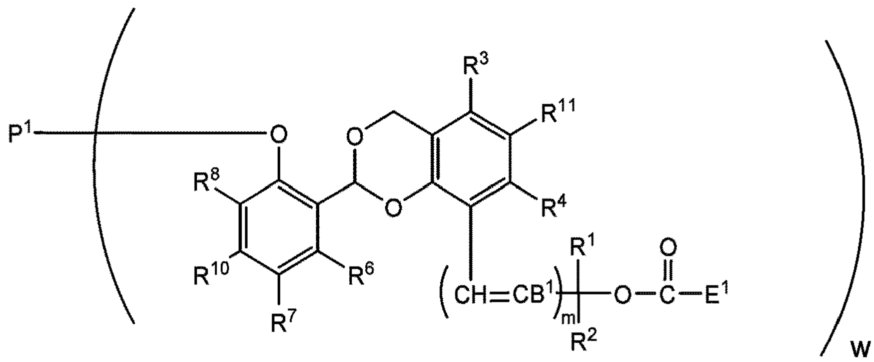
(4)

dans lequel

$B^1$ représente un atome d'hydrogène ou $-C(R^1)(R^2)OC(O)E^1$,
$E^1$ représente un groupe partant,
$P^1$ représente un dérivé de polyoxyéthylène présentant un groupe déshydroxylé,
w représente un nombre entier de 1 à 8,
$R^1$, $R^2$, $R^3$, $R^4$, $R^5$ et $R^{11}$ représentent chacun indépendamment un groupe hydrocarboné présentant 1 à 10

atomes de carbone ou un atome d'hydrogène,
$R^6$, $R^7$, $R^8$, $R^9$ et $R^{10}$ représentent chacun indépendamment un substituant attracteur d'électrons, un substituant donneur d'électrons, ou un atome d'hydrogène, et
m représente 0 ou 1.

**2.** Dérivé de polyoxyéthylène libérable de type acétal selon la revendication 1, dans lequel m représente 0, $R^1$ et $R^2$ représentent un atome d'hydrogène, et $R^3$, $R^4$, $R^5$ et $R^{11}$ représentent chacun indépendamment un atome d'hydrogène ou un groupe méthyle.

**3.** Procédé de production du dérivé de polyoxyéthylène libérable de type acétal selon la revendication 1 ou la revendication 2, le procédé comprenant :

une étape de couplage consistant à coupler un dérivé de polyoxyéthylène avec un dérivé d'hydroxybenzaldéhyde pour obtenir un produit de couplage exprimé par la Formule (5) ou (6) suivante ;
une étape d'acétalisation consistant, après l'étape de couplage, à faire réagir le produit de couplage exprimé par la Formule (5) ou (6) avec un phénol présentant un groupe hydroxyméthyle en position 2 et présentant un substituant $(-CH=CB^1)_mC(R^1)(R^2)-OH$ ($B^1$, m, $R^1$, et $R^2$ sont tels que décrits ci-dessus) en position 4 ou en position 6 dans une condition acide pour obtenir une structure acétal ; et
une étape d'introduction d'une structure de groupe partant consistant à introduire une structure de groupe partant $(-OC(O)E^1)$ à une terminaison du substituant en position 4 ou en position 6 après l'étape d'acétalisation,

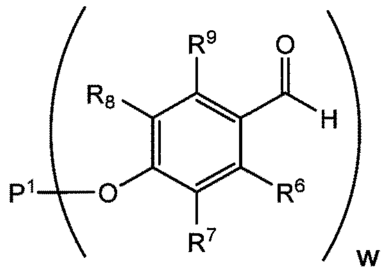
(5)

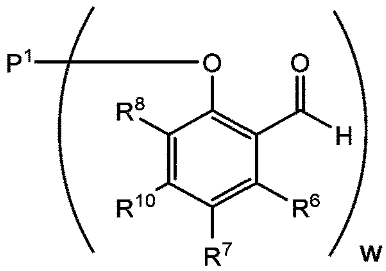
(6)

dans lequel $P^1$, w, $R^6$, $R^7$, $R^8$, $R^9$ et $R^{10}$ sont tels que décrits ci-dessus.

**4.** Procédé de production du dérivé de polyoxyéthylène libérable de type acétal selon la revendication 3, le procédé comprenant en outre, entre l'étape d'acétalisation et l'étape d'introduction d'une structure de groupe partant :

une étape de déprotection consistant à déprotéger un groupe amino protégé par un groupe protecteur dans le produit de couplage exprimé par les Formules (5) et (6) ; et
une étape consistant à introduire un groupe capable de réagir avec une molécule biofonctionnelle dans le groupe amino déprotégé après l'étape de déprotection.

**5.** Conjugué de polyoxyéthylène libérable de type acétal, exprimé par la Formule (7), (8), (9) ou (10) suivante, et clivé dans une condition physiologique,

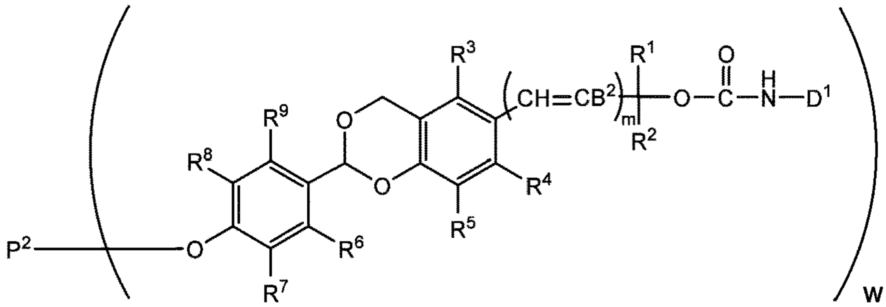

(7)

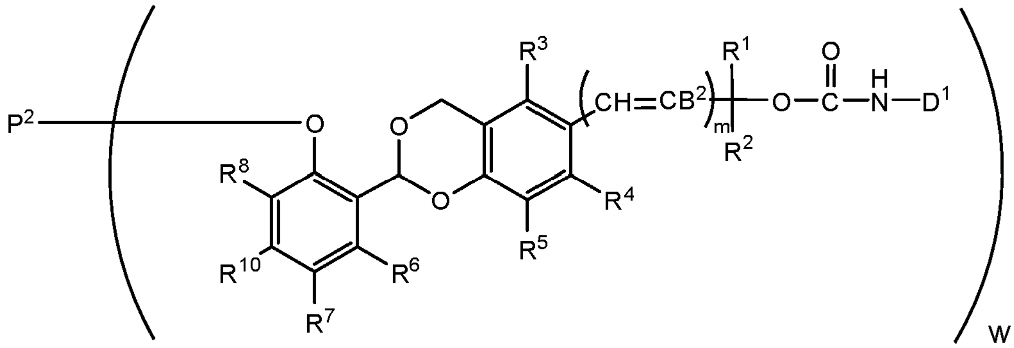

(8)

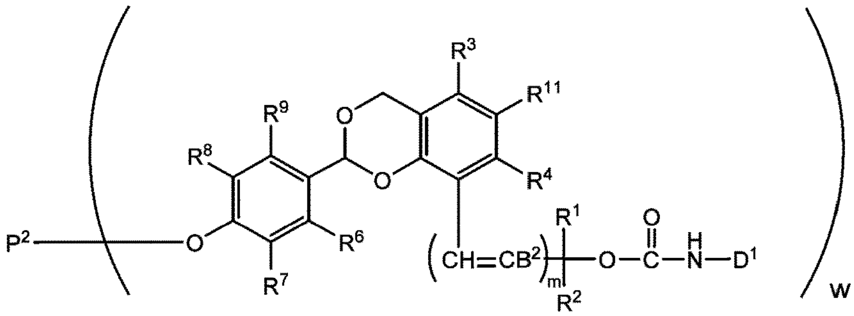

(9)

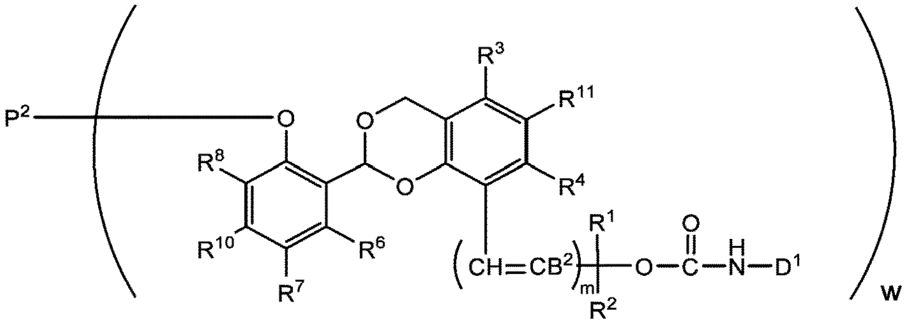

(10)

dans lequel

$B^2$ représente un atome d'hydrogène ou $-C(R^1)(R^2)OC(O)NHD^1$,

$D^1$ représente un résidu obtenu par élimination d'un groupe amino qui constitue une liaison carbamate d'un groupe amino contenu dans une molécule biofonctionnelle,

$P^2$ représente un dérivé de polyoxyéthylène présentant un groupe déshydroxylé, ou un conjugué d'une molécule biofonctionnelle et d'un dérivé de polyoxyéthylène présentant un groupe déshydroxylé,

w représente un nombre entier de 1 à 8,

$R^1$, $R^2$, $R^3$, $R^4$, $R^5$ et $R^{11}$ représentent chacun indépendamment un groupe hydrocarboné présentant 1 à 10 atomes de carbone ou un atome d'hydrogène,

$R^6$, $R^7$, $R^8$, $R^9$ et $R^{10}$ représentent chacun indépendamment un substituant attracteur d'électrons, un substituant donneur d'électrons, ou un atome d'hydrogène, et
m représente 0 ou 1.

**6.** Procédé de production du conjugué de polyoxyéthylène libérable de type acétal selon la revendication 5, le procédé comprenant :

une étape de couplage consistant à faire réagir le dérivé de polyoxyéthylène libérable de type acétal selon la revendication 1 ou la revendication 2 avec une molécule biofonctionnelle dans une solution tampon neutre ou basique qui peut contenir un solvant organique hydrosoluble ; et
une étape de purification dans une condition neutre ou basique après l'étape de couplage.

FIG. 1

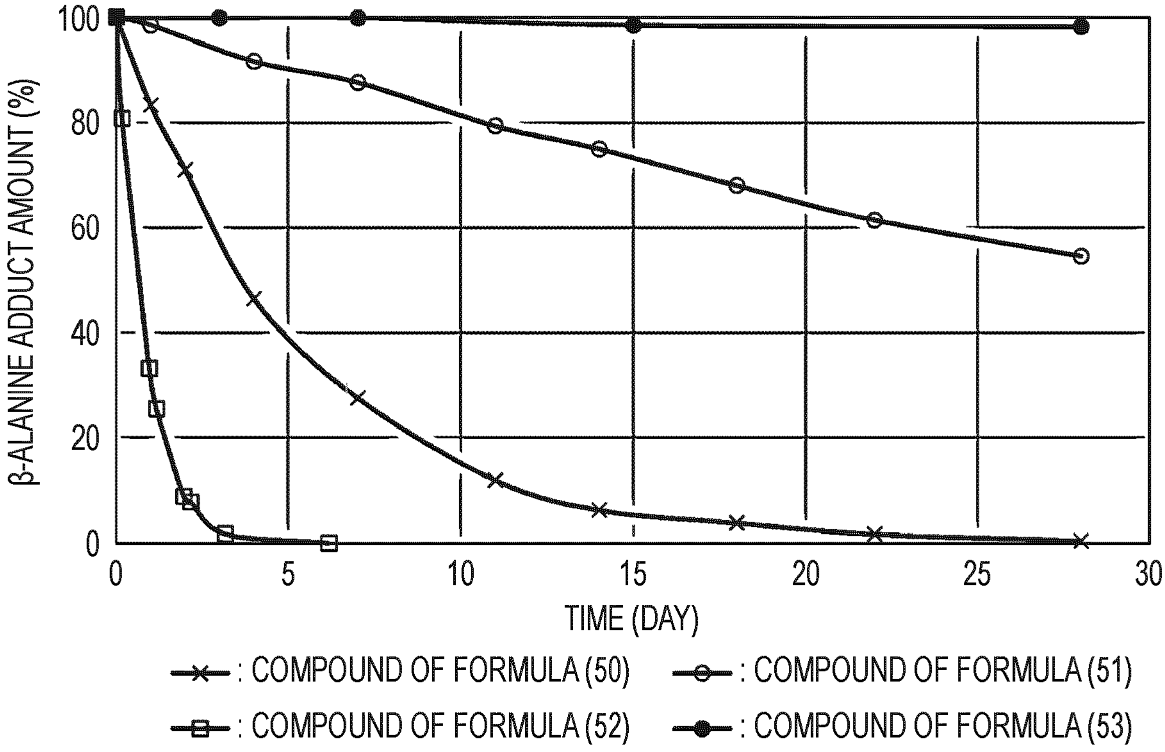
100
80
60
40
20
0
β-ALANINE ADDUCT AMOUNT (%)
0
5
10
15
20
25
30
TIME (DAY)
: COMPOUND OF FORMULA (50)
: COMPOUND OF FORMULA (51)
: COMPOUND OF FORMULA (52)
: COMPOUND OF FORMULA (53)

FIG. 2

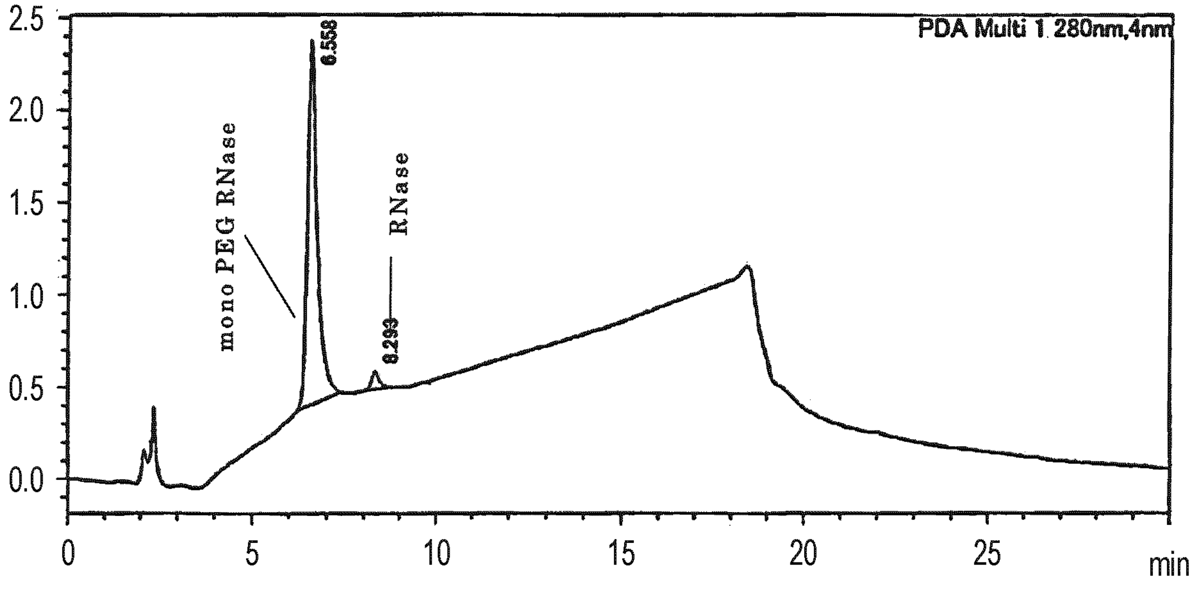
2.5
2.0
1.5
1.0
0.5
0.0
PDA Multi 1 280nm,4nm
6.558
8.293
mono PEG RNase
RNase
0
5
10
15
20
25
min

FIG. 3

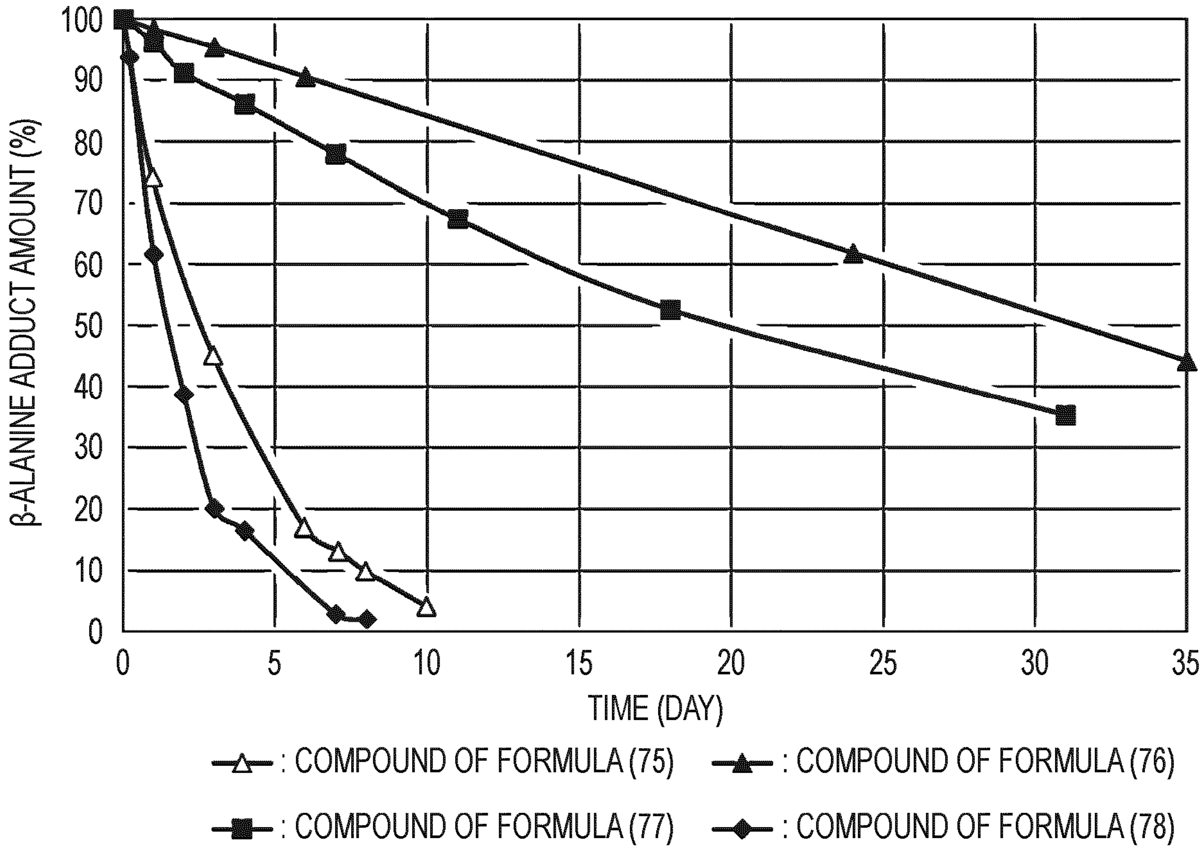


FIG. 4

NUCLEOTIDE SEQUENCE (SEQ ID NO: 1)
OF APTAMER HAVING TNFα INHIBITORY ACTIVITY

5'-GCGCCACTACAGGGGAGCTGCCATTCGAATAGGTGGGCCGC-3'

FIG. 5

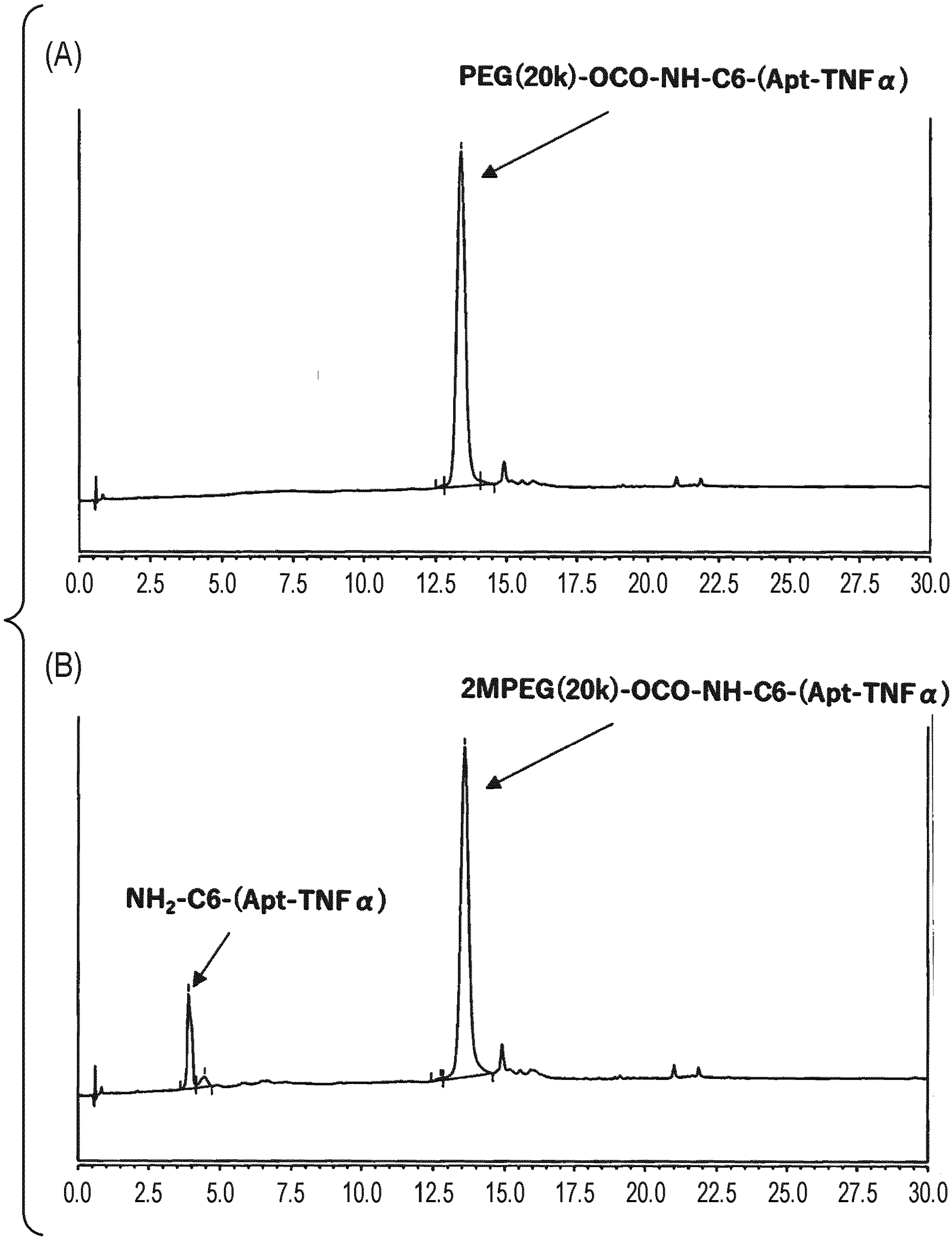
(A)
PEG(20k)-OCO-NH-C6-(Apt-TNFα)
0.0 2.5 5.0 7.5 10.0 12.5 15.0 17.5 20.0 22.5 25.0 27.5 30.0
(B)
2MPEG(20k)-OCO-NH-C6-(Apt-TNFα)
NH2-C6-(Apt-TNFα)
0.0 2.5 5.0 7.5 10.0 12.5 15.0 17.5 20.0 22.5 25.0 27.5 30.0

FIG. 6

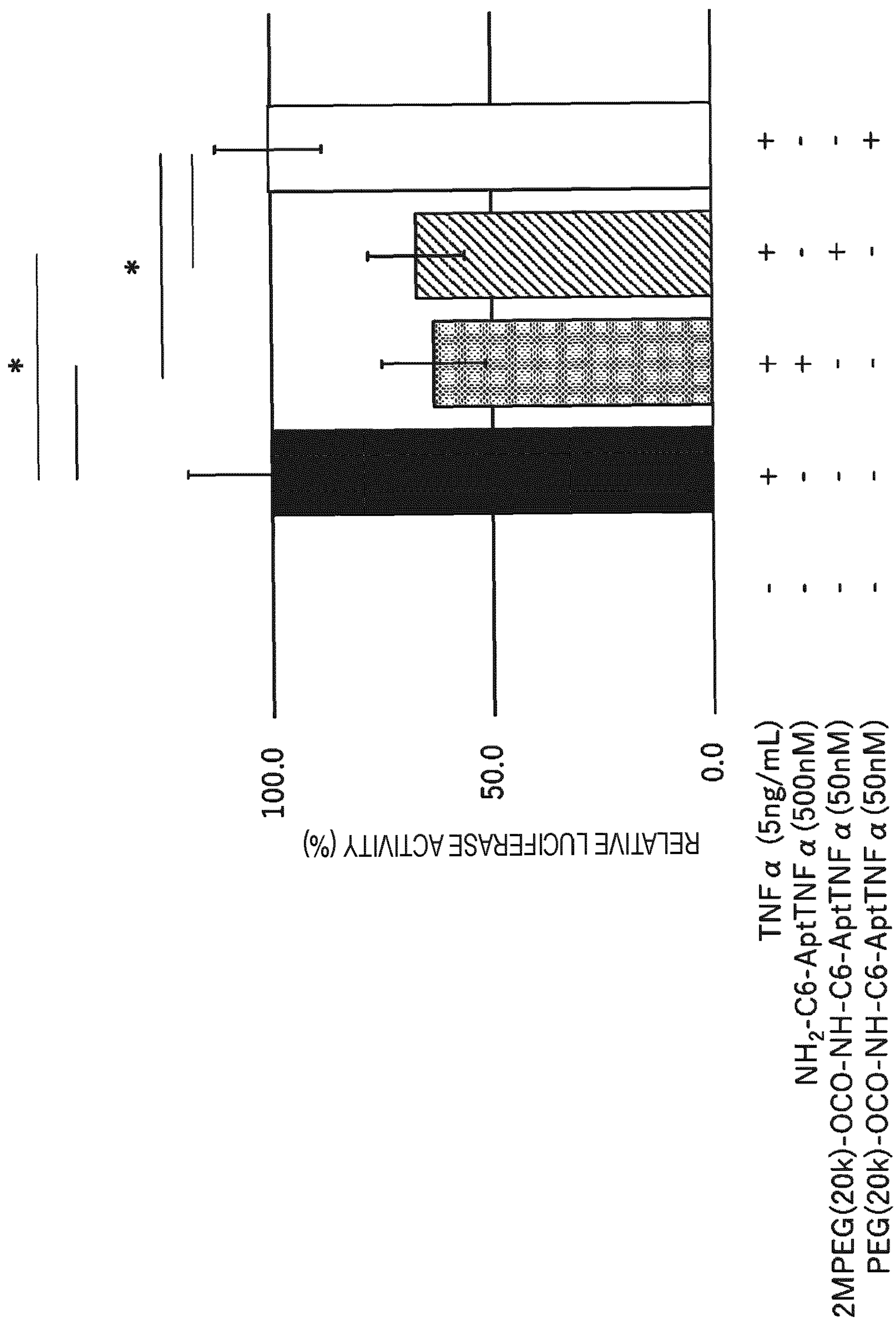
RELATIVE LUCIFERASE ACTIVITY (%)
100.0
50.0
0.0
*
*
TNF α (5ng/mL)
$NH_2$-C6-AptTNF α (500nM)
2MPEG(20k)-OCO-NH-C6-AptTNF α (50nM)
PEG(20k)-OCO-NH-C6-AptTNF α (50nM)

*FIG. 7*

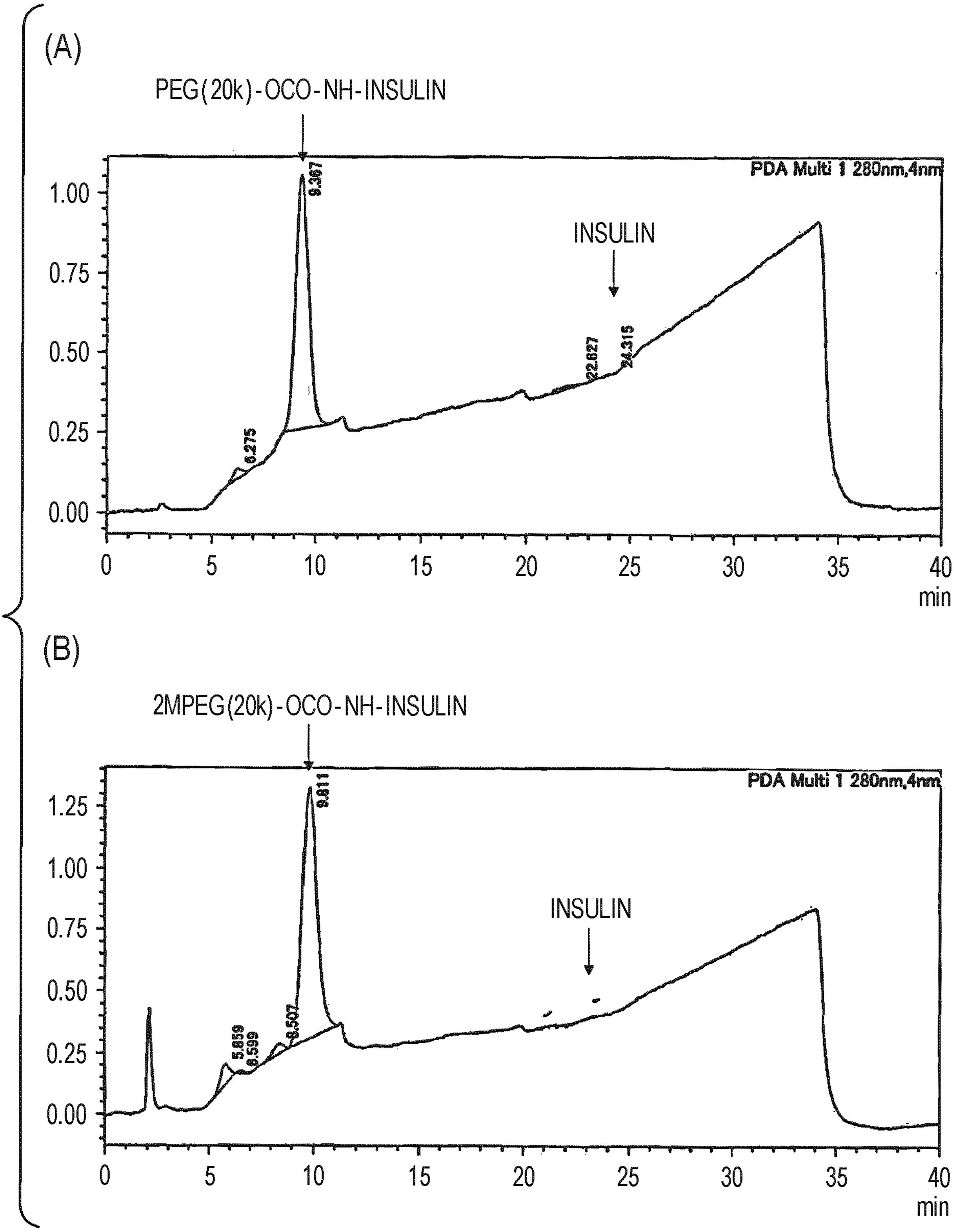
(A)
PEG(20k)-OCO-NH-INSULIN
PDA Multi 1 280nm,4nm
INSULIN
9.367
6.275
22.827
24.315
1.00
0.75
0.50
0.25
0.00
0
5
10
15
20
25
30
35
40
min
(B)
2MPEG(20k)-OCO-NH-INSULIN
PDA Multi 1 280nm,4nm
INSULIN
9.811
5.859
6.599
8.507
1.25
1.00
0.75
0.50
0.25
0.00
0
5
10
15
20
25
30
35
40
min

FIG. 8

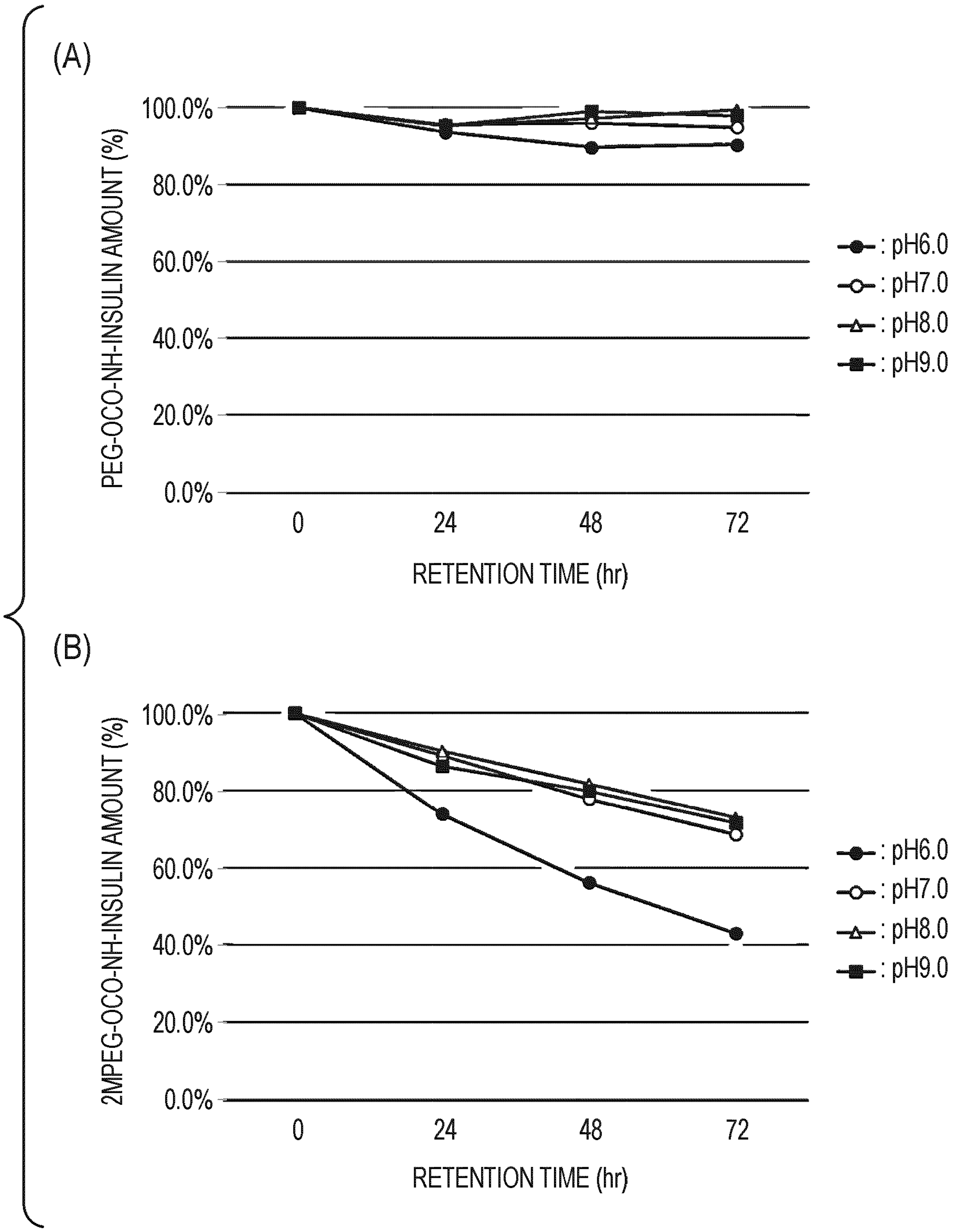
(A)
100.0%
80.0%
60.0%
40.0%
20.0%
0.0%
PEG-OCO-NH-INSULIN AMOUNT (%)
0
24
48
72
RETENTION TIME (hr)
: pH6.0
: pH7.0
: pH8.0
: pH9.0
(B)
100.0%
80.0%
60.0%
40.0%
20.0%
0.0%
2MPEG-OCO-NH-INSULIN AMOUNT (%)
0
24
48
72
RETENTION TIME (hr)
: pH6.0
: pH7.0
: pH8.0
: pH9.0

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018150311 A
- JP 2022000043 A
- JP 2018172648 A
- US 2020289656 A1
- WO 2019203904 A1


### Non-patent literature cited in the description


- **WUTS, P. G. M.** ; **GREENE, T. W.** Protective Groups in Organic Synthesis. Wiley-Interscience, 2007
- **GREG T. HERMANSO**. Bioconjugate Techniques
- **FREEMAN, J.H.** *JAm. Chem. Soc.*, 1952, vol. 74, 6257-6260